# EUROPEAN PATENT APPLICATION

(11) **EP 4 850 754 A2**
(43) Date of publication of application: **09.12.2026**
(21) Application number: 26186853.3
(22) Date of filing: 02.10.2015
(51) Int. Cl.: A61P 9/10

(54) **CARDIOSPHERE-DERIVED CELLS AND EXOSOMES SECRETED BY SUCH CELLS IN THE TREATMENT OF MUSCULAR DYSTROPHY**

(30) Priority: 03.10.2014 US 201462059308 P
(62) Document number(s) of the earlier application(s) in accordance with Art. 76 EPC: 24189285.0 / 4 494 699; 15846113.7 / 3 200 808
(71) Applicant: Cedars-Sinai Medical Center, Los Angeles, CA 90048-1865 (US)
(72) Inventor: MARBAN, Eduardo, Santa Monica, 90402 (US); AMINZADEH, Mark Amin, Los Angeles, 90048 (US)
(74) Representative: WP Thompson

(57) **Abstract**

Described herein are compositions and techniques related to generation and therapeutic application of cardiosphere-derived cells (CDCs) and CDC-derived exosomes. These cells and their secreted vesicles contain a unique milieu of biological factors, including cytokines, growth factors, transcription factors, nucleic acids including non-coding nucleic acids such as microRNAs, that serve to initiate and promote many therapeutic effects. Exosomes and their "cargo" contents, such as microRNAs can favorably modulate apoptosis, inflammation and fibrosis in the injured heart. Thus, CDC-derived exosomes represent a novel "cell-free" therapeutic candidate for tissue repair.

## Description

### STATEMENT REGARDING FEDERALLY-SPONSORED RESEARCH

This invention was made with government support under R01 HL083109 awarded by the National Institutes of Health. The government has certain rights in the invention.

### FIELD OF THE INVENTION

This invention relates to the use of cells and their extracts, specifically cellular exosomes, for therapeutic use, including treatment of heart disease.

### BACKGROUND

Duchenne muscular dystrophy (DMD) afflicts ~20,000 boys and young men in the USA. The central cause is a genetic abnormality in the dystrophin complex, with secondary damage to skeletal muscle and heart tissue. Although virtually all patients are treated with corticosteroids, no treatment has been proven effective. Heart failure (HF) secondary to DMD afflicts virtually all DMD patients aged >15 years, and is often the cause of death. DMD-associated HF aggressively progresses from the initial insult (a genetic abnormality in the dystrophin complex), to asymptomatic abnormalities in cardiac structure and function (stage B), to overt symptomatic HF (stage C), to advanced HF (stage D) and death. Progression of HF is associated with high risk of hospitalization and intense overall health care resource utilization. Modes of death during the course of DMD-associated HF include sudden cardiac death (which increases as HF worsens), or progressive HF culminating in circulatory collapse. Moreover, much of the disability in the later years of DMD is due to HF rather than to skeletal muscle disease. Thus, DMD HF represents an important, neglected target for innovative therapy.

A highly promising avenue of therapy for cardiac-related diseases and conditions includes cardiosphere-derived cells (CDCs) that are capable of stimulating regeneration, angiogenesis, and functional improvement in infarcted myocardium. Previous or ongoing trials involving CDCs target adult patients in stage B; wherein heart function is depressed, but symptoms of HF have yet to appear. In DMD-associated HF, therapeutic approaches may be most dramatic for stages C and D. These later stages of disease are associated with high mortality (>20% per year) despite optimal medical therapy, which have also never been shown to actually slow the progression of disease in DMD patients. Because of exclusionary comorbidities, heart transplantation is not an option for DMD patients. These patients are also not candidates for mechanical circulatory support devices. In summary, no treatment modality currently available addresses the underlying pathophysiology of DMD-associated HF, which is a loss of functional heart muscle and conversion of living heart muscle to scar.

Interestingly, growing evidence suggests that the positive therapeutic benefits of CDCs occur through indirect mechanisms, with most of the newly regenerated myocardium and vasculature being of endogenous origin. Perhaps due to the fact that CDCs are rich biological factories that secrete many growth factors and cytokines, the beneficial therapeutic effects of CDCs manage to persist long after the injected cells have been cleared. Of critical interest is understanding whether these positive factors may exist in cellular exosomes produced by CDCs, the lipid bilayer nanovesicles secreted by cells when multivesicular endosomes fuse with the plasma membrane. Confirming a role for secreted exosomes in these processes has yet to be considered, and understanding these processes governing CDC-initiated regeneration may open new avenues therapeutic approaches. Because no existing therapy can reverse the progression of DMD HF, CDC-derived exosomes may effectively address a major unmet medical need, by recruiting various synergistic mechanisms of benefit that have been observed in animal models of HF. This includes the ability to attract endogenous stem cells to sites of myocardial injury, promote differentiation into heart muscle and vessels, and potentially reversing the pathophysiology of HF. The potential benefits of an exosome-based approach as an alternative to cell therapy is particularly compelling given the unavailability of conventional therapy to late stage patients. The possibility exists that CDC-derived exosomes may fundamentally alter the natural history of the disease.

Described herein are compositions and techniques related to generation and therapeutic application of CDC-derived exosomes. These biological molecules contain a unique milieu of biological factors, including cytokines, growth factors, transcription factors, nucleic acids including non-coding nucleic acids such as microRNAs that serve to initiate and promote many of the therapeutic effects of CDCs. The Inventors' work demonstrates that exosomes and their constituent microRNAs favorably modulate apoptosis, inflammation and fibrosis in the injured heart, leading to functional recovery and increase tissue viability. Thus, CDC-derived exosomes represent a novel "cell-free" therapeutic candidate for tissue repair.

### SUMMARY OF THE INVENTION

Described herein is a method of treatment, including selecting a subject in need of treatment for heart failure secondary to a chronic degenerative muscular disease and administering a composition including a plurality of exosomes to the subject, wherein the plurality of the exosomes are isolated from cardiosphere-derived cells (CDCs) grown in serum-free media, include exosomes with a diameter of about 90 nm to about 200 nm and are CD81+, CD63+, or both, and further wherein administration of the composition treats the subject. In other embodiments, the chronic degenerative muscular disease is Duchenne muscular dystrophy. In other embodiments, administering a composition includes about 1 to about 100 mg exosome protein in a single dose. In other embodiments, a single dose is administered multiple times to the subject. In other embodiments, administering a composition includes injection. In other embodiments, the injection includes percutaneous injection. In other embodiments, the injection is directly into heart muscle. In other embodiments, administering a composition includes myocardial infusion. In other embodiments, myocardial infusion is intra-arterial or intravenous. In other embodiments, treatment of the subject results in decreased fibrosis, decreased inflammation, increased mitochondrial function and/or increased cardiomyogenesis. In other embodiments, decreased fibrosis includes a reduction in collagen accumulation. In other embodiments, collagen includes collagen I and/or collagen III. In other embodiments, decreased inflammation includes an increase in cytoplasmic nuclear factor (erythroid-derived 2)-like 2 (Nrf2), reduction in fatty acid peroxidation end products, reduced numbers of inflammatory cells, and/or upregulated expression of antioxidants. In other embodiments, antioxidants include heme oxygenase-1 (HO-1), catalase, superoxide dismutase-2 (SOD-2), and glutamate-cystein ligase catalytic (GCLC) subunit. In other embodiments, inflammatory cells include CD68+ macrophages and CD3+ T-cells. In other embodiments, increased mitochondrial function includes increased mitochondrial ultrastructure and/or increased mitochondrial biogenesis. In other embodiments, increased mitochondrial function includes increased nuclear PPAR-γ co-activator-1 (PGC-1) expression. In other embodiments, the exosomes include one or more microRNAs selected from the group consisting of: microRNAs miR-146a, miR148a, miR22, miR-24, miR-210, miR-150, miR-140-3p, miR-19a, miR-27b, miR-19b, miR-27a, miR-376c, miR-128, miR-320a, miR-143, miR-21, miR-130a, miR-9, miR-185, and miR-23a.

Further described herein is method of treatment, including selecting a subject in need of treatment for heart failure secondary to a chronic muscular disease and administering a composition including cardiosphere-derived cells (CDCs), wherein administration of the composition treats the subject. In other embodiments, the chronic muscular disease is Duchenne muscular dystrophy. In other embodiments, administering a composition includes about 1 x 10⁵ to about 1 x 10⁸ or more CDCs in a single dose. In other embodiments, administering a composition includes myocardial infusion. In other embodiments, myocardial infusion is intracoronary. In other embodiments, myocardial infusion is intra-arterial or intravenous. In other embodiments, treatment of the subject results in decreased fibrosis, decreased inflammation, increased mitochondrial function and/or increased cardiomyogenesis. In other embodiments, decreased fibrosis includes a reduction in collagen accumulation. In other embodiments, collagen includes collagen I and/or collagen III. In other embodiments, decreased inflammation includes an increase in cytoplasmic nuclear factor (erythroid-derived 2)-like 2 (Nrf2), reduction in fatty acid peroxidation end products, reduced numbers of inflammatory cells, and/or upregulated expression of antioxidants. In other embodiments, antioxidants include heme oxygenase-1 (HO-1), catalase, superoxide dismutase-2 (SOD-2), and glutamate-cystein ligase catalytic (GCLC) subunit. In other embodiments, inflammatory cells include CD68+ macrophages and CD3+ T-cells. In other embodiments, increased mitochondrial function includes increased mitochondrial ultrastructure and/or increased mitochondrial biogenesis. In other embodiments, increased mitochondrial function includes increased nuclear PPAR-γ co-activator-1 (PGC-1) expression.

### BRIEF DESCRIPTION OF FIGURES

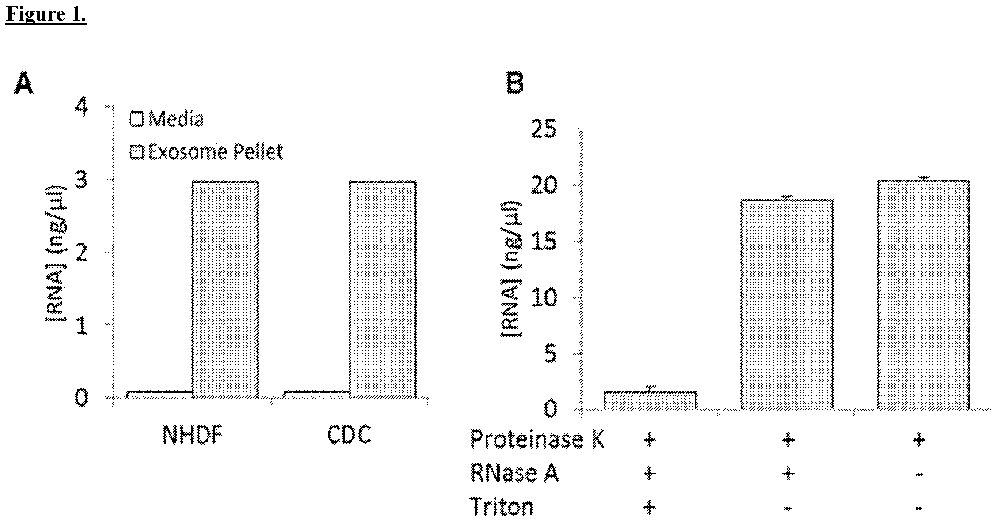

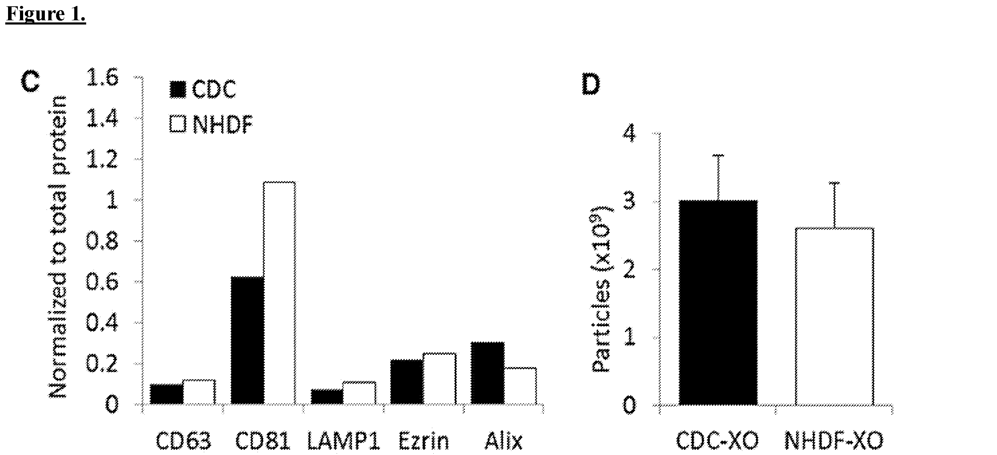

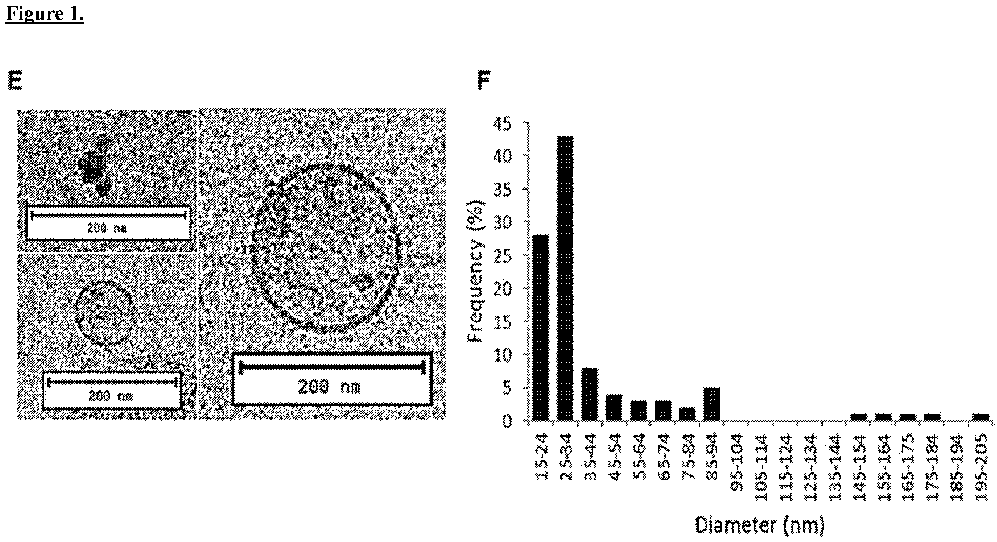

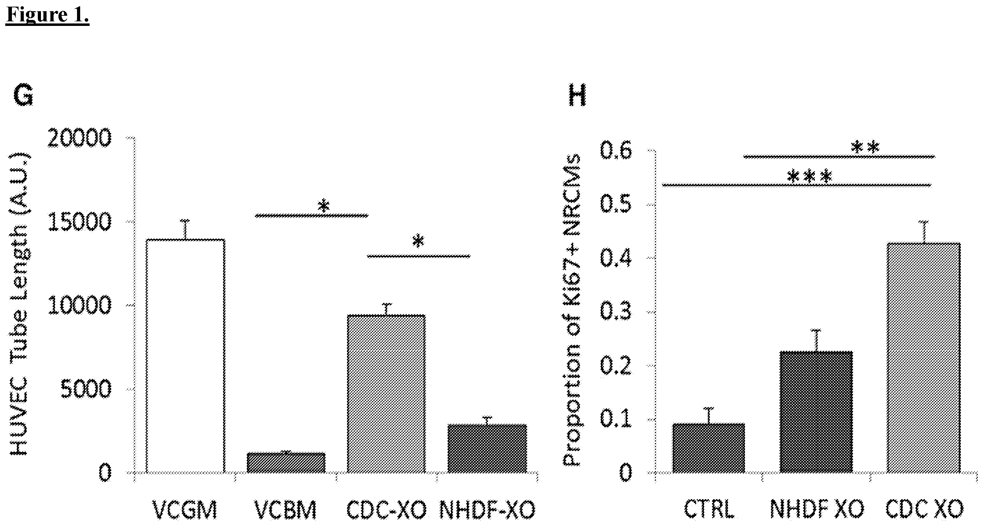

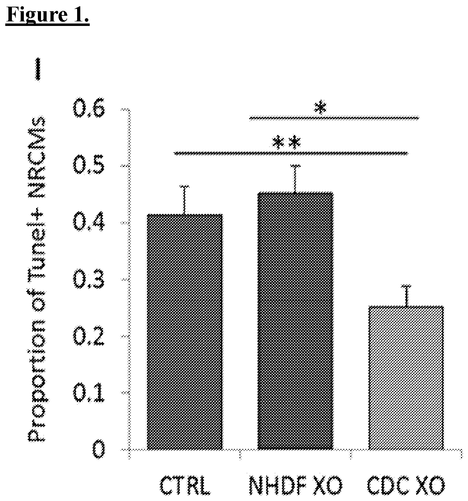

**Figure 1****. Characterization of Cardiosphere-Derived Cells Exosomes. (A)** RNA content measured in exosome pellets derived from cardiosphere-derived cells (CDCs) and normal human dermal fibroblasts (NHDF) compared to conditioned media from both samples. **(B)** Exosomal RNA is protected from RNase degradation by the lipid bilayer membrane of exosomes. Exosome pellets were treated with RNase A in the presence or absence of triton to assess protection from RNase-mediated degradation. All samples were treated with proteinase K to dissociate complexes which might otherwise shield RNA (n = 4 technical replicates). **(C)** CDC and NHDF exosomes express ubiquitous exosome markers as revealed by mass spectrometry. **(D)** Exosome quantification from CDC- and NHDF-conditioned media based on expression of the conserved CD63 marker (n = 3 technical repeats). **(E)** Exosomes isolated from CDCs Visualized by transmission electron microscopy. Three populations (by size) are illustrated. **(F)** Size distribution of exosomes derived from CDCs, measured from transmission electron microscopic images; n = 100 exosomes counted. CDC exosomes enhance angiogenesis and promote neonatal rat cardiomyocyte (NRCM) survival and proliferation *in vitro.*

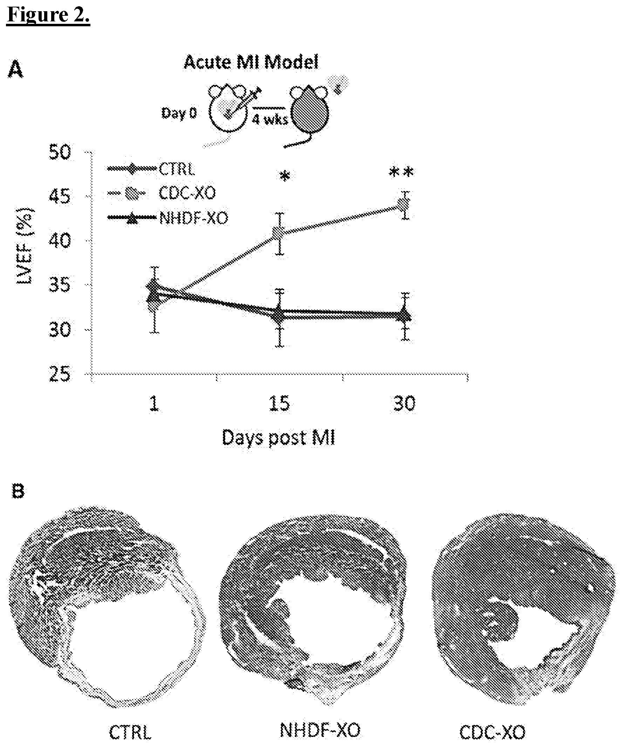

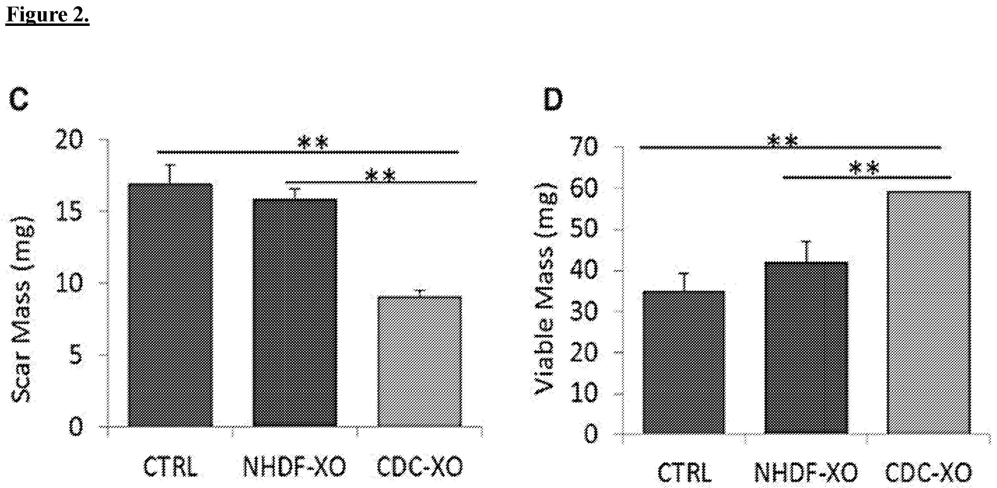

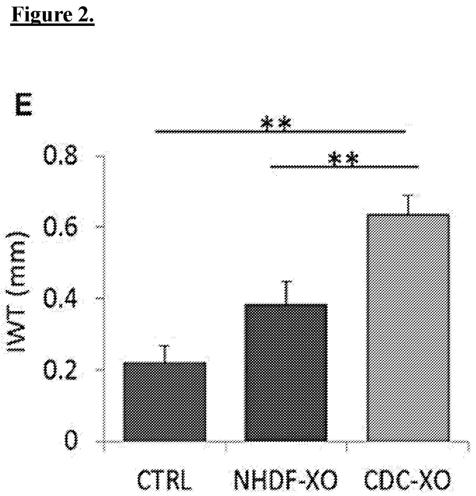

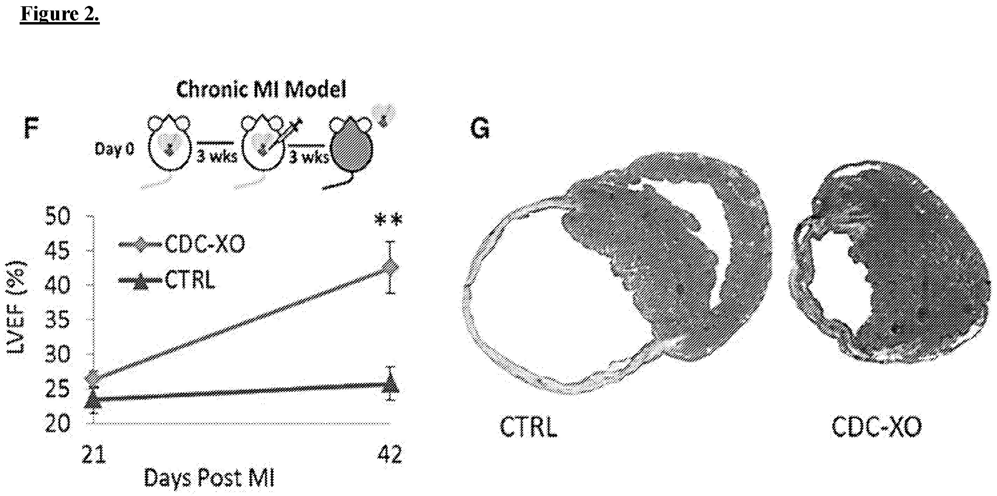

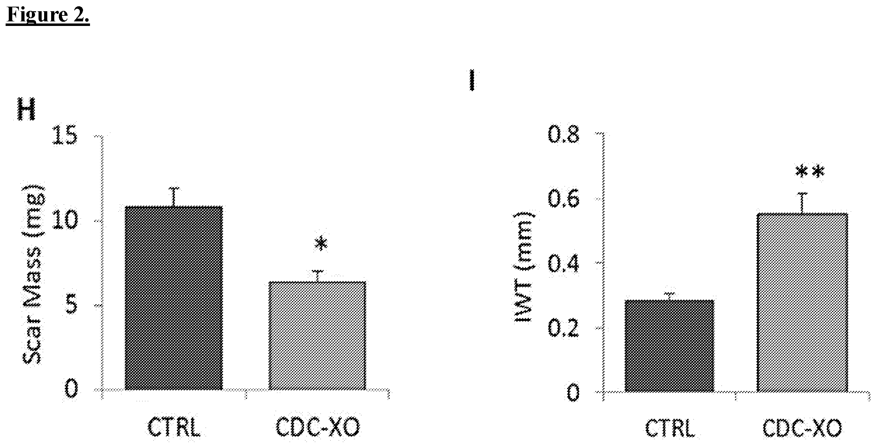

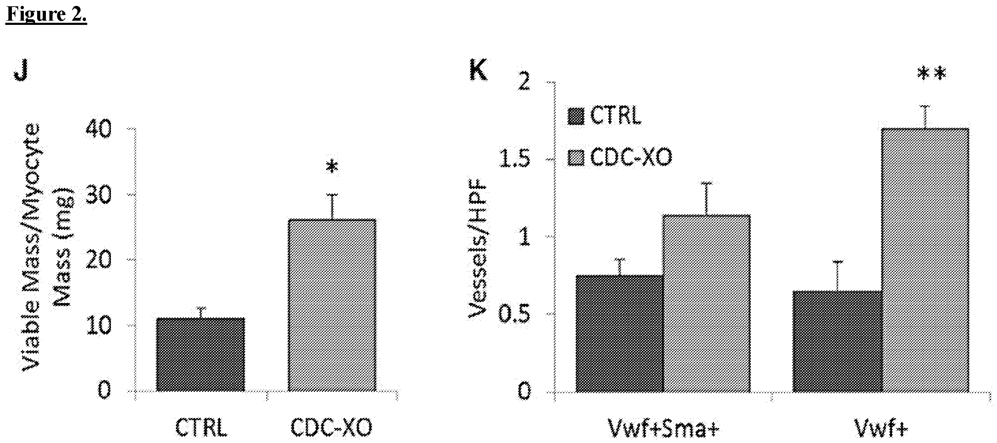

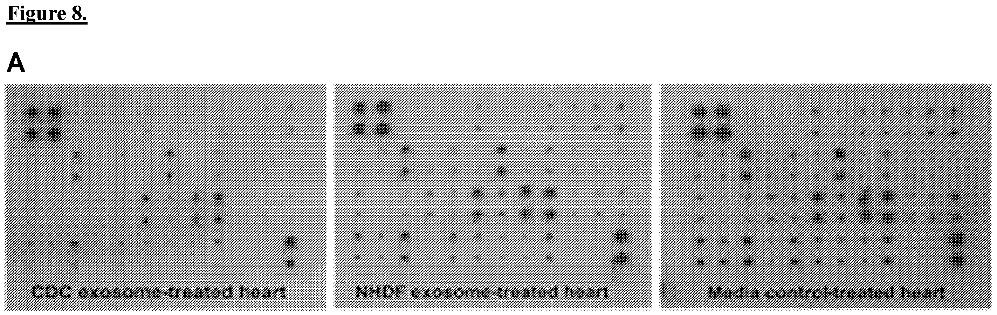

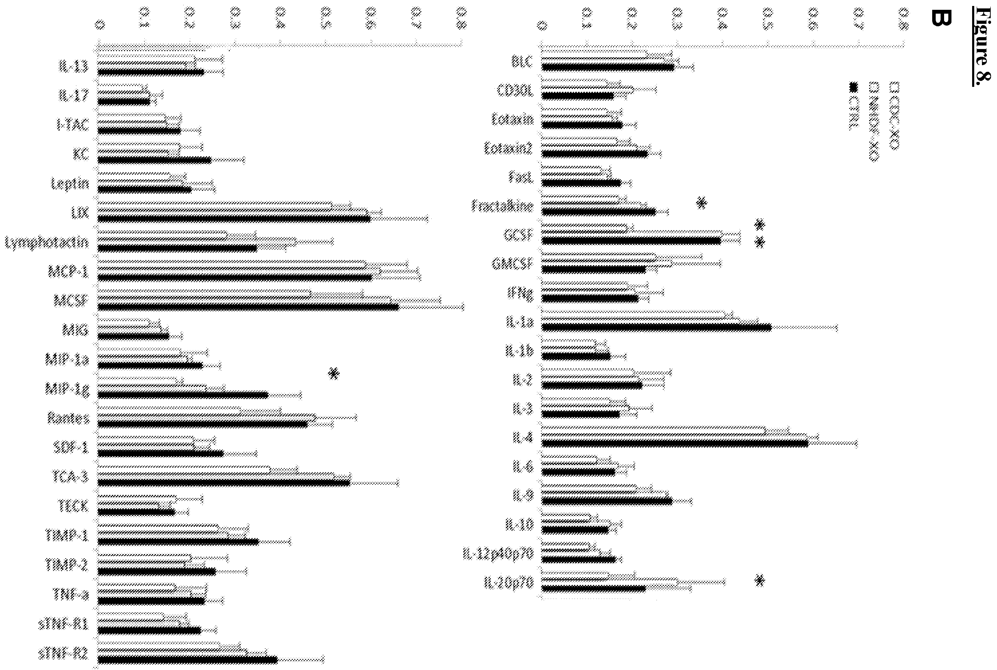

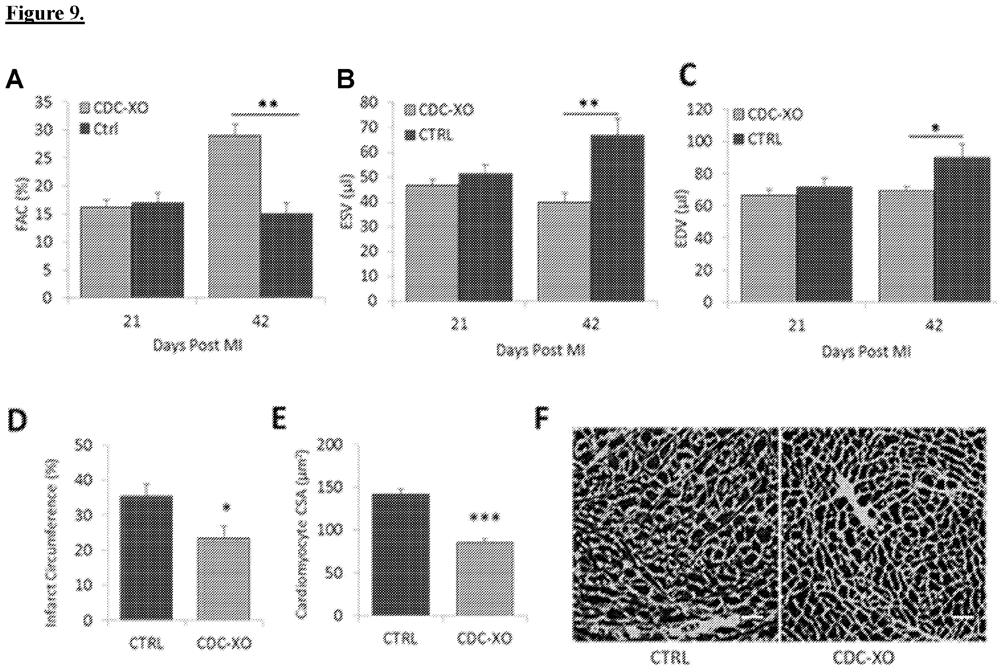

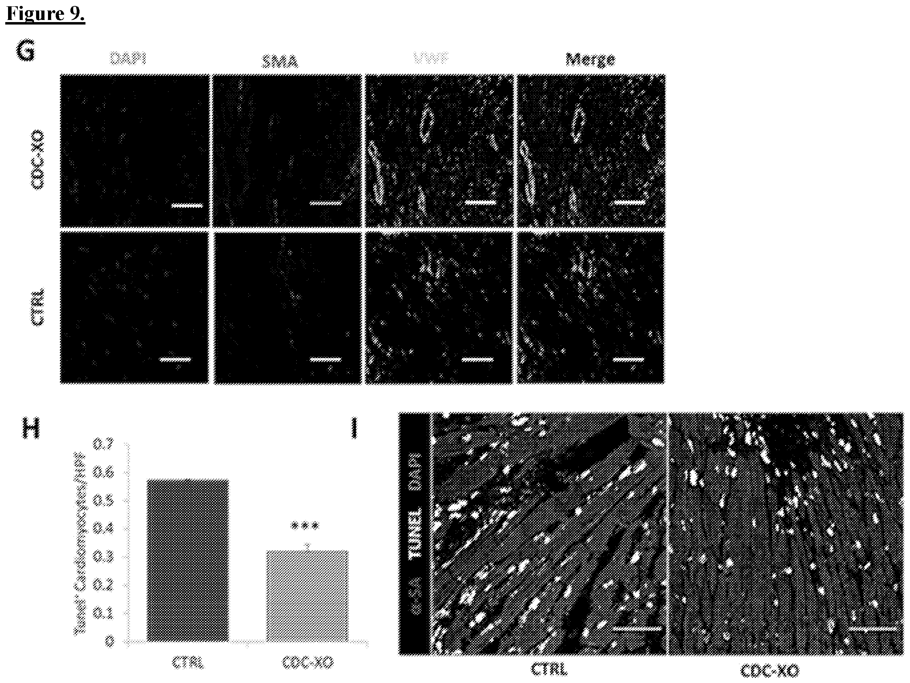

**Figure 2****. CDC Exosomes Produce Structural and Functional Benefits in Mouse Hearts after MI. (A)** In the acute model, SCID Beige mice underwent MI and hearts were injected with CDC exosomes, NHDF exosomes, or vehicle (control). Animals (n = 8 animals per group) were echoed at days 1, 15, and 30 and were then sacrificed for histological analysis. CDC exosomes increase left ventricular ejection fraction (LVEF). **(B-E)** Structural benefits of CDC exosomes. Representative Masson's trichrome-stained sections of hearts from each of the three groups **(B)** and pooled morphometric analysis **(C-E;** n = 3 hearts per group) reveal decreased scar and increased viable mass in hearts injected with CDC exosomes. **(F)** In the chronic model, 3-month-old SCID Beige mice (n = 6 animals per group) underwent MI. Three weeks later, animals were injected intramyocardially with CDC exosomes or control. Functional measurements were taken 24 hr before injection (day 21) and 3 weeks later (day 42), after which animals were sacrificed for histological analysis. **(G-J)** As in the acute MI model, CDC exosomes produce functional and structural benefits in mouse hearts (n = 4 hearts per group) in a model of chronic MI. *p < 0.05, **p < 0.01, and ***p < 0.001 using one-way ANOVA with Tukey's post hoc test and two-tailed Student's t test. Data are represented as mean and SEM. See also Figures 8 and 9.

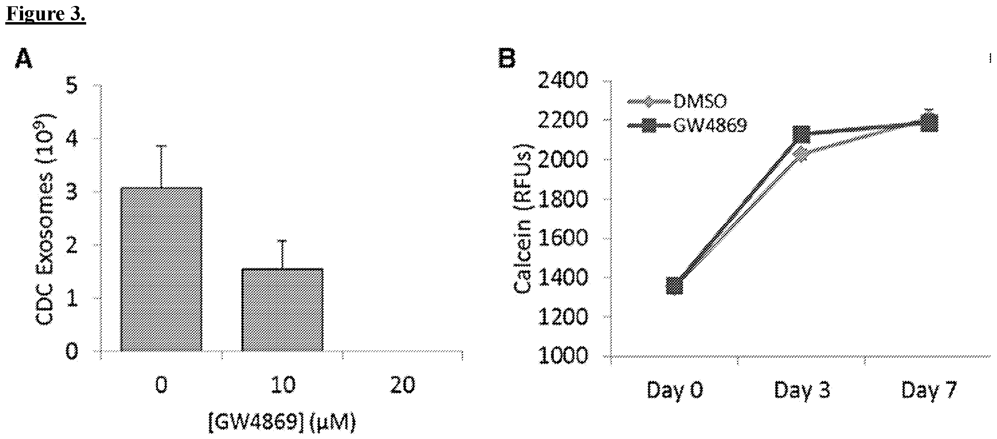

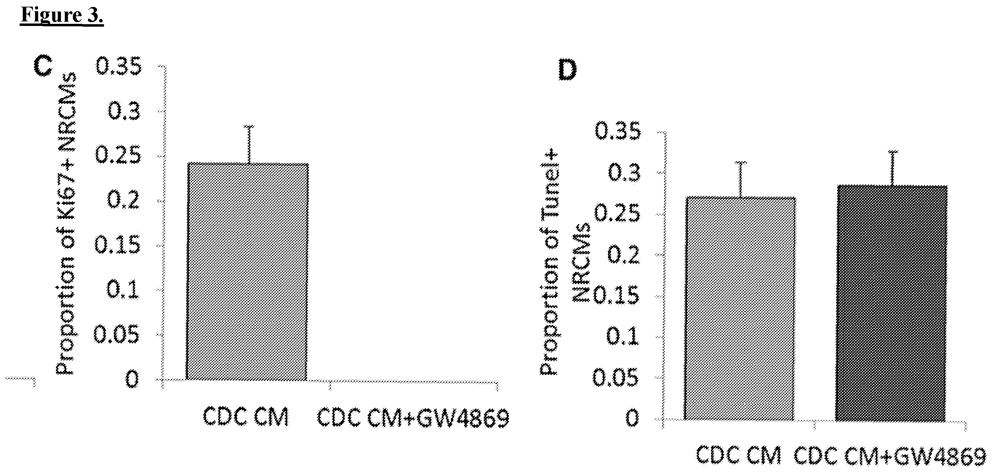

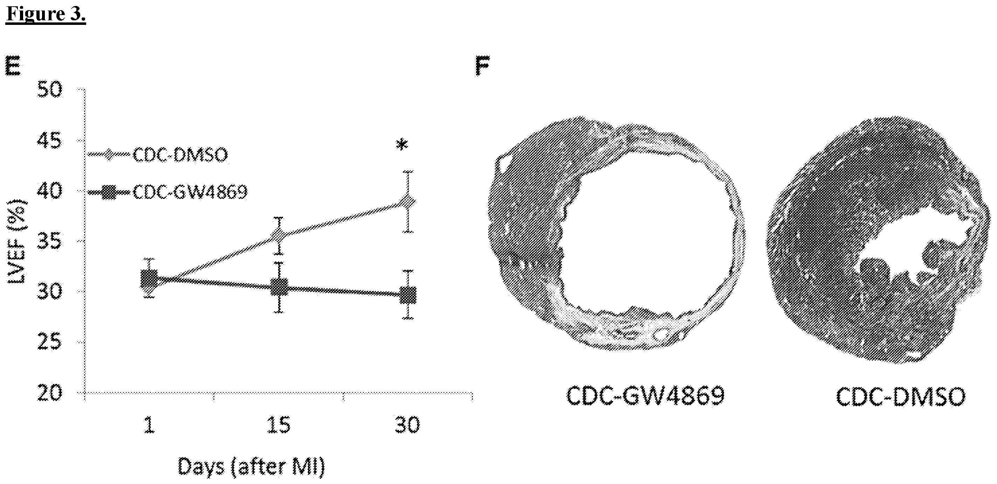

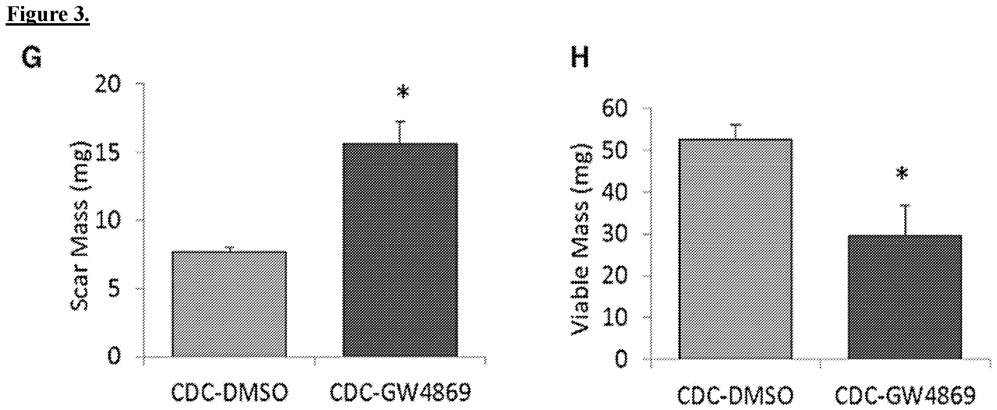

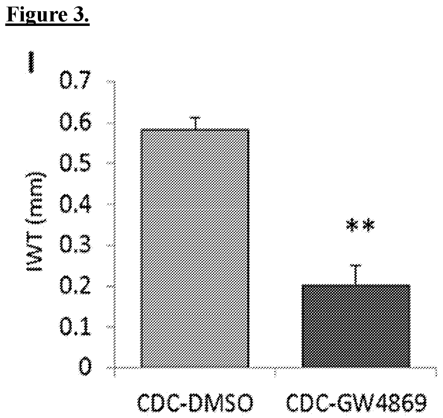

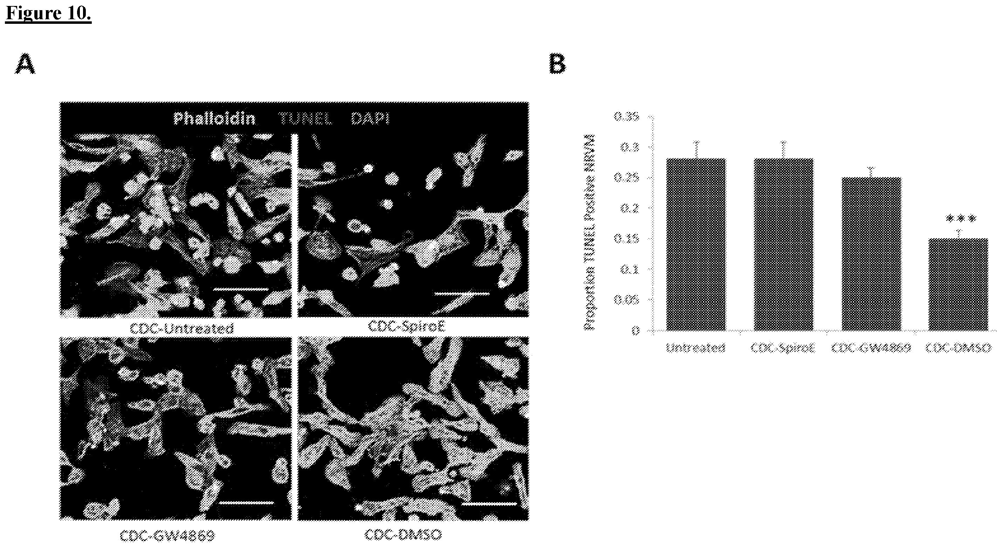

**Figure 3****. Exosome Inhibition Attenuates CDC-Mediated Benefits. (A)** GW4869 inhibited exosome production in CDCs in a dose-dependent manner (n = 3 technical replicates). **(B)** GW4869 does not affect CDC viability as shown by calcein assay of CDCs treated with GW4869 or its solvent DMSO (n = 4 technical replicates). **(C and D)** Neonatal rat cardiomyocytes (NRCMs) were cultured on chamber slides and treated with media conditioned by CDCs exposed to either GW4869 or DMSO. NRCMs were then treated with culture media and after 5 days, slides were stained for Ki67 and TUNEL to assess proliferation and apoptosis (n = 4 technical replicates per group). **(E)** Pooled data for left ventricular ejection fraction (n = 8 animals per group). **(F-I)** Representative Masson's trichrome-stained heart sections from two groups **(F)** and pooled morphometric analysis (G-I; n = 4 hearts per group) reveal impairment of CDC-mediated benefit as evident in pooled data for scar mass, viable mass, and infarct wall thickness (IWT) in hearts injected with GW869-treated CDCs. *p < 0.05 and **p < 0.01 using Student's t test. Data are represented as mean and SEM. See also Figure 10.

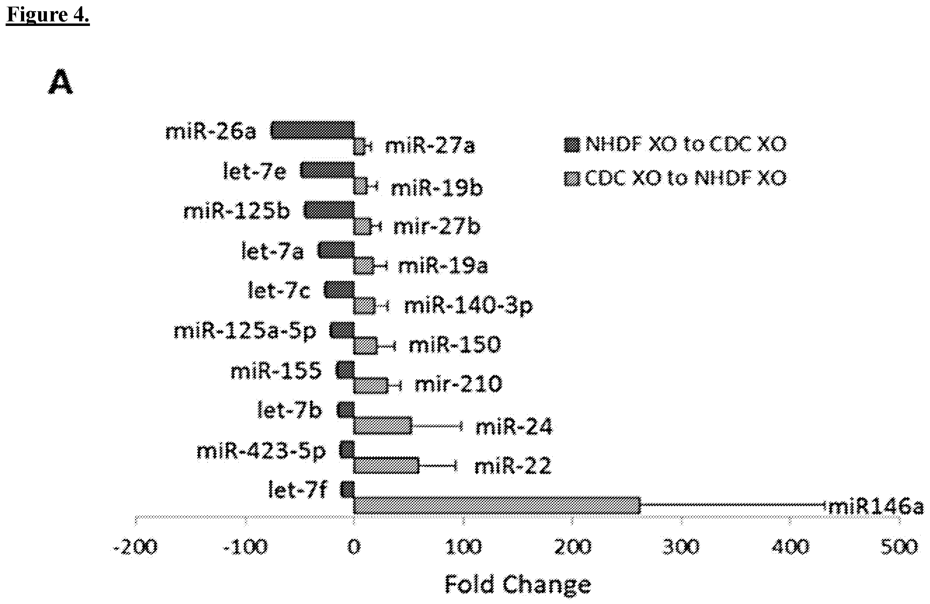

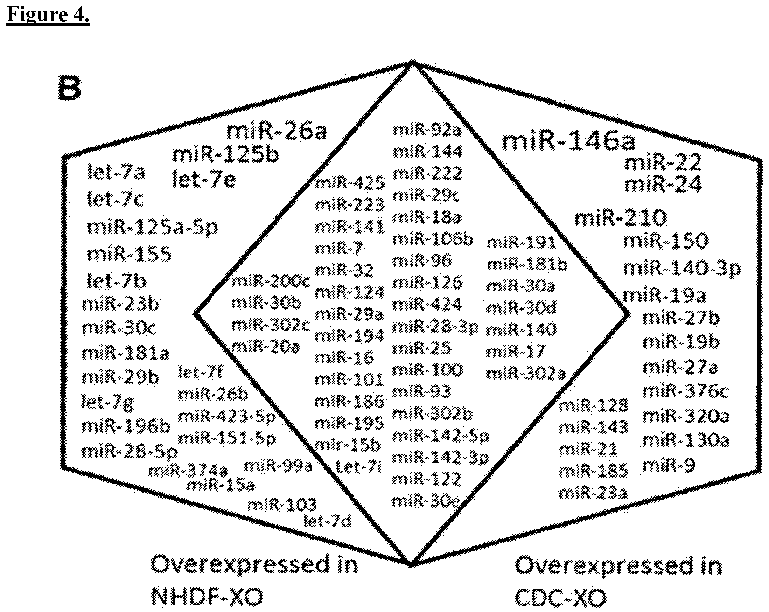

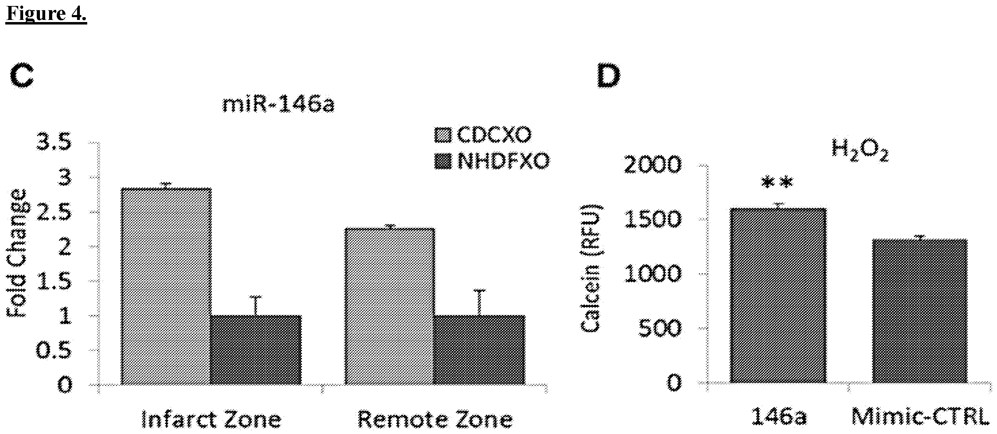

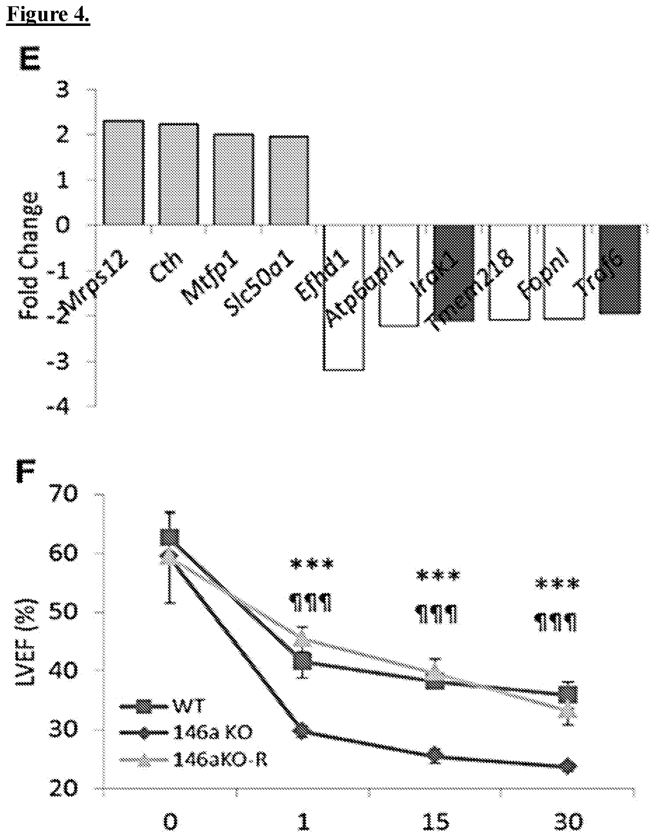

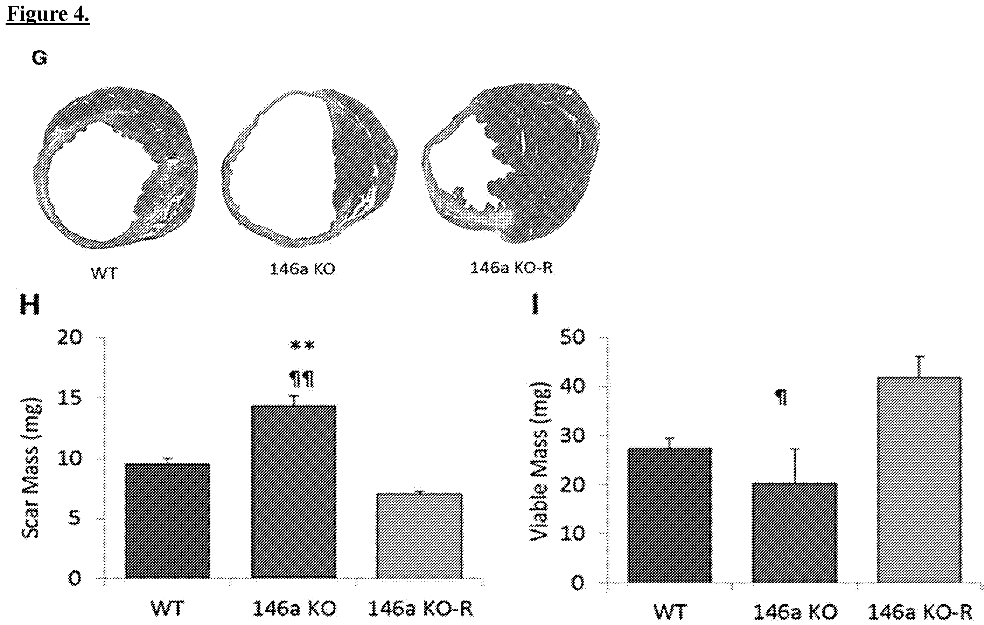

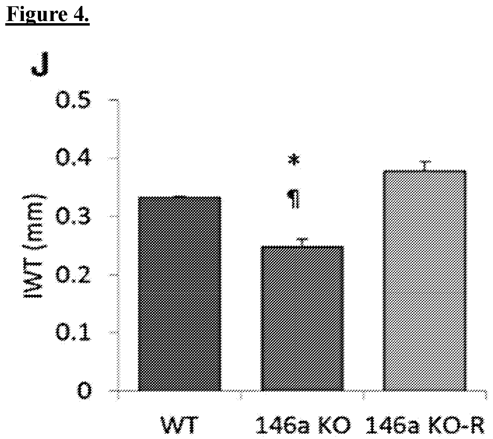

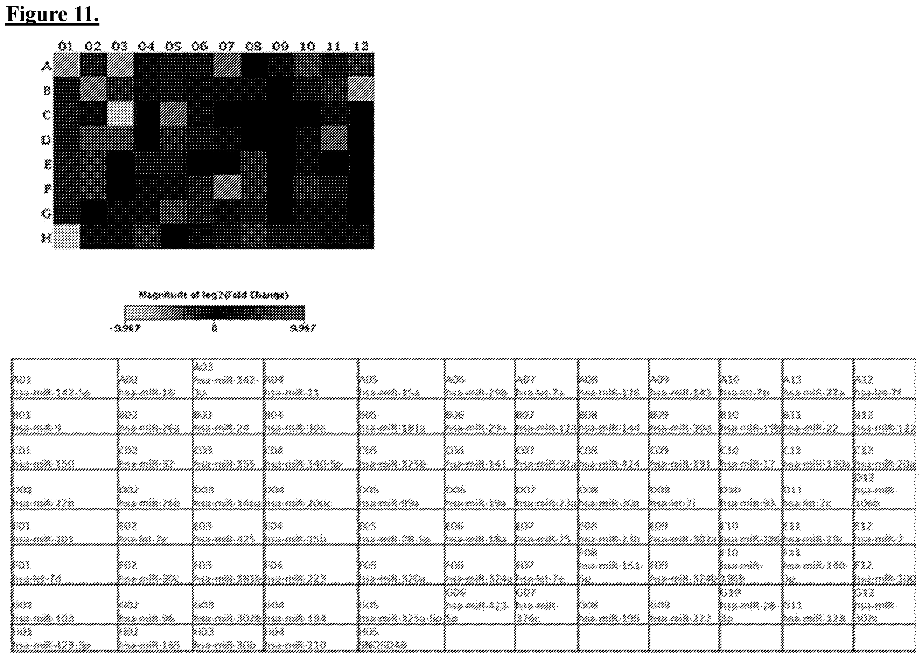

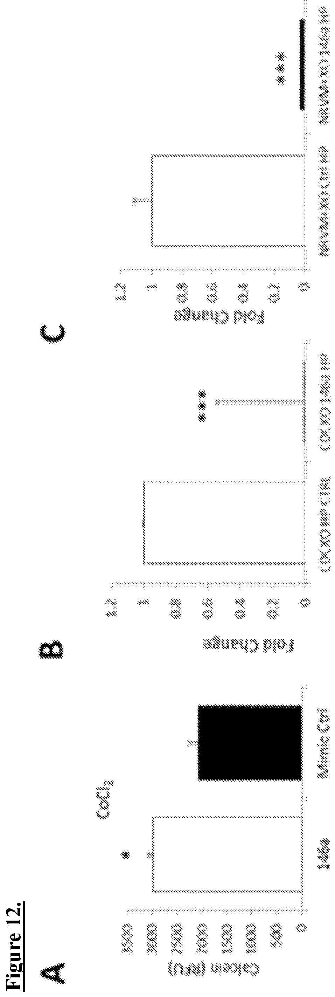

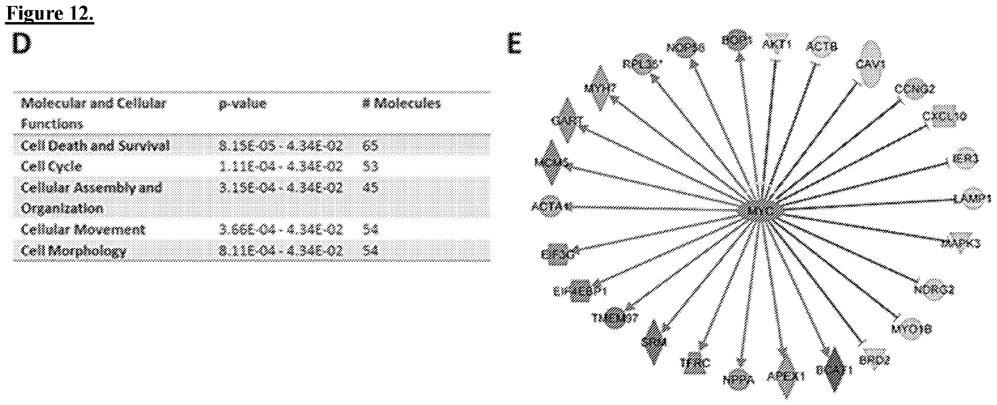

**Figure 4****. miR-146a Is Highly Enriched in CDC Exosomes and Confers Therapeutic Benefit In Vitro and In Vivo. (A)** Fold changes of microRNA abundance in CDC exosomes compared to NHDF exosomes (n = 4 independent experiments). Total RNA(including microRNAs) was isolated from CDC exosomes and NHDF exosomes. qRT-PCR was performed on an microRNA array. **(B)** Venn diagram showing the variable microRNA profile between CDC and NHDF exosomes. Font size reflects the magnitude of differential expression of each microRNA. **(C)** Infarcted mouse hearts treated with CDC-derived exosomes have elevated levels of miR-146a compared to NHDF exosome-treated hearts(n = 2 animals per group, three technical replicates per group). **(D)** miR-146a protects stressed neonatal rat cardiomyocytes. Cardiomyocytes were pretreated with 80 nM miR-146a mimic or mimic control then exposed to 100 mM hydrogen peroxide for 2.5 hr in serum-free media (n = 4 technical replicates; neonatal rat cardiomyocytes under study were derived from 20 to 30 rat pups from three different mothers). **(E)** Microarray data showing fold differences in mRNA abundance between miR-146a and mimic-control treated cardiomyocytes. miR-146asuppresses Irak1 and Traf6 in stressed neonatal rat cardiomyocytes. **(F)** miR-146a-deficient animals have severely impaired cardiac function and structure following acute MI. Pooled data for left ventricular ejection fraction (n = 8 animals per group). **(G-J)** Representative Masson's trichrome-stained sections of hearts from three groups **(G)** and pooled morphometric analysis (H-J; n = 4 hearts per group) reveal impairment of CDC-mediated benefit as evident in pooled data for scar mass, viable mass, and infarct wall thickness (IWT) in hearts injected with GW869-treated CDCs. *p < 0.05, {p < 0.05; **p < 0.01, and {{ p < 0.01 using Student's t test (*KO versus WT; {KO versus KO-R). Data are represented as mean and SEM. See also Figures 11 and 12.

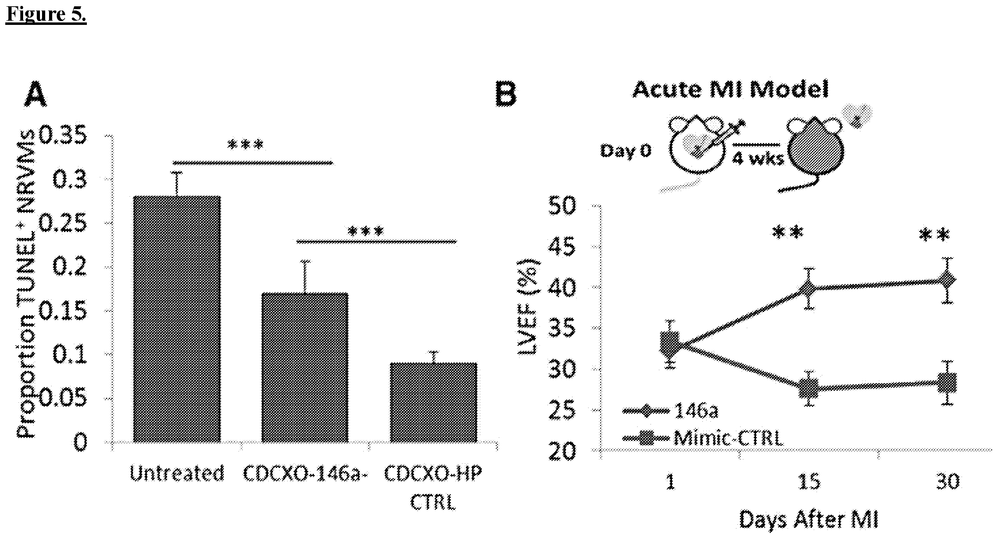

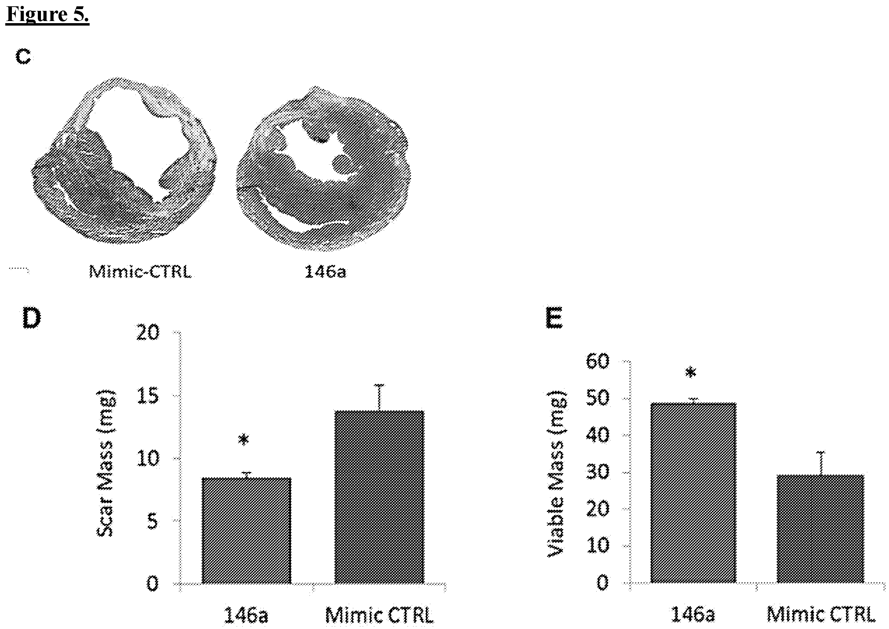

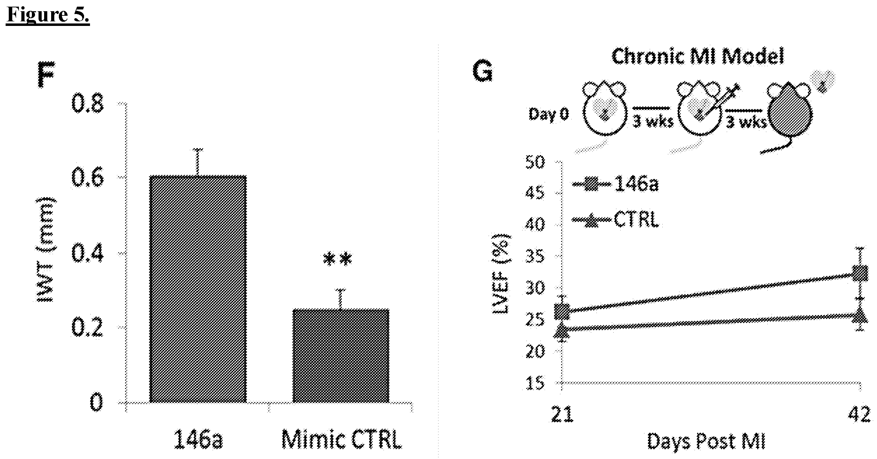

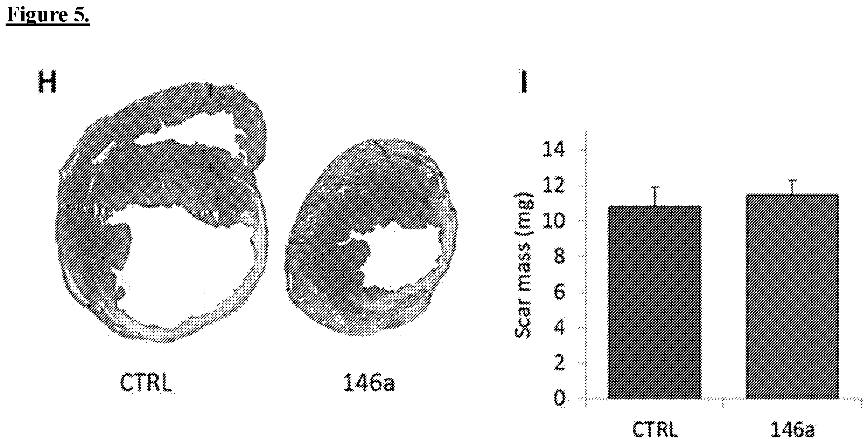

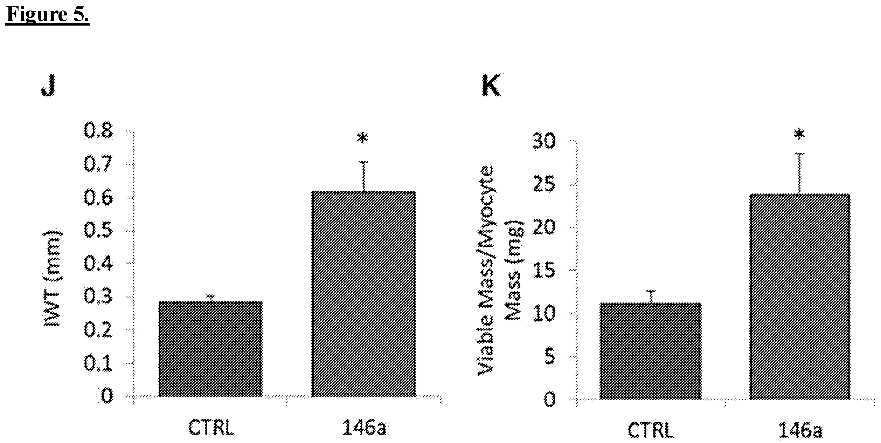

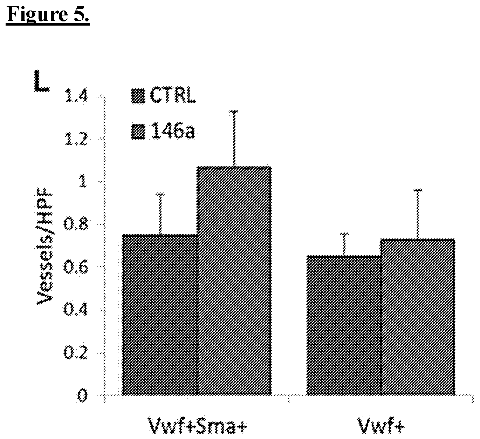

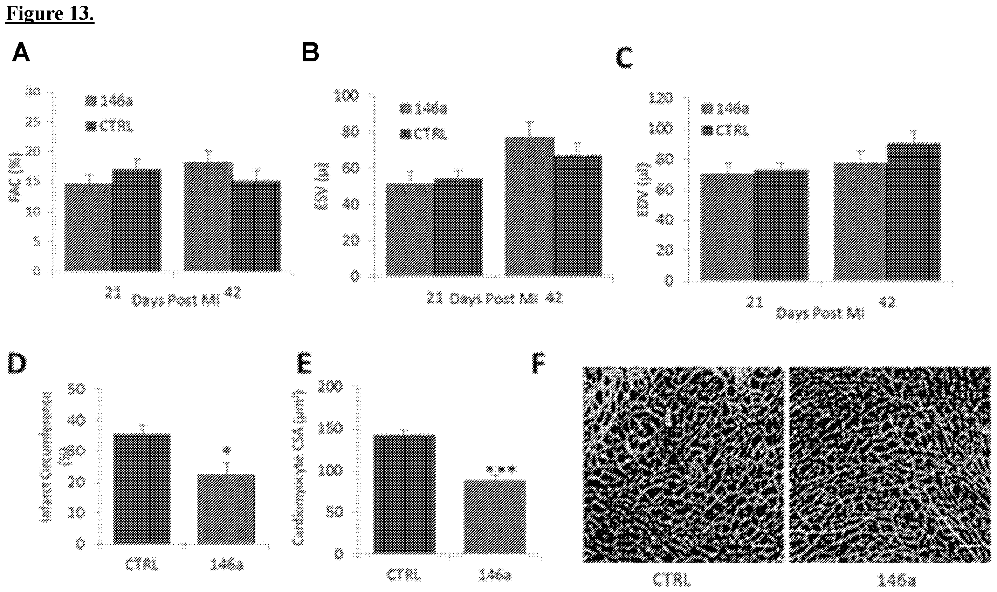

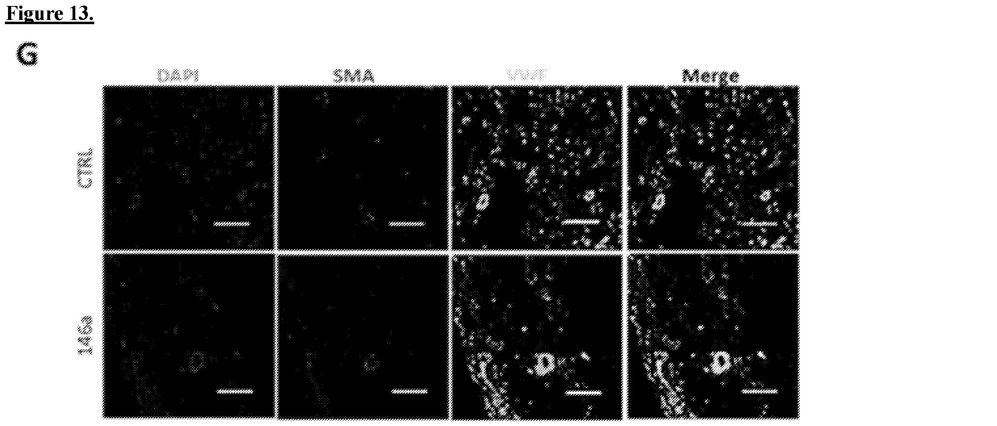

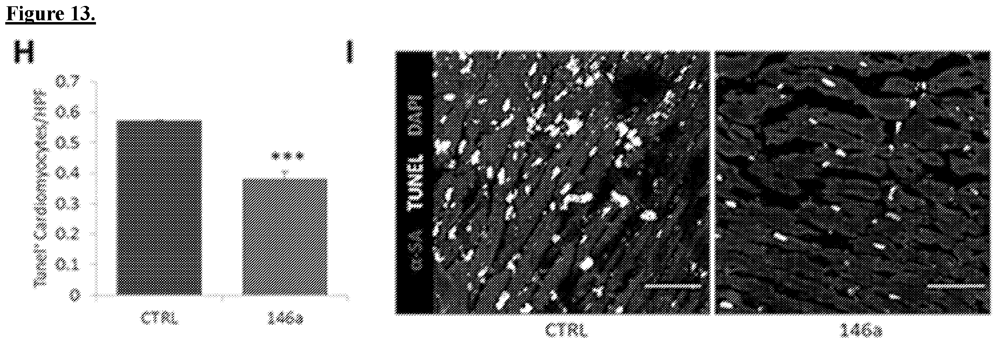

**Figure 5****. miR-146a Improves Systolic Function in Acute and Chronic Mouse Models of MI. (A)** miR-146a knockdown in CDC exosomes diminishes their capacity to protect stressed NRVMs *in vitro* (n = three technical replicates; neonatal rat cardiomyocytes were derived from 20 to 30 rat pups from three different mothers). CDCs were transfected with either a miR-146a inhibitor or a hairpin control with a sequence based on Caenorhabditis elegans microRNAs (HP-CTRL). **(B-F)** Acute MI protocol data. Time course of left ventricular ejection fraction (n = 6 animals per group; B). Representative Masson's trichrome-stained sections of hearts from each of the two groups **(C)** and pooled morphometric analysis (n = 4 hearts per group) reveal decreased scar mass, increased viable mass, and increased infarct wall thickness in animals treated with miR-146a compared to microRNA control **(D-F). (G-L)** miR-146a reproduces some of the structural and functional benefits seen in CDC-exosome-treated hearts in a mouse model of chronic MI (miR-146a mimic or mimic control injected on day 21 after MI; n = 6 animals per group). Three weeks later (day 42), animals treated with miR-146a showed comparable cardiac function to control **(G)** but adverse remodeling was significantly attenuated **(H).** Scar mass was also similar **(I).** Viability and infarct wall thickness were significant structural benefits **(J and K),** but scar mass was not reduced **(I).** Analysis was done using Student's t test; *p < 0.05, **p < 0.01, and ***p < 0.001. Data are represented as mean and SEM. See also Figures 12 and 13.

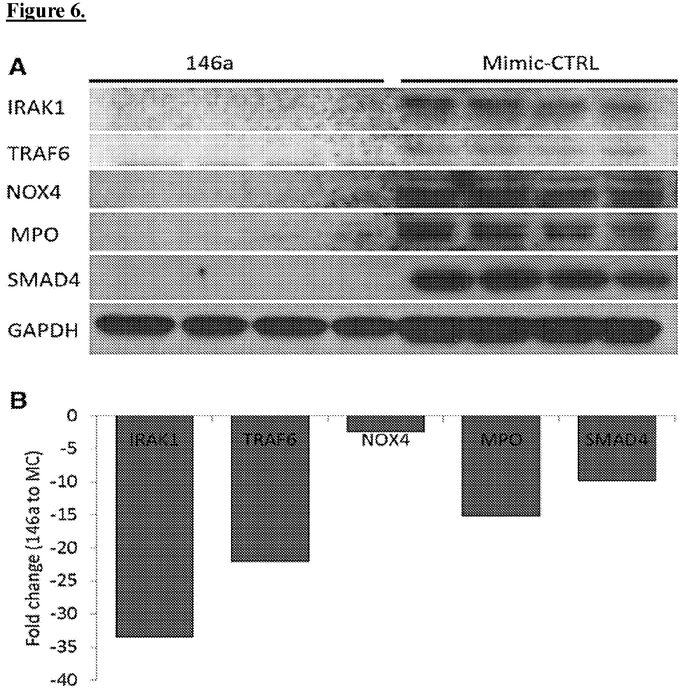

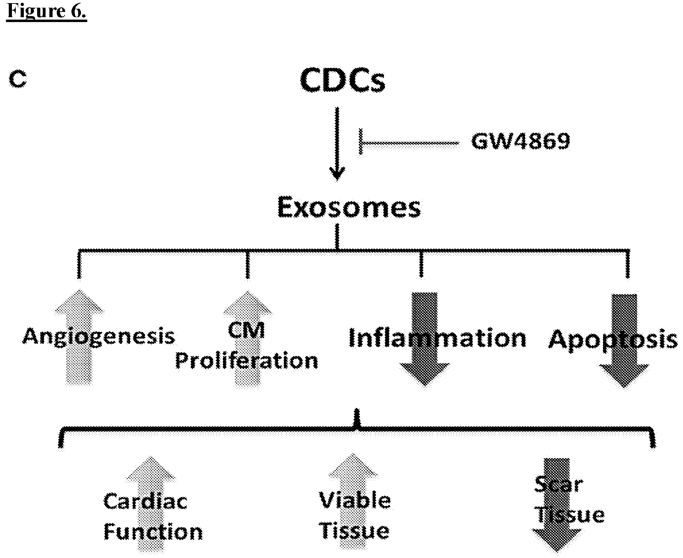

**Figure 6****. miR-146a Targets Genes Involved in MI Pathology. (A and B)** Downregulation of known miR-146a targets in chronic MI mouse hearts 7 days after injection of miR-146a or mimic control. **(A)** Western blot for IRAK, TRAF6, SMAD4, NOX4, and MPO (a marker of neutrophil infiltration). Each well is loaded with protein lysate pooled from two hearts per group, so that the blot represents pooled samples of two animals each with n = 4 technical replicates. **(B)** Densitometric analysis of blot in **(A)** normalized to glyceraldehyde 3-phosphate dehydrogenase (GAPDH). **(C)** Schematic of the Inventors' working hypothesis. CDCs promote functional and structural benefits in the injured myocardium in a primarily paracrine manner. CDCs secrete exosomes that contain microRNAs that mediate benefits in the injured myocardium. These microRNAs target transcripts in the various compartments of the myocardium, which ultimately leads to increased cardiac function, increases in viable tissue, and decrease of scar after MI.

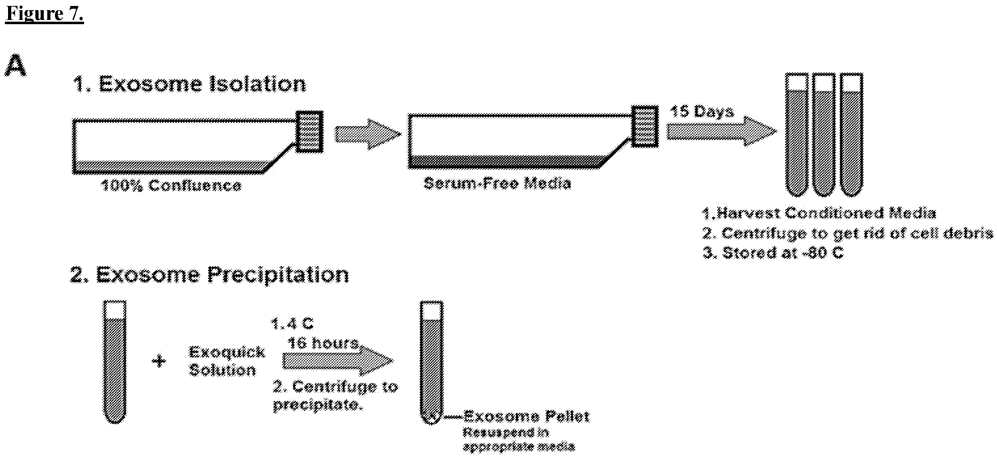

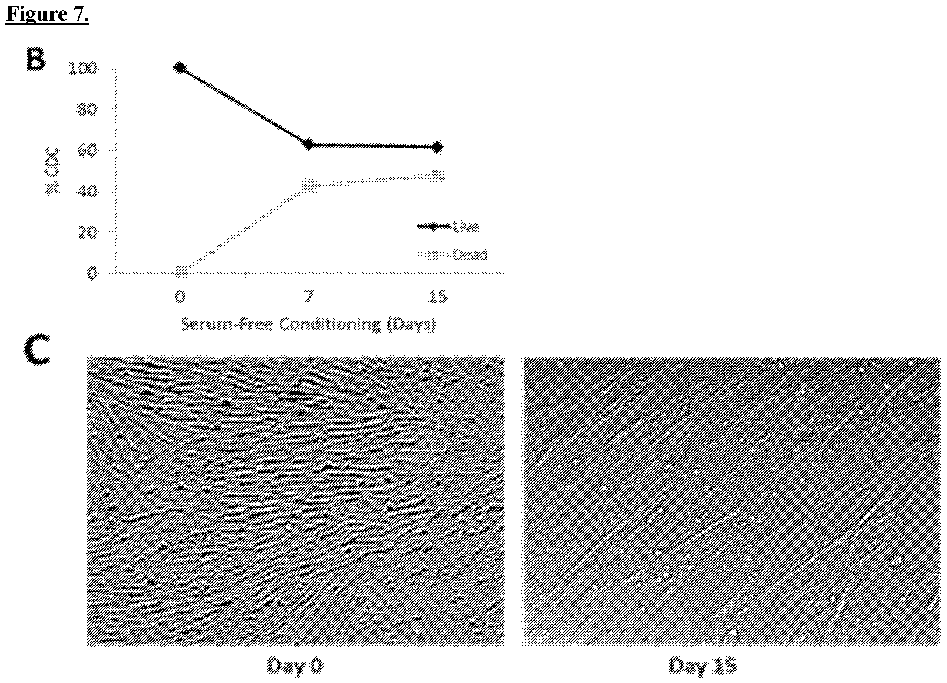

**Figure 7****. Isolation of Exosomes from CDCs. (A)** Graphical representation of exosome isolation and purification for exosomes. **(B)** Cell viability (calcein) and cell death (Ethidium homodimcr-1) assay performed on CDCs over the 15 day serum-free conditioning period. **(C)** Representative images of CDCs before and after serum-free conditioning.
**Figure 8****. CDC Exosomes Reduce Inflammation In A Mouse Model Of Acute MI. (A)** Representative protein arrays for 40 pro-inflammatory markers. **(B)** Quantification of inflammatory proteins in mouse hearts treated with CDC-exosomes, NHDF-exosomes, or control. Data comes from three mouse hearts per group. Analysis was done using one-way ANOVA (95% CI) (n=3 hearts per group). Data represented as mean and standard error of the mean.
**Figure 9****. CDC-Exosomes Produce Structural And Functional Benefits In Mouse Hearts After MI.** CDC-exosomes stimulate functional improvement and attenuate adverse remodeling and cardiac hypertrophy in a mouse model of chronic MI. Animals treated with CDC-exosomes showed significant functional improvement compared to control as shown by fractional area change **(A),** end systolic volume **(B)** and end diastolic volume **(C)** (A-C, n=6 animals per group). Animals treated with CDCexosomes also showed structural improvements as noted as seen in percent of the circumference of tissue sections that are scar **(D),** decreased cardiomyocyte hypertrophy **(E)** as measured by staining with wheat germ agglutinin and DAPI **(F)** and increased angiogenesis in the infarct zone **(G).** Less cardiomyocyte death was observed in the border zone of CDC-exosome-treated animals compared to control. **(H, I)** (D-I n=4 hearts per group) *P<0.05, **P<0.01, ***P<0.001. using Student's t test, all scale bars represent 50 µm. Data represented as mean and standard error of the mean.
**Figure 10****. Inhibition Of Exosome Secretion In CDCs Diminishes The Protective Effects Of CDCs In Vitro.** Neonatal rat ventricular myocytes were stressed with 50 µM H2O2 for 15 minutes followed by trans-well treatment with CDCs pre-treated with 5 µM of Spiroepoxide, 20 µM of GW4869, or vehicle (DMSO). **(A)** Cell death was measured using TUNEL staining (red), Phalloidin (green), and DAPI (blue). **(B)** Pooled data of the four groups represented as proportion of TUNEL positive cardiomyocyte nuclei of total cells counted (n=3 technical replicates from neonatal rat cardiomyocytes derived from 20-30 rat pups from 3 different mothers) **(B).** *P<0.05, **P<0.01, ***P<0.001 using Student's t test, all scale bars represent 50 µm. Data represented as mean and standard error of the mean.
**Figure 11****. Heat Map Of Mir PCR Array Identifies Mir-146a As The Most Differentially Expressed microRNA.** Heat map showing fold regulation differential abundance data for transcripts between CDC exosomes and NHDF exosomes overlaid onto the PCR Array plate layout.
**Figure 12****. miR-146a Protects Stressed Neonatal Rat Cardiomyocytes. (A)** Cardiomyocytes were pre-treated with 80 nM miR-146a mimic or mimic-control then exposed to 5 mM cobalt chloride for 2 hours (n=4 technical replicates per group of neonatal rat cardiomyocytes derived from 20-30 rat pups from 3 different mothers) **(B, C)** CDC exosomes derived from CDCs transfected with mir-146a hairpin inhibitor. Exosomes were derived from conditioned media and mir146a knockdown confirmed by qPCR in exosomes. **(C)** Decreased levels of mir-146a in NRVMs treated with 146a-free exosomes compared to control (n=3 technical replicates per group of neonatal rat cardiomyocytes derived from 20-30 rat pups from 3 different mothers). Pathway analysis derived from transcriptome data showing affected pathways and **(B)** Pathway depiction showing MYC activation as a putative hub, based on microarray data analysis.
**Figure 13****. miR-146a Reproduces Some But Not All The Effects Of CDC-Exosomes.** miR-146a attenuates adverse remodeling and cardiac hypertrophy in a mouse model of chronic MI. **(A,C)** Animals treated with CDC-exosomes showed no significant functional improvement compared to control as shown by fractional area change **(A)** , end systolic volume **(B)** and end diastolic volume **(C)** (AC, n= 6 animals per group). Structural improvements however were noted as seen in percent of the circumference of tissue sections that are scar **(D),** and decreased cardiomyocyte hypertrophy **(E)** as measured by staining with wheat germ agglutinin and DAPI. No differences in angiogenesis were observed between the two groups **(G).** Less cardiomyocyte death was observed in the border zone of mir 146a-treated animals compared to control. **(H, I)** (D-I, n=4 hearts per group) *P<0.05, **P<0.01, ***P<0.001 using Student's t test, all scale bars represent 50 µm. Data represented as mean and standard error of the mean.

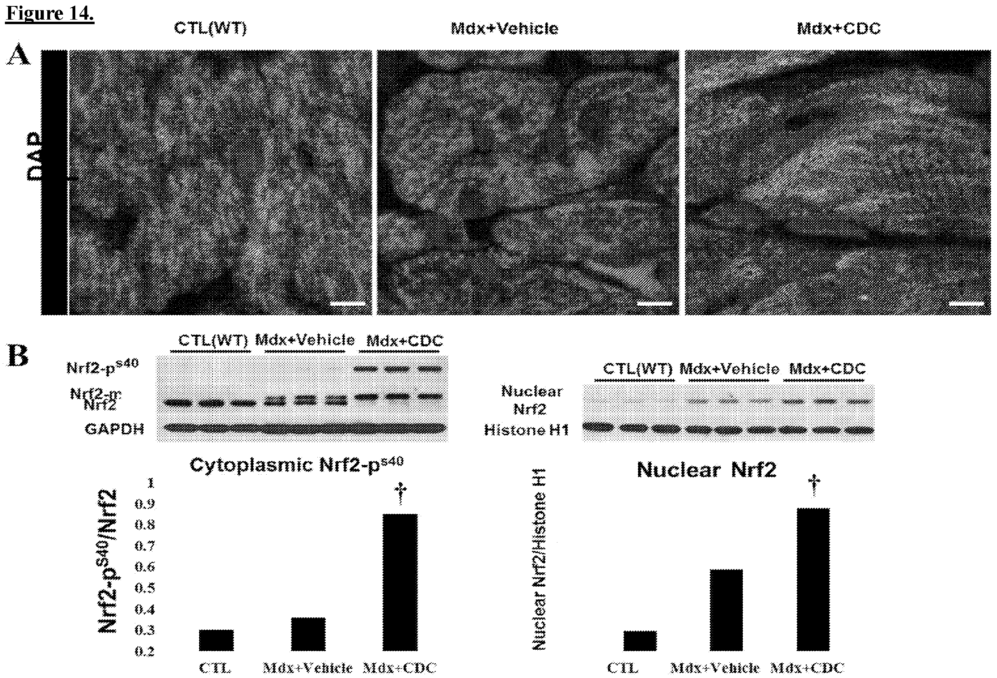

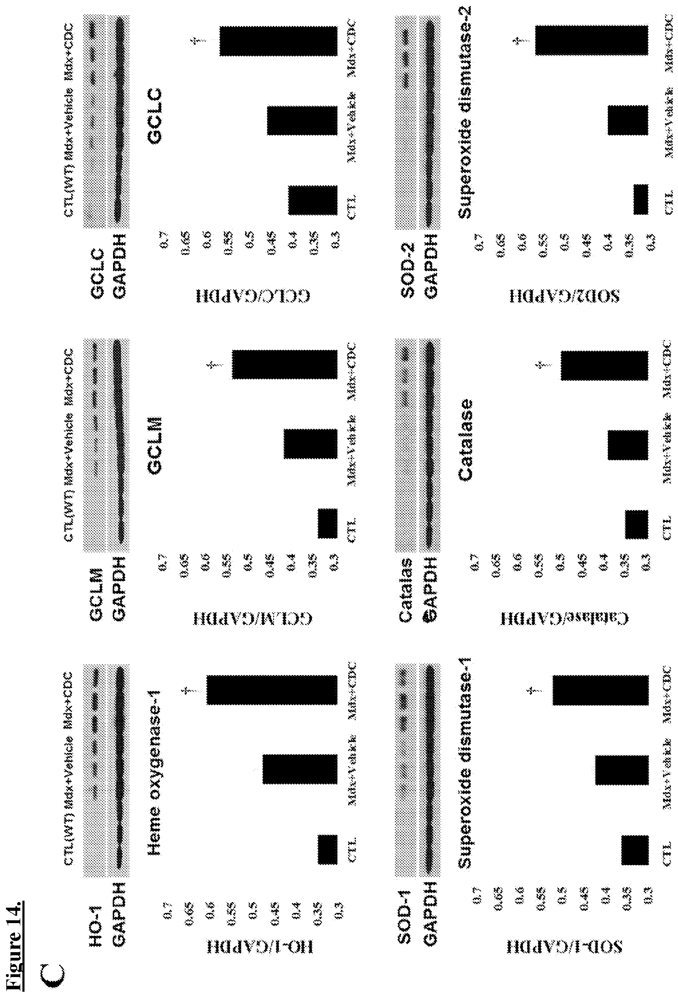

**Figure 14****. CDC Treatment Heightened Activity Of Nrf2 Antioxidative Pathway And Increased Expression Of Nrf2 Downstream Gene Products. (A)** Representative immunohistochemical images depicting Nrf2 in the mdx mouse hearts three weeks after treatment with vehicle (Mdx+Vehicle) or CDC (Mdx+CDC). Age-matched wild type mice (CTL) served as control. **(B)** and **(C):** Representative western blots and pool data demonstrating cytoplasmic and nuclear Nrf2 content (B) and the protein abundance of Nrf2 downstream-gene products **(C):** HO-1 , modulatory (GCLM) and catalytic (GCLC) subunits of glutamate-cysteine ligase, SOD-1, catalase and SOD-2 in the mdx mouse hearts 3 weeks after treatment with vehicle or CDC. The experimental mice were recruited at 10 months of age. Marked increase in phosphorylated Nrf2 (Nrf2-ps40) in the cytoplasm was accompanied with augmented nuclear Nrf2 content and increased expression of Nrf2 downstream gene products in the CDC-treated mdx mice **(B,C).** Data are means ± SEM; n = 7 in each group. †P < 0.05 vs. Mdx+Vehicle and control (CTL; wild type); Scale bars: 5 µm.

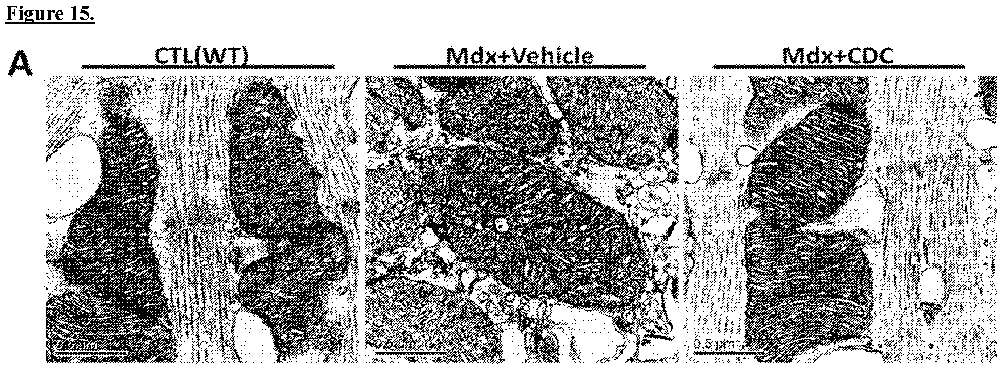

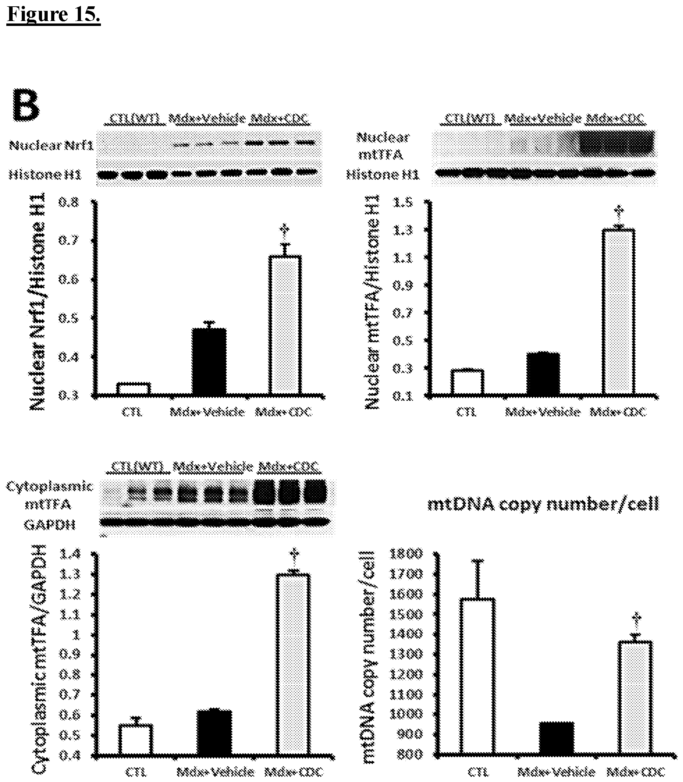

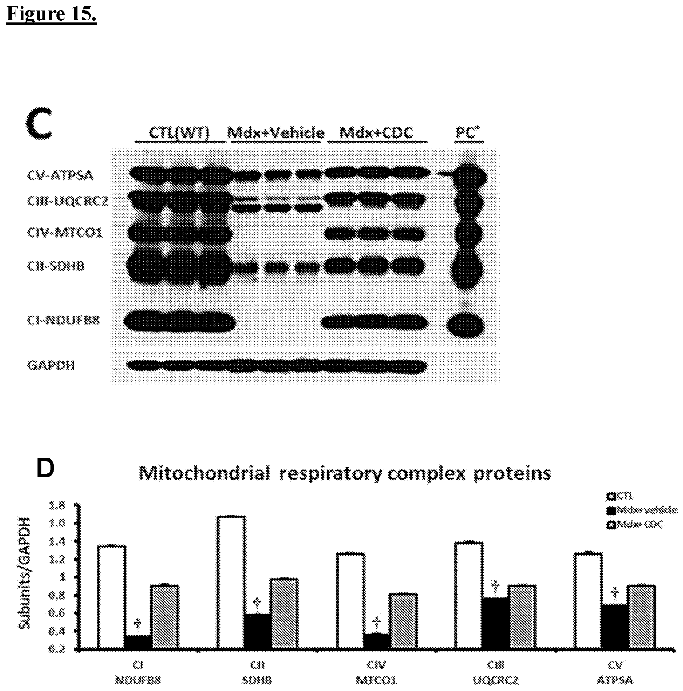

**Figure 15****. CDC Treatment Markedly Restored Mitochondrial Structure And Content And, Enhanced Expression Of Respiratory Chain Subunits In The Heart Tissue Of Mdx Mice. (A):** Representative images of transmission electron microscopy of cardiomyocyte mitochondria in mdx mice 3 weeks after treatment with vehicle (Mdx+Vehicle) or CDC (Mdx+CDC). Elongated mitochondria with altered (rounded/tubular) crista were predominant in the cardiomyocyte of mdx mice at 10 months of age. CDC treatment significantly restored cardiomyocyte mitochondrial size and crista structure (lamellar crista). **(B):** Representative western blots and pool data depicting nuclear Nrf1 protein content and protein abundance of cytoplasmic and nuclear mitochondrial transcription factor A (mtTFA) in the heart tissue of vehicle/CDC-treated Mdx mice and age-matched wild-type mice (CTL) 3 weeks after treatment. **(C):** Bar graph demonstrating mitochondrial DNA copy numbers per cell in the heart tissue of experimental animals 3 weeks after treatment. **(D):** Representative western blots and pool data showing protein content of mitochondrial respiratory chain subunits in the heart tissue of Mdx mice 3 weeks after treatment with vehicle (Mdx+vehicle) and CDC (Mdx+CDC). Concomitant upregulation of Nrf1 and mtTFA were associated with increased mitochondrial DNA copy numbers and accompanied with restored expression of mitochondrial respiratory chain subunits. PC*: positive control. Data are means ± SEM; n = 7 in each group. †P < 0.05 vs. Mdx+Vehicle and control (CTL; wild type); †† P < 0.001 vs. Mdx+CDC and control (CTL; wild type).

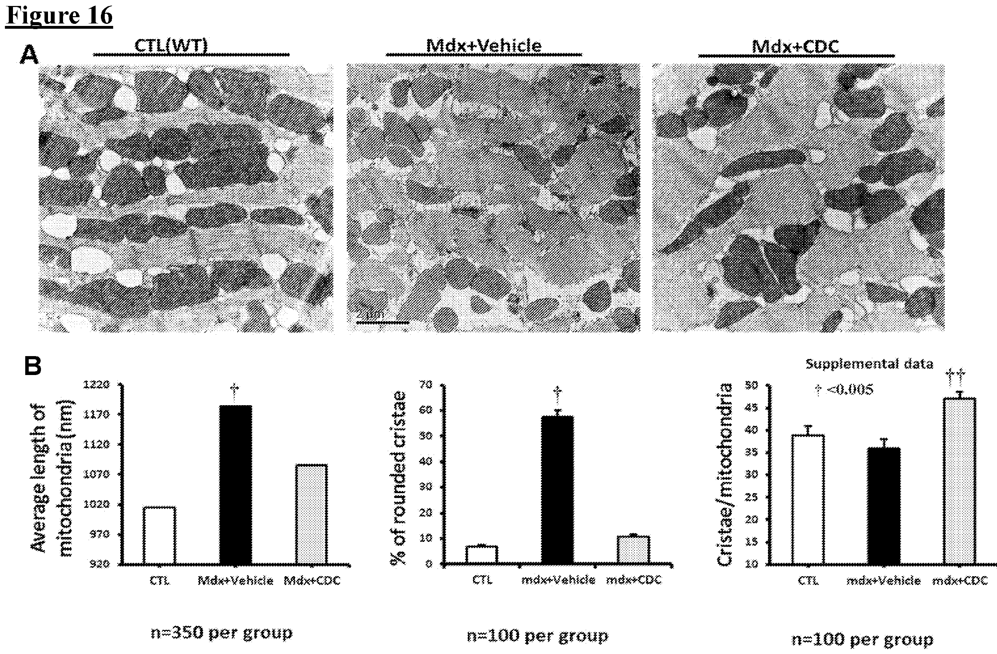

**Figure 16****. Ultrastructeral Degenerative Alterations In The Heart Of 10-Month-Old Mdx Mice Diminished Markedly 3 Weeks After Treatment With CDC. (A):** Representative images of transmission electron microscopy of cardiomyocytes illustrating intracellular accumulation of amorphous proteins, extensive sarcomeric disruption and irregularities (Z streaming) and disorganized altered interfibrillar mitochondria in the 10-month-old Mdx mice. CDC markedly decreased cardiomyocyte degenerative alterations 3 weeks after intramyocardial injection. **(B):** Bar graphs depicting average length of mitochondria and total number and percentage of rounded crista in mitochondria in wild type control mice (CTL) and in vehicle- (Mdx+vehicle) and CDC-treated Mdx mice(Mdx+CDC) 3 weeks after treatment. Data are means ± SEM;†P < 0.005 vs. Mdx+CDC and control (CTL; wild type); †† P < 0.005 vs. Mdx+vehicle and control (CTL; wild type).

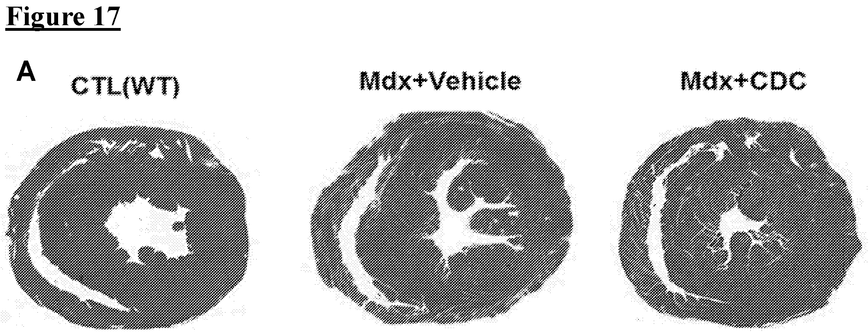

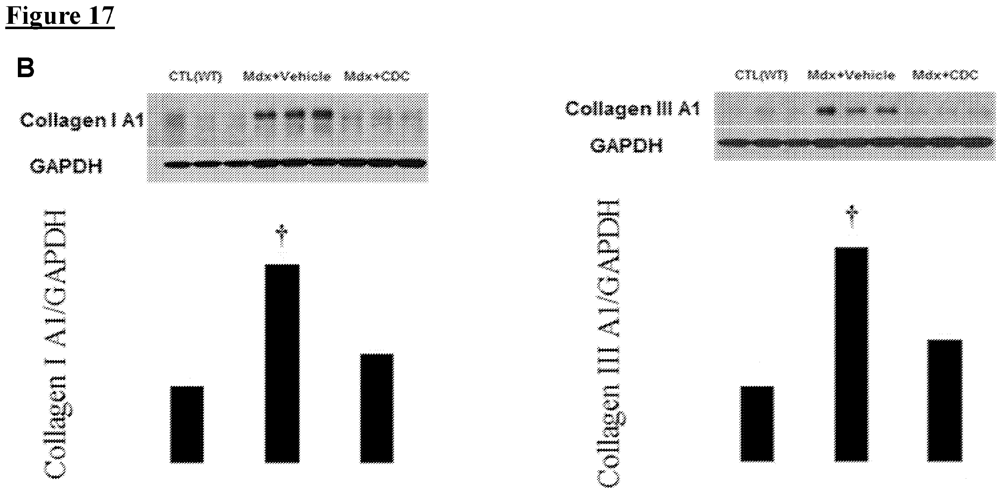

**Figure 17****. CDC Treatment Reduced Cardiac Collagen Content And Fibrosis.** Representative Masson trichrome images **(A)** and western blots and pooled data **(B)** representing fibrosis and collagen content in the mdx mouse hearts 3 weeks after treatment with vehicle (Mdx+Vehicle) or CDC (Mdx+CDC). Age-matched wild type mice (CTL) served as control. Collagen band size: 90-150 kDa. Data are means ± SEM; n = 7 in each group. †P < 0.01 vs. Mdx+CDC and control (CTL; wild type). Scale bars: 1mm

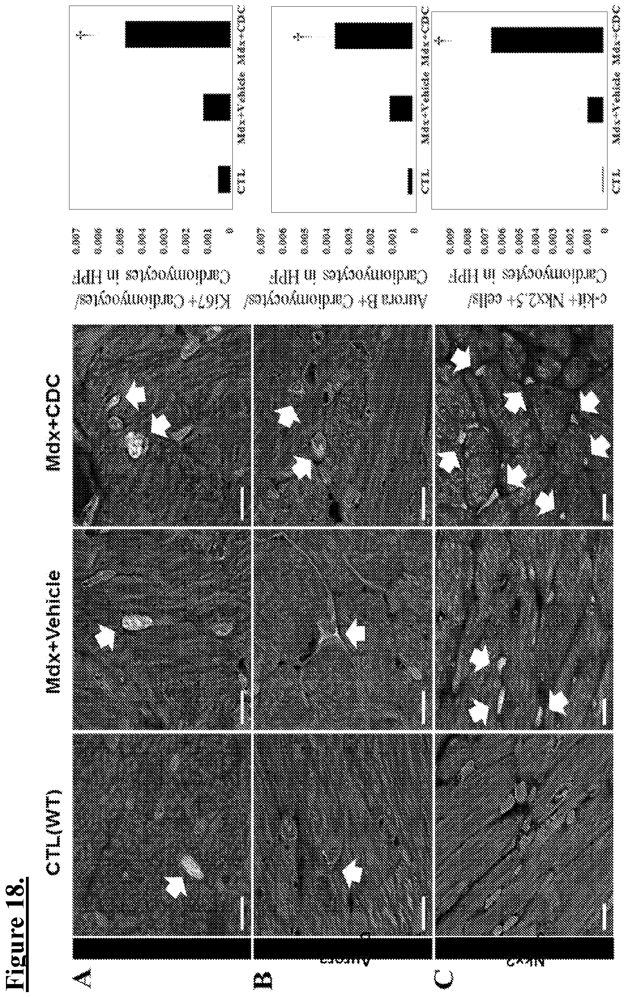

**Figure 18****. CDC Treatment Increased Cardiomyocyte Cycling And Proliferation And Augmented Number Of C-Kit Positive Cells Differentiating Into Cardiac Lineage (C-Kit+Nkx2.5+).** Representative immunohistochemical images and pooled data **((A)-(C);** CTL [wild type], vehicle and CDC-treated Mdx mouse hearts stained for Ki67 **(A),** aurora B **(B),** c-kit and Nkx2.5 **(C)**) from Mdx mice treated at 10 months of age. Arrows point to Ki67+ **(A)** and aurora B+ **(B)** cardiomyocytes and the cells positive for both c-kit and Nkx2.5 **(C).** Fractions of cycling (Ki67+) and proliferating (Aurora B+) cardiomyocytes are expressed as the number of Ki67+ and aurora B+ cardiomyocytes divided by the total number of cardiomyocytes per high-power field (HPF), respectively (Pooled data **(A), (B)**)**.** The portion of c-kit+Nkx2.5+ cells was calculated as the number of c-kit+Nkx2.5+ cells divided by the total number of cardiomyocytes per HPF (Pooled data (C)). WGA (Wheatgerm agglutinin) was applied for staining and delineation of cell membrane. Data are means ± SEM; n =7 in each group. †P < 0.01 vs. Mdx+Vehicle and control (CTL; wild type); Scale bars: 10 µm.

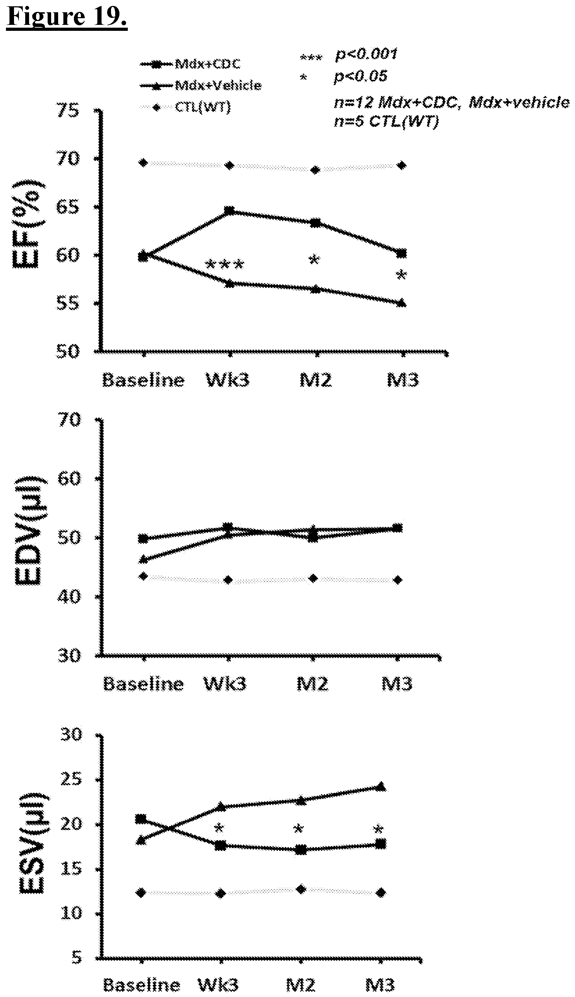

**Figure 19****. Functional Benefits After Cardiosphere-Derived Cell (CDC) Transplantation.** Pooled data for left ventricular ejection fraction (EF) and LV end-diastolic (LV EDV) and end-systolic (LV ESV) volumes show that CDC transplantation resulted in a sustained improvement of EF, LV EDV and LV ESV for 3 months in Mdx mice that received CDC at 10 months of age. Data are means ± SEM; n=5 (control wild type) and n=12 (Mdx+vehicle, Mdx+CDC). * P<0.05 vs Gq+CDC; *** P<0.001 vs Gq+CDC.

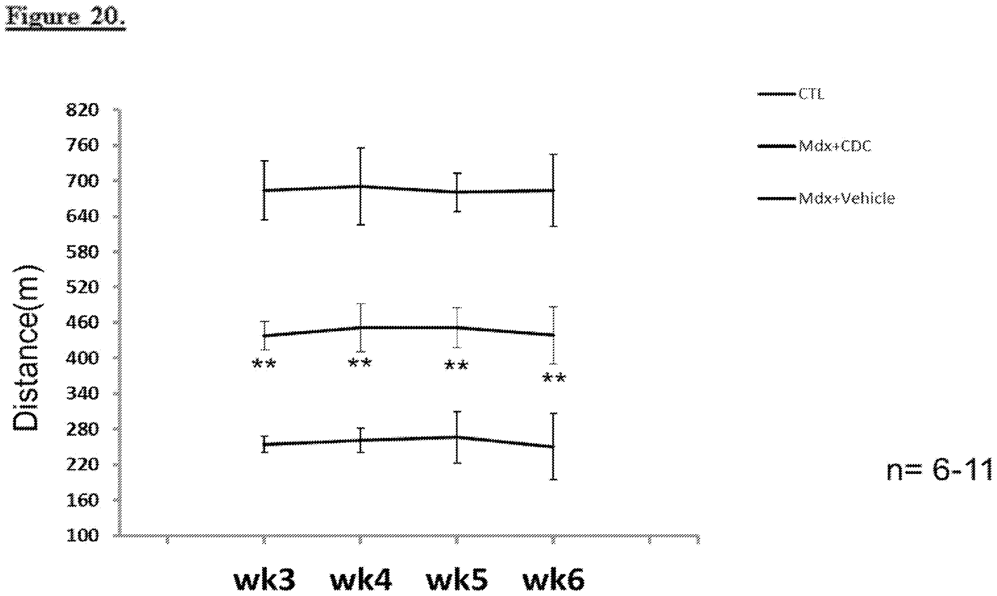

**Figure 20****. Enhanced Maximal Exercise Capacity With CDC Treatment.** Age-matched wild type mice (CTL) and 10-month-old Mdx mice treated with vehicle (Mdx+vehicle) or CDC (Mdx+CDC) were subjected to weekly high intensity exercise (stepwise increase in average speed every two minutes until exhaustion), starting 3 weeks after CDC/vehicle treatment. Sustained improvement of exercise capacity was observed in CDC-treated mdx mice relative to vehicle-treated mice. Data are means ± SEM; n=6 (control wild type) and n=11 (Mdx+vehicle, Mdx+CDC). * P<0.05 vs Gq+Vehicle.

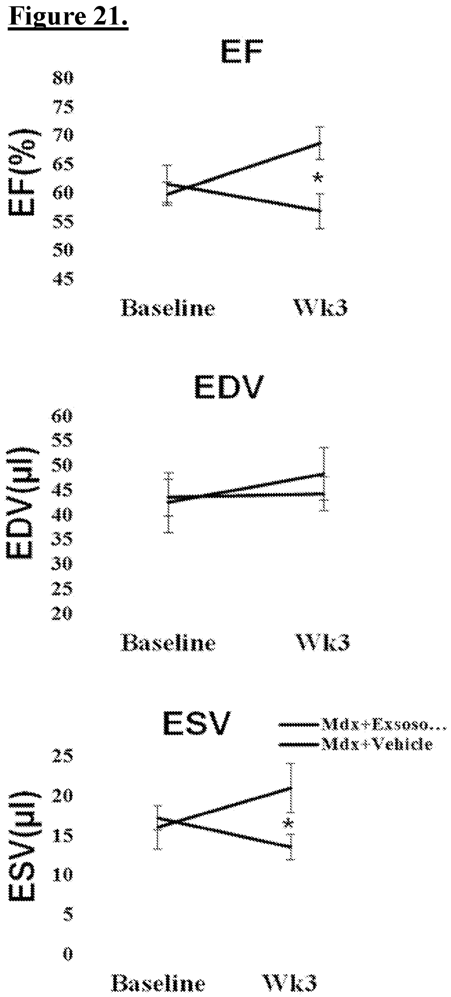

**Figure 21****. Functional Benefits After Transplantation Of Human CDC-Derived Exosomes.** Pooled data for left ventricular ejection fraction (EF) and LV end-diastolic (LV EDV) and end-systolic (LV ESV) volumes show that exosome transplantation resulted in improvement of EF, LV EDV and LV ESV three weeks after intramyocardial injection in 10-month-old Mdx mice. Data are means ± SEM; n=11 in each group. * P<0.05 vs Gq+CDC.

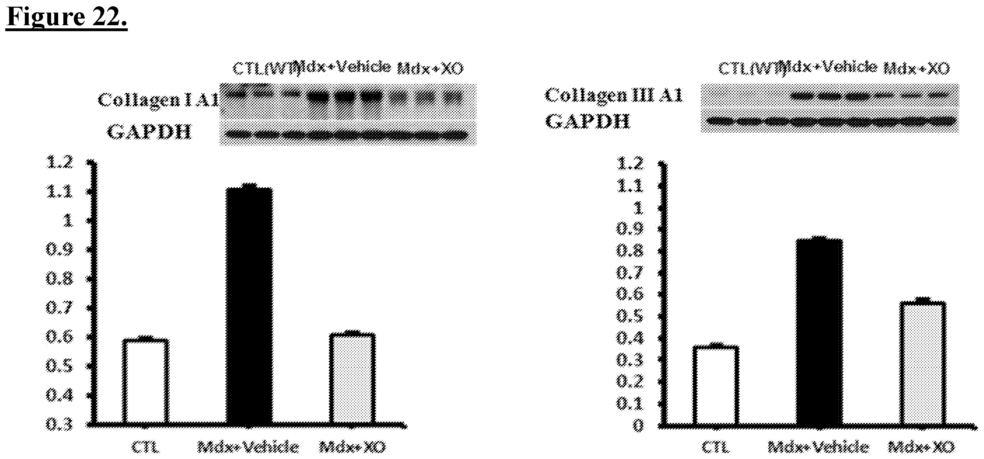

**Figure. 22****. CDC-Derived Exosomes Reduce Cardiac Collagen Content And Fibrosis.** Representative western blots and pooled data depicting collagen I and III protein content in the mdx mouse hearts 3 weeks after treatment with vehicle (Mdx+Vehicle) or exosomes (Mdx+XO). Age-matched wild type mice (CTL) served as control. Collagen band size: 90-150 kDa. Data are means ± SEM; n = 7 in each group. †P < 0.01 vs. Mdx+XO and control (CTL; wild type).

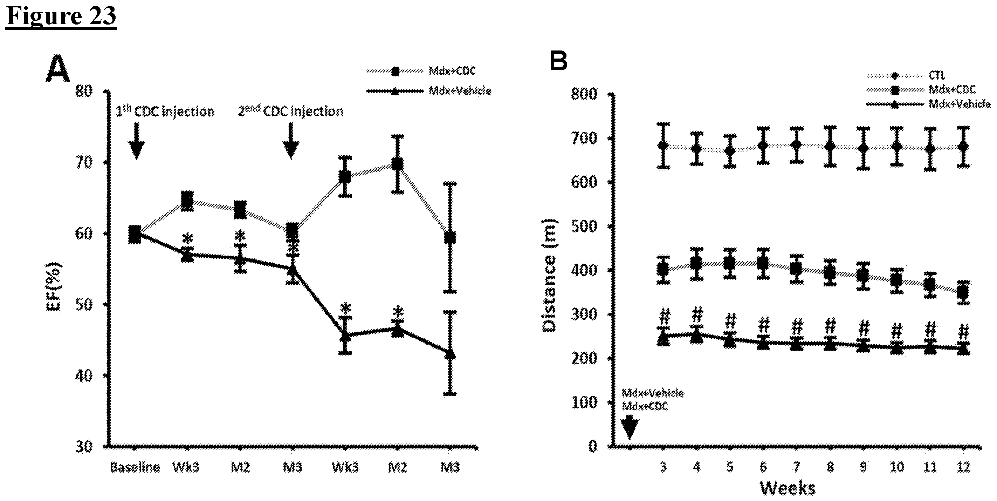

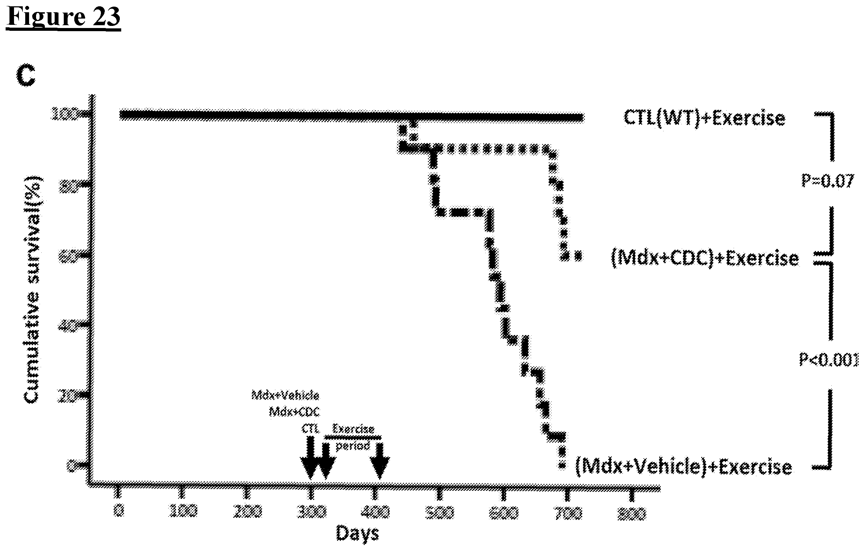

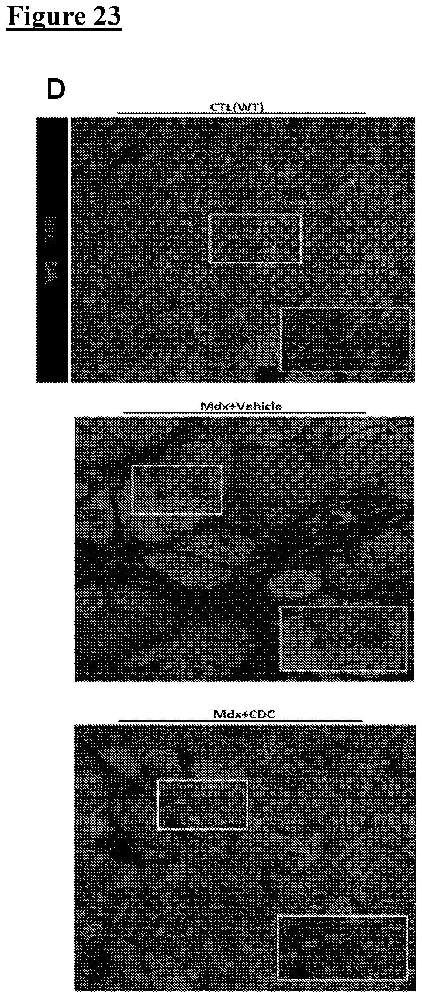

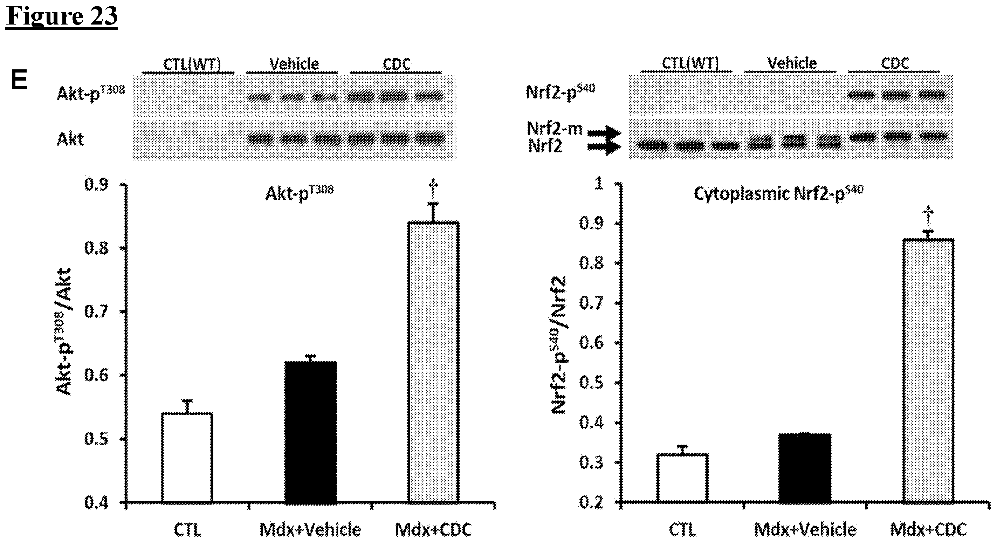

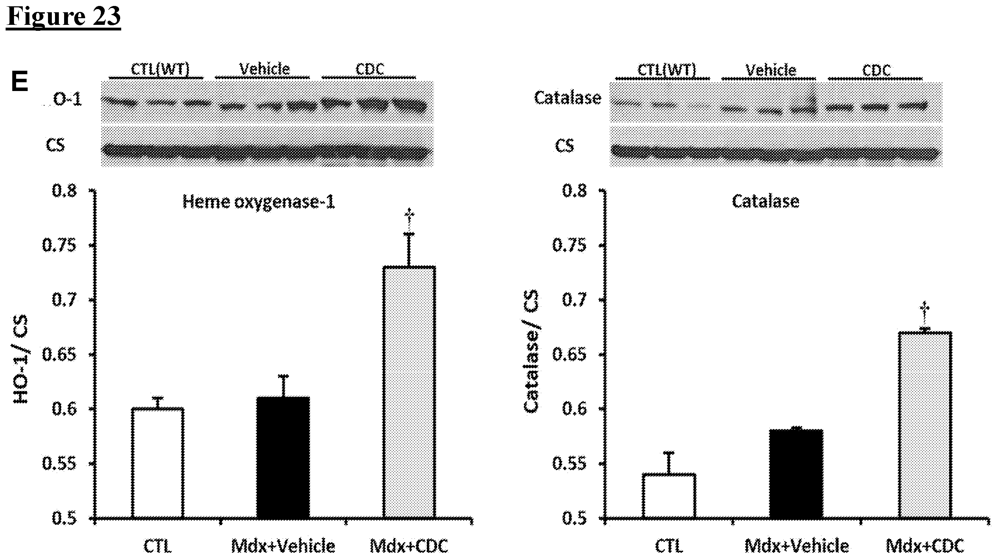

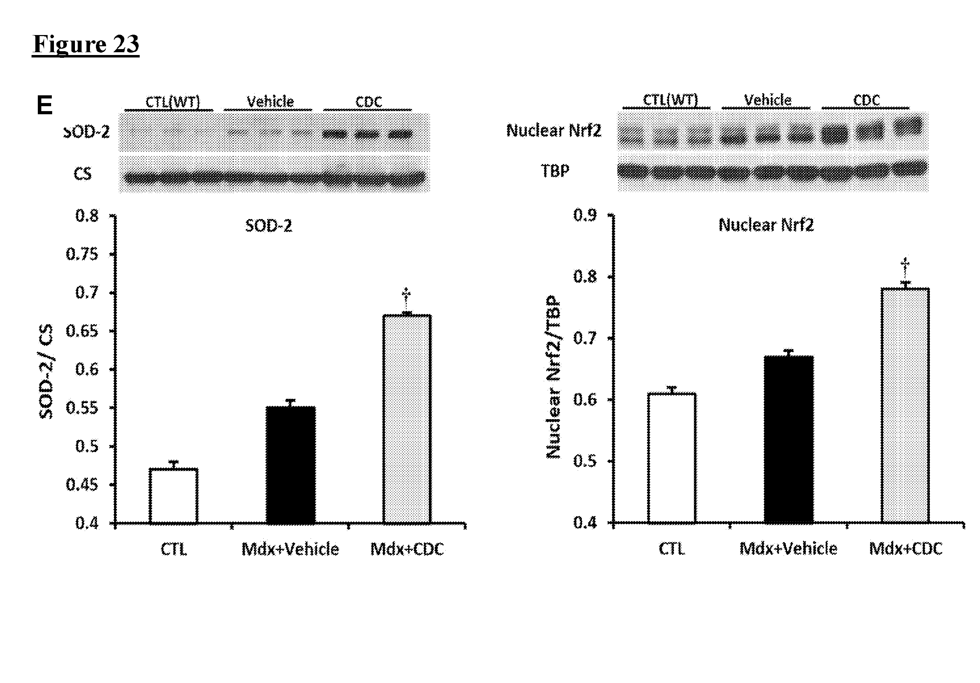

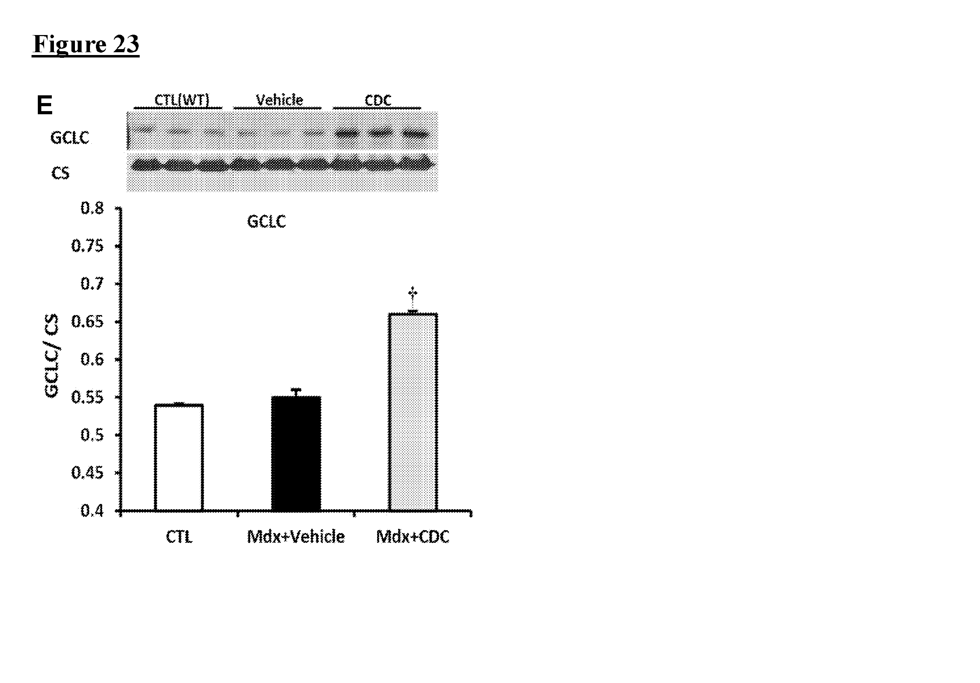

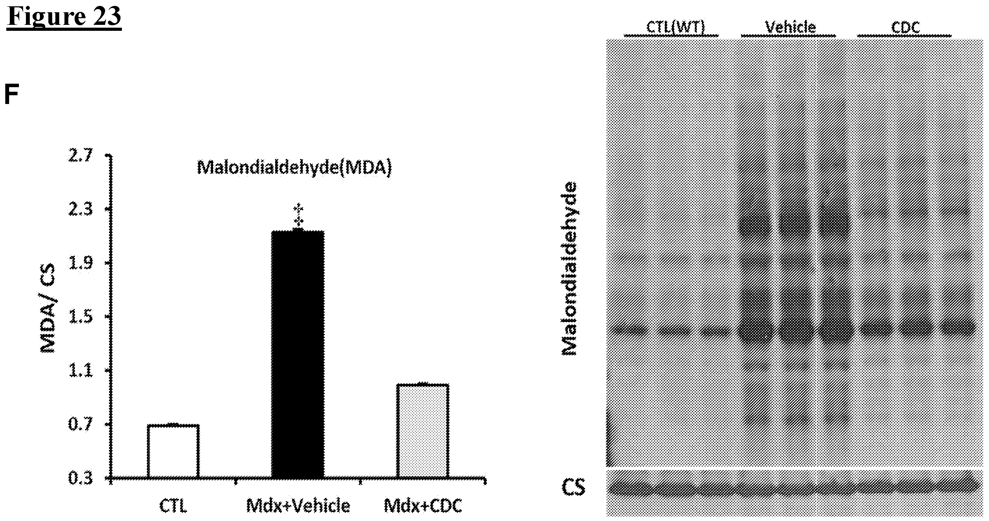

**Figure 23****. Function, survival and antioxidant pathways improved by CDC transplantation in *mdx* mice.** A: Ejection fraction (EF) in CDC-injected *mdx* mice (Mdx+CDC) and vehicle-injected *mdx* mice (Mdx+Vehicle) in response to injections at baseline (10 mos of age) and 3 months later (n=12 each). B: Exercise capacity in mice subjected to weekly high-intensity treadmill exercise, starting 3 weeks after CDC or vehicle administration (CTL: n=7; Mdx+Vehicle & Mdx+CDC: n=11 each). C: Kaplan-Meier analysis of survival in the same animals as B shows lower survival in vehicle-treated *mdx* mice than in CDC-treated *mdx* mice or wild-type controls (p<0.001, log rank test); the latter two groups, however, were statistically comparable. D: Immunohistochemical images of Nrf2 in *mdx* mouse hearts 3 weeks after administration of vehicle or CDCs. Age-matched wild-type mice (CTL) served as control. Scale bars: 10 µm. E: Western blots and pooled data for protein abundance of phospho-Akt(Akt-p^{T308}), cytoplasmic phospho-Nrf2 (Nrf2-p^{S40}), nuclear Nrf2 and downstream gene products: heme oxygenase-1 (HO-1), catalase, superoxide dismutase2 (SOD-2), and catalytic subunit of glutamate-cysteine ligase (GCLC) in *mdx* mouse hearts 3 weeks after administration of vehicle or CDCs (n=4-6). F: Pooled data and representative western blot of myocardial malondialdehyde protein adducts 3 weeks after injections as indicated, showing attenuation of oxidative stress in Mdx+CDC. Pooled data are means ± SEM. **P<0.05 vs. Mdx+CDC; #P<0.005 vs. Mdx+CDC; †P<0.05 vs. Mdx+Vehicle and CTL (WT, wild type mice); ‡P<0.002 vs. Mdx+CDC and CTL (WT, wild type mice).*

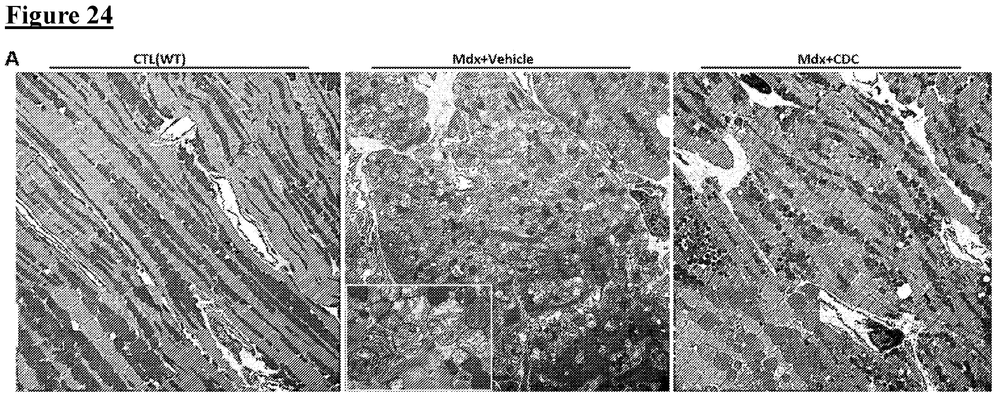

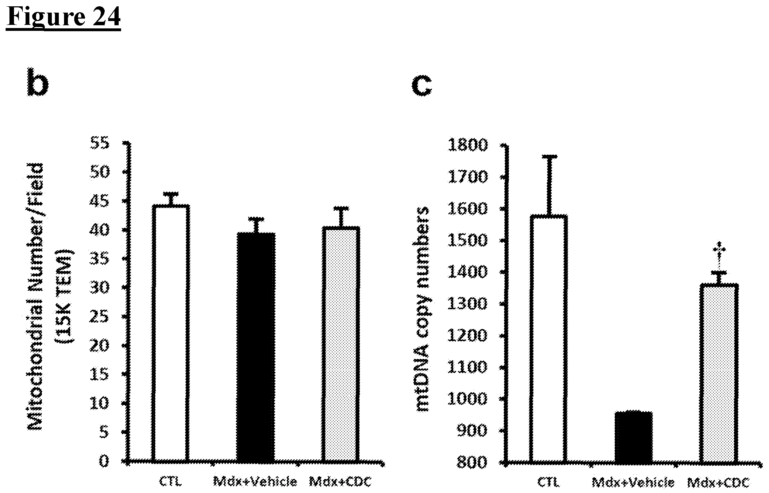

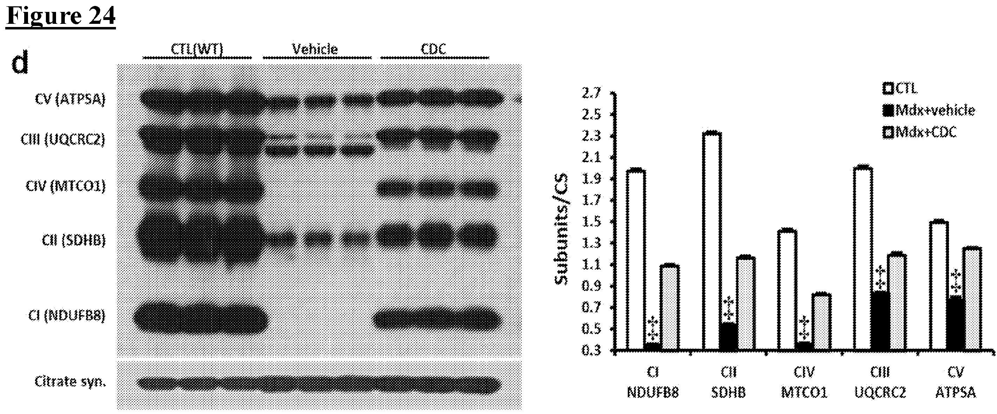

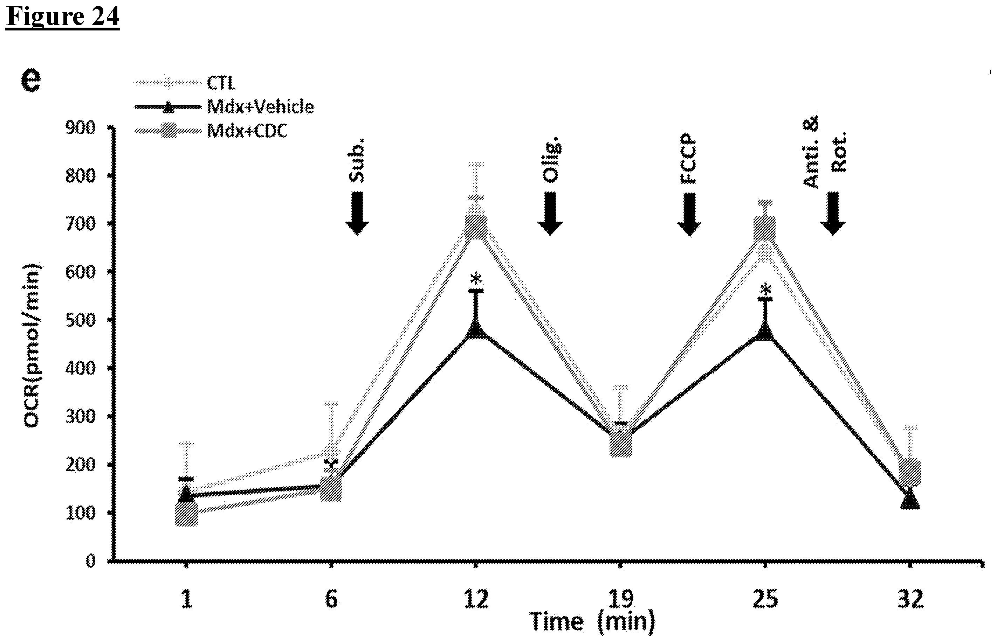

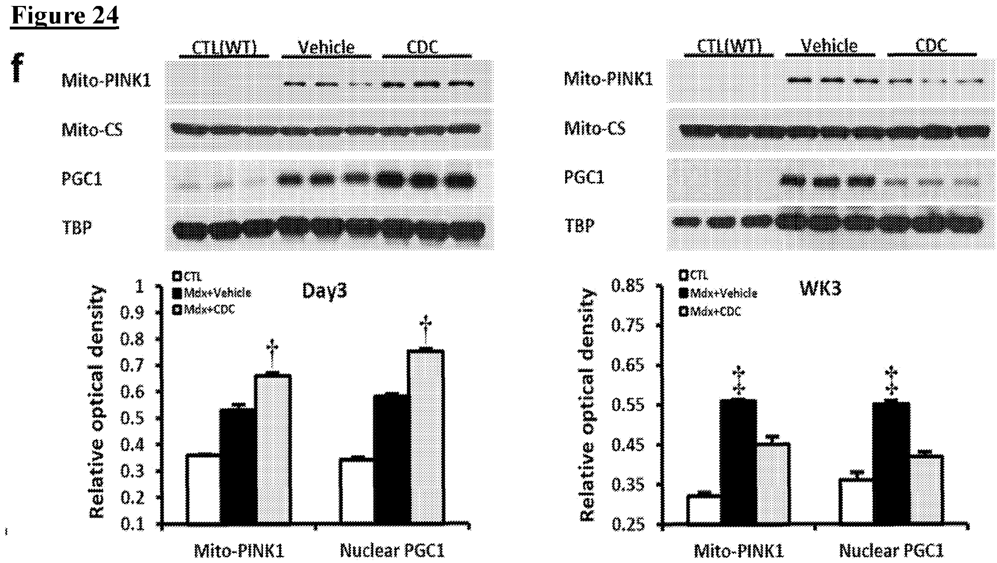

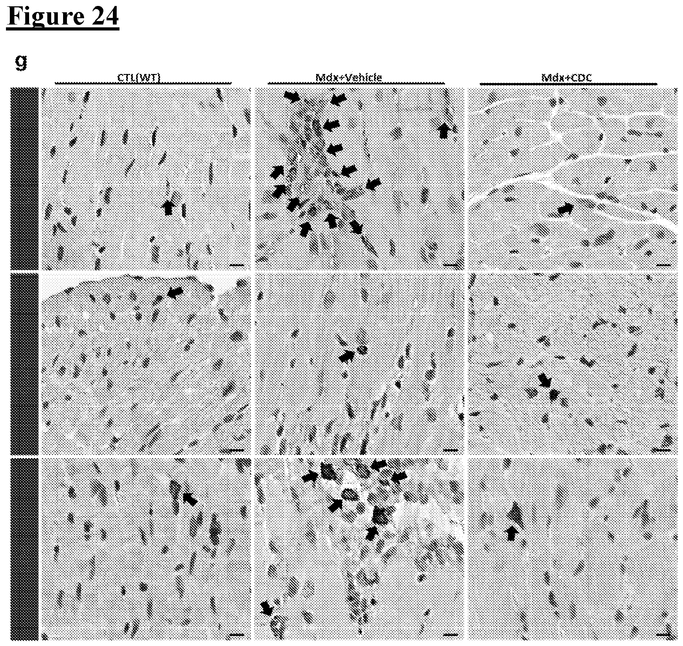

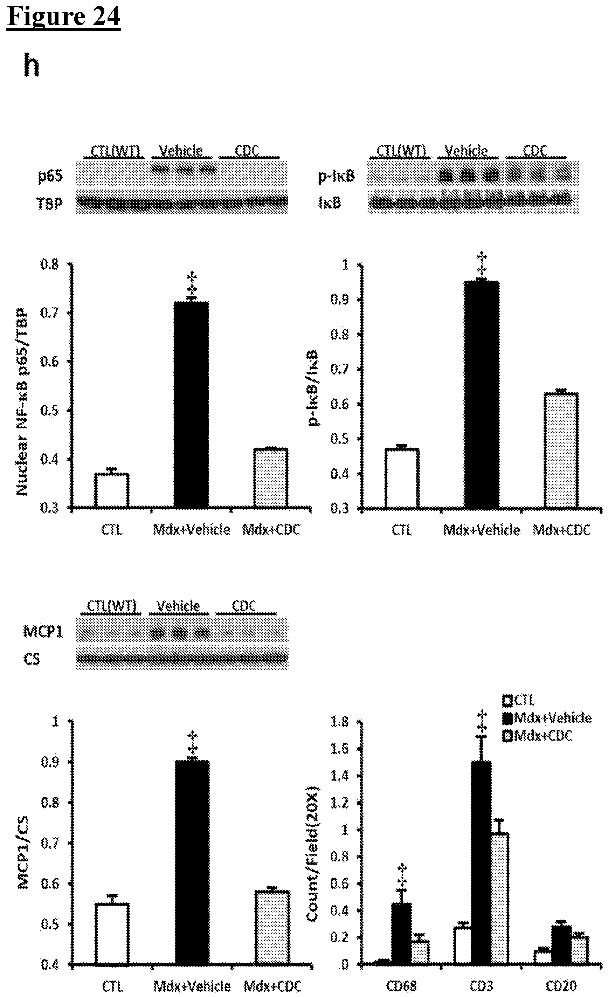

**Figure 24****. Mitochondrial dysfunction and inflammation attenuated by CDC transplantation in *mdx* mouse hearts.** A: Transmission electron microscopy (TEM) images from *mdx* mouse hearts 3 weeks after administration of vehicle (Mdx+Vehicle) or CDC (Mdx+CDC). Age-matched wild-type mice (CTL) served as control. B: Numbers of mitochondria from TEM images. C: Mitochondrial DNA copy numbers (per nuclear genome) in the heart tissue 3 weeks after treatment. D: Representative western blots and pooled data for mitochondrial respiratory chain subunits in heart tissue from CTL and *mdx* mice 3 wks after treatment (n=4-6 per group). E: Oxygen consumption rate (OCR) of mitochondria isolated from the hearts of CTL and CDC- or vehicle-treated *mdx* mice 3 weeks after treatment (CTL: n=3; Mdx+Vehicle & Mdx+CDC: n=8 each). Substrates (pyruvate, malate and ADP), a selective uncoupler (FCCP) and blockers (Oligomycin [Olig.]; Antimycin & Rotenone [Anti. & Rot.]) of oxidative phosphorylation were applied when indicated. F: Western blots and pooled data depicting protein abundance of mitochondrial PINK1 and nuclear PPARγ co-activator-1 (PGC-1) 3 days and 3 weeks after CDC administration in *mdx* mouse hearts (n=4-6). G: Immunohistochemical images of hearts stained for inflammatory cell markers CD68, CD20 and CD3. H: Western blots, pooled data and bar graph (lower right) representing average number of indicated inflammatory cells in *mdx* mouse hearts. In CDC-treated mice, accumulation of CD68⁺ macrophages (upper row) and CD3⁺ T cells (lower row) was reduced in association with inhibition of NF-κB pathway. Data are means ± SEM. *†P<0.05 vs. Mdx+Vehicle and CTL (WT, wild type mice); IP<0.003 vs. Mdx+CDC and CTL (WT, wild type mice); *P<0.05 vs. Mdx+CDC. Scale bars: 5µm(A); 10 µm (G).*

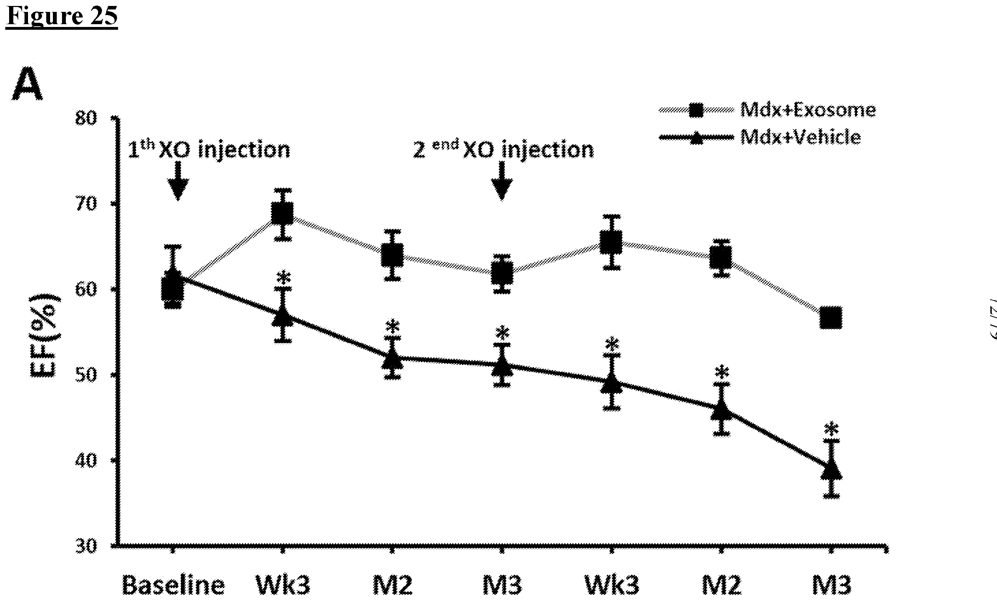

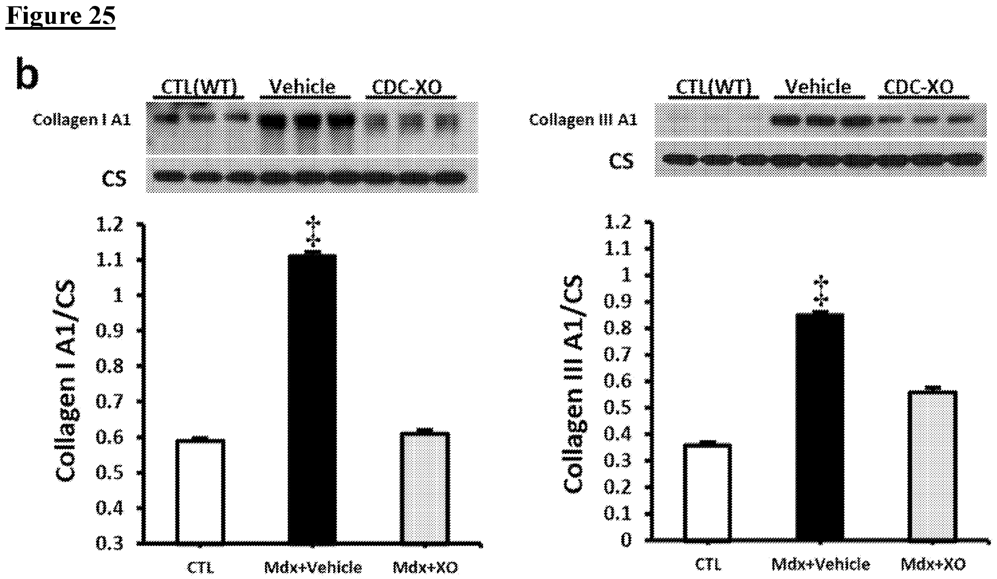

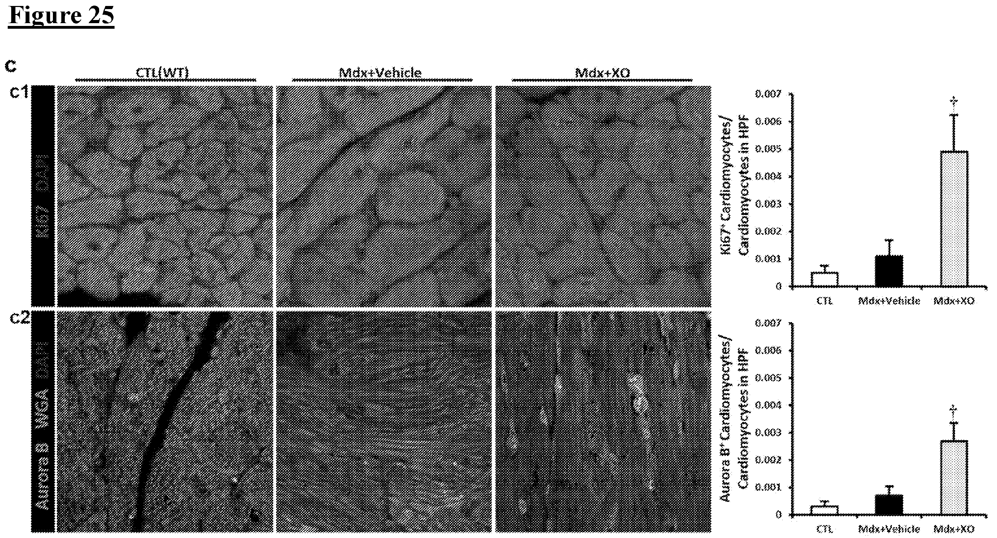

**Figure 25****. CDC exosomes reproduce benefits of CDCs in *mdx* mice.** A: Sustained functional benefit for at least 3 months with each of two sequential CDC exosome injections in *mdx* mice (n=11). B&C: Diminished cardiac collagen content (B) and enhanced cardiomyogenesis (C) 3 weeks after CDC exosome injection. Western blots and pooled data for cardiac collagen IA and IIIA (B), and immunohistochemical images and pooled data (C: CTL [wild type], vehicle and CDCexosome-treated [Mdx+XO] *mdx* mouse hearts stained for Ki67[C1] and Aurora B [C2]; n=4-6 per group). Arrows point to Ki67⁺ (C1) and Aurora B⁺ (C2) cardiomyocytes. WGA (Wheat germ agglutinin) was applied for staining and delineation of cell membrane. Data are means ± SEM; **P<0.05 vs. Mdx+XO; †P<0.02 vs. Mdx*+*Vehicle and CTL (WT, wild type mice); ‡P<0.01 vs. Mdx+XO and CTL (WT, wild type mice), scale bar:10µm.*

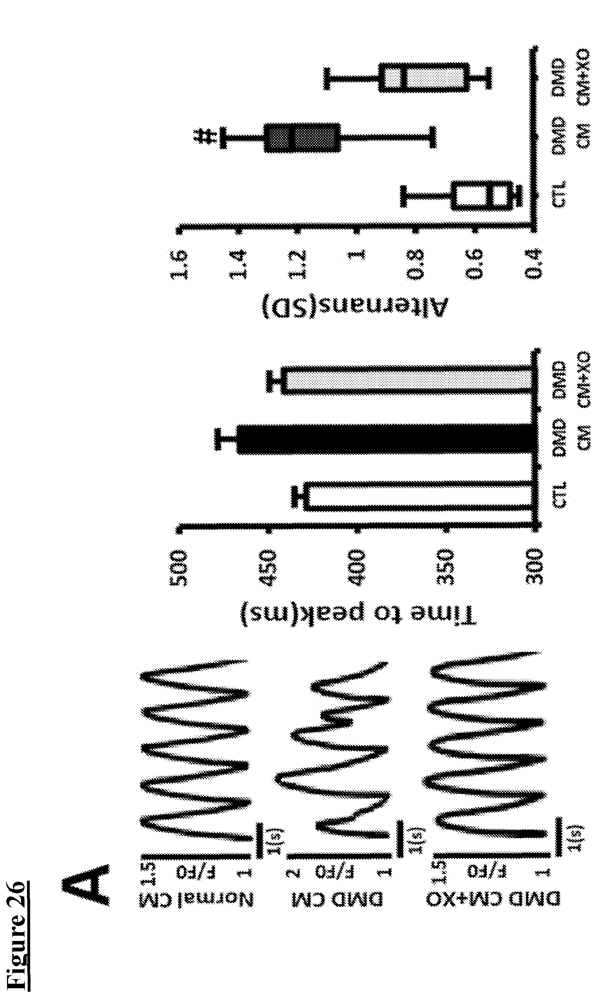

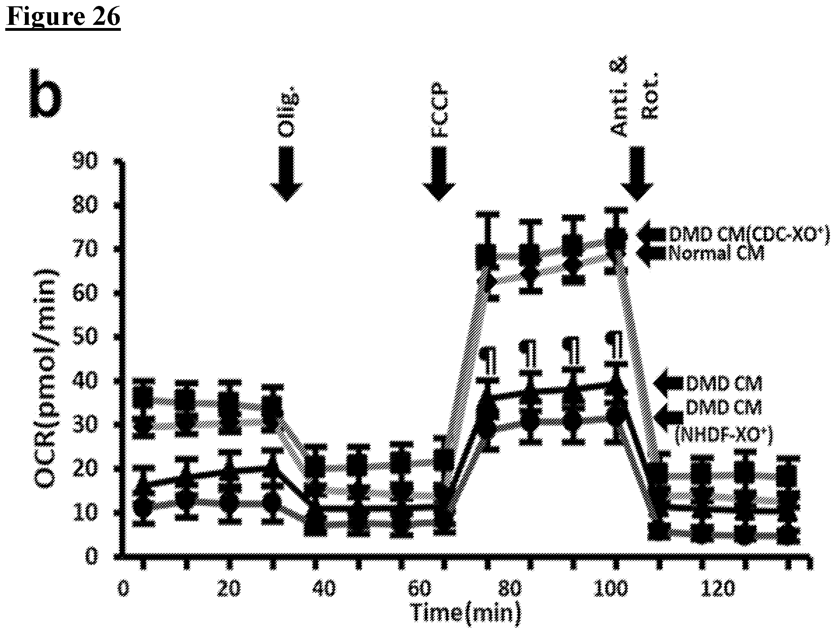

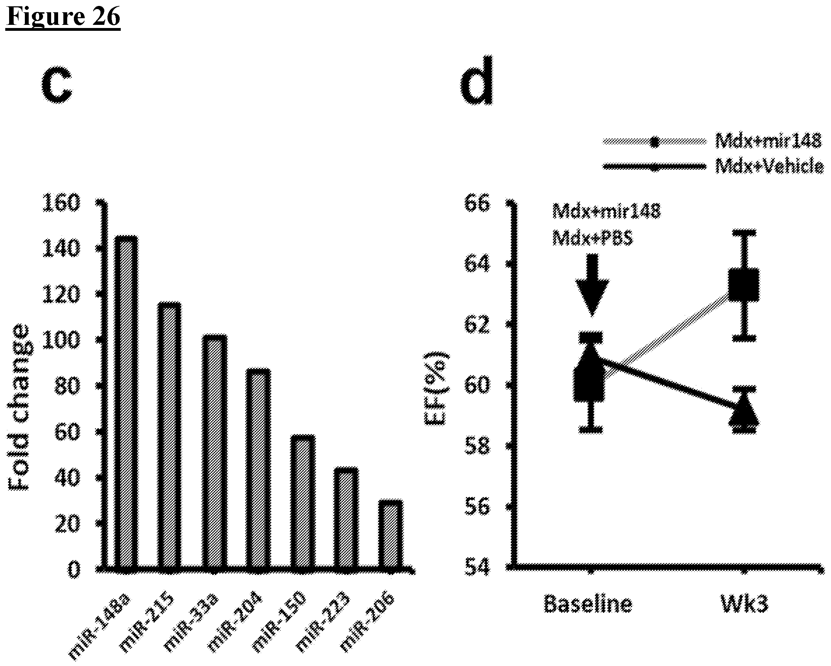

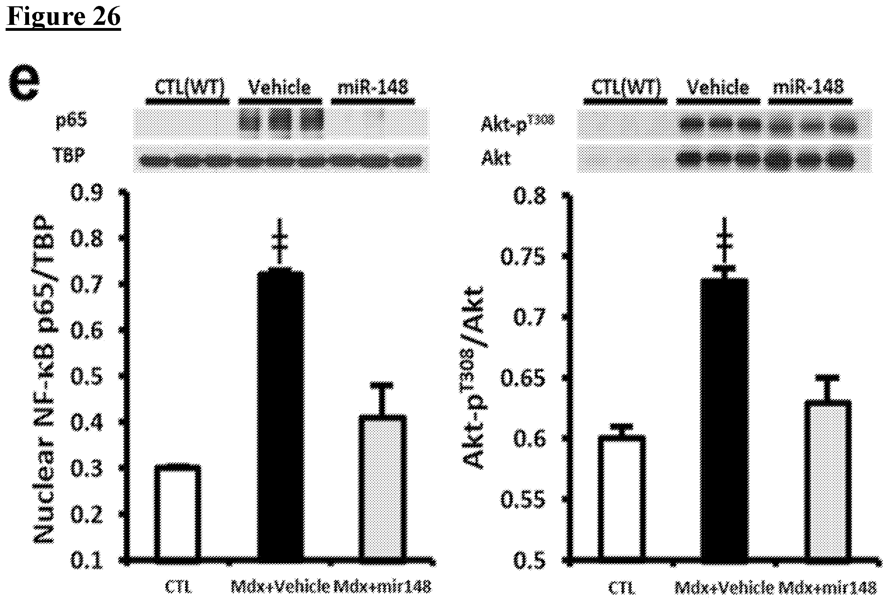

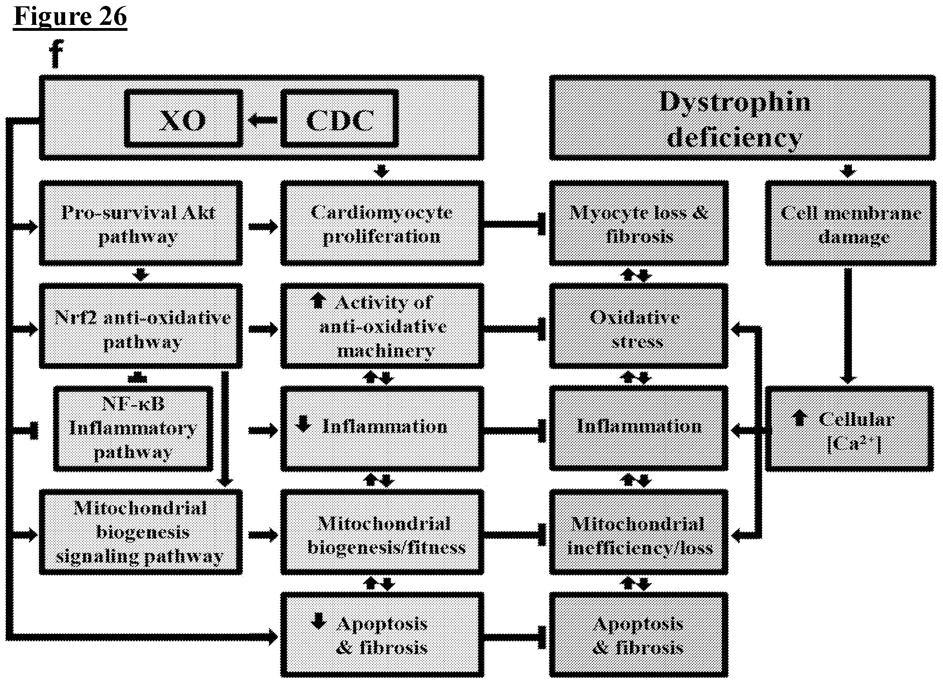

**Figure 26****. CDC exosomes in human Duchenne cardiomyocytes and miR-148 in *mdx* mice.** A: Calcium transients from normal and Duchenne human iPS-derived cardiomyocytes measured during 1Hz burst pacing. Duchenne cardiomyocytes primed with vehicle (DMD CM) or CDC exosomes 1 week before assessment (DMD CM+XO). Bar graphs of calcium transient: time to peak and alternans (variation in beat-to-beat calcium transient amplitude). B: Oxygen consumption rate (OCR) in human Duchenne cardiomyocytes primed with CDC exosomes [DMD CM (CDC-XO⁺)] or exosomes from normal human dermal fibroblasts [NHDF, as control; DMD CM (NHDF-XO⁺)] 1 week before OCR measurement. Normal (Normal CM) and non-treated Duchenne cardiomyocytes (DMD CM) were studied in parallel. See Fig. 2 legend for abbreviations. C: Differential expression of microRNAs in CDC exosomes isolated from hypoxic conditioned media (2% O₂) compared to CDC exosomes isolated from normoxic conditioned media (n=2), including only miRs with >20 sequence hits. D: Injection of miR-148 mimic intramyocardially partially restored cardiac function in *mdx* mouse hearts 3 weeks after treatment. E: Western blots and pooled data for nuclear p65 (left) and phosphorylated Akt (right) in *mdx* mouse hearts 3 weeks after miR-148 treatment. F: Schematic of pathophysiological mechanisms operative in Duchenne cardiomyopathy and the cellular mechanisms recruited by CDCs and their exosomes (XO). All data are means ± SEM except for the box plot (means ± SD).
**Figure 27. LV end-diastolic (LV EDV) and end-systolic (LV ESV) volumes after cardiospherederived cell (CDC), CDC exosome (CDC-XO) and miR-148 administrations.** CDC and CDC-XO transplantations resulted in a sustained improvement of LV EDV and LV ESV for 3 months after both first and second (3 months interval) injections in *mdx* mice, relative to placebo. Three weeks after miR148 injection, LV EDV and LV ESV were partially improved. Data are means ± SEM; n = 12 in each group. *#P<0.05* vs Mdx+Vehicle.
**Figure 28. Percentage engraftment of CDCs 1, 2 and 3 weeks after transplantation. Percentage** engraftment of CDCs at 1 week was ~8% and <1% at 2 weeks. By 3 weeks, no surviving CDCs could be detected. n = 3 at each time point.
**Figure 29. Cardiomyogenesis and diminished fibrosis with CDC treatment in *mdx* mice.** Enhanced cardiomyogenesis (A&B) and diminished cardiac fibrosis (C) and collagen content (D) 3 weeks after CDC injection in *mdx* mice. Representative immunohistochemical images and pooled data (A&B: CTL [wild type], vehicle and CDC-treated [Mdx+CDC] mdx mouse hearts stained for Ki67 [A] and Aurora B [B]; n=4-6 per group). Arrows point to Ki67⁺ (A) and aurora B⁺ (B) cardiomyocytes. Representative Masson trichrome images (C) and western blots and pooled data (D) depicting cardiac collagen IA and IIIA. Data are means ± SEM; *†*P<0.05 vs. Mdx+Vehicle and CTL (control); #P<0.05 vs. Mdx+CDC and CTL (control). *Scale bars: 10µm (A).*
**Figure 30. Fold changes of microRNAs in CDC exosomes isolated from hypoxic conditioned media.** Under hypoxic conditions (2% O₂) compared to CDC exosomes isolated from normoxic conditioned media; fold change >10 and <20 were included. NEBNext Small RNA Library Prep kit (New England BioLabs, Ipswich, MA) was used for miRNA-seq library preparation of extracted small RNAs from the exosomes. RNAs were extracted from exosomes using miRNeasy Serum/Plasma Kit (QIAGEN, Germantown, MD).
**Figure 31. Isolated exosomes by ultracentrifugation were analyzed by nanoparticle tracking.** Using the NanoSight NS300 system (NanoSight Ltd, UK), videos were collected and analyzed using NTAsoftware (version 2.3), with the minimal expected particle size, minimum track length, and blur setting, all set to automatic. Camera shutter speed was fixed at 30.01 ms and camera gain was set to 500. Camera sensitivity and detection threshold were set close to maximum (15 or 16) and minimum (3 or 4), respectively, to reveal small particles. Ambient temperature was recorded manually, ranging from 24 to 27°C. For each sample, five videos of 60 seconds duration were recorded, with a 10-second delay between recordings, generating five replicate histograms that were averaged. Representative five replicate histograms depicting size/concentration. Standard error of the mean concentration, calculated from 5 replicates, is shown in red in right graph.

### DETAILED DESCRIPTION

All references cited herein are incorporated by reference in their entirety as though fully set forth. Unless defined otherwise, technical and scientific terms used herein have the same meaning as commonly understood by one of ordinary skill in the art to which this invention belongs. Allen et al., Remington: The Science and Practice of Pharmacy 22nd ed., Pharmaceutical Press (September 15, 2012); Hornyak et al., Introduction to Nanoscience and Nanotechnology, CRC Press (2008); Singleton and Sainsbury, Dictionary of Microbiology and Molecular Biology 3rd ed., revised ed., J. Wiley & Sons (New York, NY 2006); Smith, March's Advanced Organic Chemistry Reactions, Mechanisms and Structure 7th ed., J. Wiley & Sons (New York, NY 2013); Singleton, Dictionary of DNA and Genome Technology 3rd ed., Wiley-Blackwell (November 28, 2012); and Green and Sambrook, Molecular Cloning: A Laboratory Manual 4th ed., Cold Spring Harbor Laboratory Press (Cold Spring Harbor, NY 2012), provide one skilled in the art with a general guide to many of the terms used in the present application. For references on how to prepare antibodies, see Greenfield, Antibodies A Laboratory Manual 2nd ed., Cold Spring Harbor Press (Cold Spring Harbor NY, 2013); Köhler and Milstein, Derivation of specific antibody-producing tissue culture and tumor lines by cell fusion, Eur. J. Immunol. 1976 Jul, 6(7):511-9; Queen and Selick, Humanized immunoglobulins, U. S. Patent No. 5,585,089 (1996 Dcc); and Riechmann et al., Reshaping human antibodies for therapy, Nature 1988 Mar 24, 332(6162):323-7.

One skilled in the art will recognize many methods and materials similar or equivalent to those described herein, which could be used in the practice of the present invention. Indeed, the present invention is in no way limited to the methods and materials described. For purposes of the present invention, the following terms are defined below.

As used in the description herein and throughout the claims that follow, the meaning of "a," "an," and "the" includes plural reference unless the context clearly dictates otherwise. Also, as used in the description herein, the meaning of "in" includes "in" and "on" unless the context clearly dictates otherwise.

Duchenne muscular dystrophy, a crippling genetic disease leading to premature death, affects the heart as well as skeletal muscle. Indeed, cardiomyopathy is the leading cause of death in Duchenne patients. There are no approved treatments for the cardiomyopathy, and novel Duchenne-specific experimental approaches such as exon skipping do not benefit the heart. Here the Inventors demonstrate that cardiosphere-derived cells (CDCs), in advanced clinical testing for therapeutic regeneration after myocardial infarction, reverse the key pathophysiological hallmarks of Duchenne cardiomyopathy (oxidative
stress, inflammation, fibrosis and mitochondrial dysfunction) in *mdx* mice. Exosomes secreted by human CDCs reproduce the benefits of CDCs in *mdx* mice, and reverse abnormalities of calcium cycling and mitochondrial respiration in human Duchenne cardiomyocytes.

Absence of dystrophin in Duchenne muscular dystrophy (DMD) leads to membrane fragility and secondary damage to muscle (both skeletal and cardiac). Early disability is due predominantly to the skeletal myopathy, but heart failure is the most common cause of death. No currently available treatment modality addresses the underlying pathophysiology of DMD-associated heart failure, a loss of functional heart muscle and conversion of living heart muscle to scar. Cardiosphere-derived cells (CDCs) may represent a viable therapeutic option. Healthy heart muscle regrew and scar decreased in the first-in-human CADUCEUS trial of CDCs in myocardial infarction; these findings are now being further tested in a randomized, placebocontrolled multicenter clinical trial of allogeneic CDCs. Preclinical studies show that CDCs are not only regenerative, but also anti-inflammatory and anti-fibrotic; they work indirectly via the secretion of exosomes laden with noncoding RNA including microRNAs (miRs). In a murine model of myocardial infarction, CDC-exosomes mimic the functional and structural benefits of CDCs, while blockade of exosome biosynthesis renders CDCs ineffective. Given the clinical data and the mechanism of action, the Inventors reasoned that CDCs might be useful in treating Duchenne cardiomyopathy. The goal is not to replace dystrophin, but rather to offset the pathophysiological consequences of dystrophin deletion, by recruiting regeneration, reversing fibrosis and targeting inflammation.

Exosomes, secreted lipid vesicles containing a rich milieu of biological factors, provide powerful paracrine signals by which stem cells potentiate their biological effects to neighboring cells, including diseased or injured cells. Through the encapsulation and transfer of proteins, bio-active lipid and nucleic acid "cargo", there is increasing recognition that these natural delivery devices are capable of inducing significant phenotypic and functional changes in recipient cells that lead to activation of regenerative programs. The role of such indirect mechanisms to in stem cell initiated regeneration is strongly suggested by growing evidence that after stem cell administration and clearance from delivery sites in tissue and organs, regeneration processes nevertheless persist and arise from endogenous tissues.

The "paracrine hypothesis" of stem cell regenerative activity has created a paradigm shift by which clinical applications based on exosomes secreted by the stem cells may prove superior, or provide distinct advantages, when compared to transplant and delivery of stem cells themselves. Stem cell-derived exosomes have been identified and isolated from supernatants of several cell types with demonstrated therapeutic potential, including mesenchymal stromal (MSC), (bone marrow stem cells) mononuclear (MNC) cells, immune cells (dendritic and CD34+), human neural stem cells (hNSCs), among others. In the context of heart disease, human cardiosphere derived cells (CDCs) are known to improve myocardium and vasculature. Stem cell-derived exosomes, including those produced by CDCs, may provide a potent and rich source for developing "cell-free" therapies.

In addition, exosome-based, "cell-free" therapies, in contrast to cell therapy, provide distinct advantages in regenerative medicine. As non-viable entities, with reduced or non-existent immunogenic or tumorigenic potential, these features significantly obviate safety issues. For example, stem cell-derived exosomes are less immunogenic than parental cells, as a result of a lower content of membrane-bound proteins, including MHC complex molecules. Replacing the administration of live cells with their secreted exosomes, mitigates many of the safety concerns and limitations associated with the transplantation of viable replicating cells. In addition, exosome encapsulation of bioactive components in lipid vesicles allows protection of contents from degradation *in vivo,* thereby potentially negating obstacles often associated with delivery of soluble molecules such as cytokines, growth factors, transcription factors and RNAs. This comparative ease of administration may ultimately allow for repeated and sustained delivery to patients, thereby maximizing the potential for regeneration and repair of diseased and/or dysfunctional tissue.

Also, exosome production under defined conditions allows for easier manufacture and scale-up opportunity. Manufacture of exosomes is akin to conventional biopharmacological product manufacture, allowing for standardization in production and quality control for dosage and biological activity testing. Further, the durability of exosomes in culture allows for the acquisition of large quantities of exosomes through their collection from a culture medium in which the exosomes are secreted over periods of time.

While it is now well-established that exosomes are involved in intercellular communication between different cell types, much remains to be discovered in regard to the mechanisms of their production within parental cells of origin and effects on target recipient cells. Exosomes have been reported to be involved in numerous cellular, tissue and physiological processes, including immune modulating processes, angiogenesis, migration of endothelial cells in connection with tumor growth, or reducing damage in ischemia reperfusion injury. Of critical scientific interest in establishing whether exosomes secreted by cells, such as cardiosphere-derived cells (CDCs), are capable of reproducing the therapeutic benefits of their parental cells, or possibly, are indispensable in effectuating such therapeutic benefits.

**General Features of Exosomes.** Secreted by a wide range of cell types, exosomes are lipid bilayer vesicles that are enriched in a variety of biological factors, including cytokines, growth factors, transcription factors, lipids, and coding and non-coding nucleic acids. Exosomes are found in blood, urine, amniotic fluid, interstitial and extracellular spaces. These exocytosed vesicles of endosomal origin can range in size between 30-200 nm, including sizes of 40-100 nm, and possess a cup-shaped morphology, as revealed by electron microscopy. Their initial formation begins with inward budding of the cell membrane to form endosomes, which is followed by invagination of the limiting membrane of late endosomes to form multivesicular bodies (MVB). Fusion of the MVB with the plasma membrane results in the release of the internal vesicles to the extracellular space, through the formation of vesicles thereafter known as exosomes.

As described, the "cargo" contents of exosomes reflect their parental cellular origin, as containing distinct subsets of biological factors in connection with their parent cellular origin, including the cell regulatory state of the parental cells when formed. The rich biological milieu of different proteins, including cytokines and growth factors, lipids, coding and noncoding RNA molecules, within exosomes are all necessarily derived from their parental cells. In addition to containing a rich array of cytosolic derivatives, exosomes further express the extracellular domain of membrane-bound receptors at the surface of the membrane.

The described encapsulation and formation processes necessarily create heterogeneity in exosome compositions based on parental cellular origin and regulatory state at time of formation. Nevertheless, generic budding formation and release mechanisms establish a common set of features as a consequence of their origin, such as endosome-associated proteins (e.g., Rab GTPase, SNAREs, Annexins, and flotillin), proteins that are known to cluster into microdomains at the plasma membrane or at endosomes (four transmembrane domain tetraspanins, e.g., CD63, CD81, CD82, CD53, and CD37), lipid raft associated proteins (e.g., glycosylphosphatidylinositol- anchored proteins and flotillin), cholesterol, sphingomyelin, and hexosylceramides, as examples.

In addition to these core components reflecting their vesicle origin, a critical property of exosomes is a demonstrated capability to contain both mRNA and microRNA associated with signaling processes, with both cargo mRNA being capable to translation in recipient cells, or microRNA functionally degrading target mRNA in recipient cells. Other noncoding RNAs, capable for influencing gene expression, may also be present in exosomes. While the processes governing the selective incorporation of mRNA or microRNA populations into exosomes is not entirely understood, it is clear that RNA molecules are selectively, not randomly incorporated into exosomes, as revealed by studies reporting enrichment of exosome cargo RNAs when compared to the RNA profiles of the originating cells. Given the growing understanding of how such RNA molecules play a role in disease pathogenesis and regenerative processes, the presence of RNA molecules in exosomes and apparent potency in affecting target recipient cells suggests critical features that can be deployed in therapeutic approaches.

Importantly, the natural bilayer membrane encapsulation of exosomes provides a protected and controlled internal microenvironment that allows cargo contents to persist or migrate in the bloodstream or within tissues without degradation. Their release into the extracellular environment allows for interaction with recipient cells via adhesion to the cell surface mediated by lipid-ligand receptor interactions, internalization via endocytic uptake, or by direct fusion of the vesicles and cell membrane. These processes lead to the release of exosome cargo content into the target cell.

The net result of exosome-cell interactions is modulation of genetic pathways in the target recipient cell, as induced through any of several different mechanisms including antigen presentation, the transfer of transcription factors, cytokines, growth factors, nucleic acid such as mRNA and microRNAs. In the stem cell context, embryonic stem cell (ESC)-derived exosomes have been demonstrated to shuttle/transfer mRNA and proteins to hematopoietic progenitors. Other studies have shown that adult stem cell-derived exosomes also shuttle selected patterns of mRNA, microRNA and pre-microRNA associated with several cellular functions involved in the control of transcription, proliferation and cell immune regulation.

**Isolation and Preparation of Exosomes.** Exosome isolation relies on exploiting their generic biochemical and biophysical features for separation and analysis. For example, differential ultracentrifugation has become a leading technique wherein secreted exosomes are isolated from the supernatants of cultured cells. This approach allows for separation of exosomes from nonmembranous particles, by exploiting their relatively low buoyant density. Size exclusion allows for their separation from biochemically similar, but biophysically different microvesicles, which possess larger diameters of up to 1,000 nm. Differences in flotation velocity further allows for separation of differentially sized exosomes. In general, exosome sizes will possess a diameter ranging from 30-200 nm, including sizes of 40-100 nm. Further purification may rely on specific properties of the particular exosomes of interest. This includes, for example, use of immunoadsorption with a protein of interest to select specific vesicles with exoplasmic or outward orientations.

Among current methods (differential centrifugation, discontinuous density gradients, immunoaffinity, ultrafiltration and high performance liquid chromatography (HPLC), differential ultracentrifugation is the most commonly used for exosome isolation. This technique utilizes increasing centrifugal force from 2000xg to 10,000xg to separate the medium- and larger-sized particles and cell debris from the exosome pellet at 100,000xg. Centrifugation alone allows for significant separation/collection of exosomes from a conditioned medium, although it is insufficient to remove various protein aggregates, genetic materials, particulates from media and cell debris that are common contaminants. Enhanced specificity of exosome purification may deploy sequential centrifugation in combination with ultrafiltration, or equilibrium density gradient centrifugation in a sucrose density gradient, to provide for the greater purity of the exosome preparation (flotation density 1.1-1.2g/ml) or application of a discrete sugar cushion in preparation.

Importantly, ultrafiltration can be used to purify exosomes without compromising their biological activity. Membranes with different pore sizes - such as 100 kDa molecular weight cut-off (MWCO) and gel filtration to eliminate smaller particles - have been used to avoid the use of a nonneutral pH or non-physiological salt concentration. Currently available tangential flow filtration (TFF) systems are scalable (to >10,000L), allowing one to not only purify, but concentrate the exosome fractions, and such approaches are less time consuming than differential centrifugation. HPLC can also be used to purify exosomes to homogeneouslysized particles and preserve their biological activity as the preparation is maintained at a physiological pH and salt concentration.

Other chemical methods have exploit differential solubility of exosomes for precipitation techniques, addition to volume-excluding polymers (e.g., polyethylene glycols (PEGs)), possibly combined additional rounds of centrifugation or filtration. For example, a precipitation reagent, ExoQuick^{®}, can be added to conditioned cell media to quickly and rapidly precipitate a population of exosomes, although re-suspension of pellets prepared via this technique may be difficult. Flow field-flow fractionation (FlFFF) is an elution-based technique that is used to separate and characterize macromolecules (e.g., proteins) and nano-to micro-sized particles (e.g., organelles and cells) and which has been successfully applied to fractionate exosomes from culture media.

Beyond these techniques relying on general biochemical and biophysical features, focused techniques may be applied to isolated specific exosomes of interest. This includes relying on antibody immunoaffinity to recognizing certain exosome-associated antigens. As described, exosomes further express the extracellular domain of membrane-bound receptors at the surface of the membrane. This presents a ripe opportunity for isolating and segregating exosomes in connections with their parental cellular origin, based on a shared antigenic profile. Conjugation to magnetic beads, chromatography matrices, plates or microfluidic devices allows isolating of specific exosome populations of interest as may be related to their production from a parent cell of interest or associated cellular regulatory state. Other affinity-capture methods use lectins which bind to specific saccharide residues on the exosome surface.

**Exosome-Based Therapies.** A chief goal of developing exosome-based therapy is the creation of "cell-free" therapies, wherein the benefits of cellular therapeutics can be provided with reduced risks or in scenarios in which cell therapy would be unavailable. For example, Duchenne muscular dystrophy (DMD) associated heart failure (HF), particularly at later stages, presents significant exclusionary comorbidities, wherein cell, tissue, heart or mechanical transplantation may not be an option for late stages C and D. As described, the therapeutic benefits of cell-based therapies such as cardiosphere-derived cells (CDCs) appear to occur through indirect mechanisms involving regenerated myocardium and vasculature arising from endogenous origin. Cellular exosomes produced by CDCs may allow for production and delivery of growth factors, transcription factors, cytokines and nucleic acids for new therapeutic approaches in a manner that not only ameliorates progression of the disease, but repairs and regenerates disease and/or dysfunctional tissue. In this regard, CDC-derived exosomes may effectively address a major unmet medical need, by recruiting synergistic mechanisms to attract endogenous stem cells to sites of myocardial injury, promote differentiation into heart muscle and vessels, thereby reversing the pathophysiology of HF.

More specifically, DMD is an X-linked recessive disorder characterized by myopathy (cell membrane damage in muscle fiber) as exemplified by a variety of pathological features. this includes skeletal muscle weakness starting 3-5 years from onset, progressive weakness, wheelchair dependency at approximately 13 years from onset. Importantly, cardiomyopathy is observed to take hold in 1/3 of patients from less than 13 years from onset, increasing to 1/2 of patients less than 18 years from onset, and in all patients after 18 years. Dilated cardiomyopathy includes left ventricle posterobasal fibrosis; conduction abnormalities are mainly intra-atrial: SVT with abnormal AV nodal conduction. Patients may further suffer from smooth muscle myopathy including vascular dysfunction, further including GI and urinary tract systems involvement. Common prognosis is death from respiratory insufficiency or cardiomyopathy. Underlying these clinical features is dystrophin gene mutation (deletion) wherein loss of dystrophin results in cellular membrane damage and leakage of extracellular Ca²⁺ into the cell. Elevated intracellular levels ultimately result in increased oxidative and/or nitrosative stress and inflammation, and activation of calpain. The combination of these effects results in muscle proteolysis and apoptosis, leading to the degradative features described above.

Based on this pathophysiology of DMD patients, including an environment of increased oxidative and/or nitrosative stress, elevated inflammation, pro-apoptotic and remodeling states, therapeutic approaches involving CDCs may provide significant benefits in reversing the course of the disease. CDCs have been demonstrated as promoting antioxidative, anti-inflammatory, anti-apoptotic, anti-remodeling effects, in addition to enhancing regenerative capacity. In this regard, it is suggested that CDC administration is beneficial in retarding/reversing DMD, and exosome populations derived from CDCs may allow for these benefits to be delivered, while avoiding obstacles associated with cell-based therapy.

In particular, stem cell-derived exosomes are likely to be less immunogenic than parental cells. The possibility of replacing the administration of live cells with secreted exosomes, mitigates many of the safety concerns and limitations associated with the transplantation of viable cells. In addition, exosome encapsulation of bioactive components in lipid vesicles allows protection of contents from degradation in vivo, thereby potentially negating obstacles often associated with delivery of soluble molecules such as cytokines, growth factors, transcription factors and RNAs, while potentially allowing for increased concentrations to be provided. Particularly for chronic conditions, such as DMD, repeated and sustained delivery to patients may maximize the potential for regeneration and repair of diseased and/or dysfunctional tissue, in a manner that would be difficult or unsafe with a cell-based therapy. Fully realizing these benefits requires an improved understanding of whether exosomes secreted by cells such as CDCs, are alone capable of reproducing therapeutic benefits of their parental cells, or possibly indispensable in these processes. Confirming the role of exosomes in such processes will allow their application in new therapeutic approaches, including "cell-free" use in subjects for which cellular transplant or administration is unavailable (e.g., late stage heart disease), as pharmacological, device-based intervention or surgery may not be prudent treatment modalities for such subject. There is a great need in the art for identifying means by which to deliver the benefits of stem cell regeneration, without resorting to mechanisms involving administration or transplant of the cell themselves.

Described herein are compositions and methods and compositions providing significant benefits in the repair or regeneration of damaged or diseased tissues via "cell-free" methods involving exosomes. Specifically, human cardiosphere-derived cells (CDC)-derived exosomes are demonstrated as effective in reducing scar size and regenerating viable myocardium. Such results confirm that the major benefits of CDC cell therapy are mediated by exosomes, including specific microRNAs identified by the Inventors as enriched in CDCs.

Described herein is a method of treatment, including selecting a subject in need of treatment for heart failure secondary to a chronic degenerative muscular disease and administering a composition including a plurality of exosomes to the subject, wherein the plurality of the exosomes are isolated from cardiosphere-derived cells (CDCs) grown in serum-free media, include exosomes with a diameter of about 90 nm to about 200 nm and are CD81+, CD63+, or both, and further wherein administration of the composition treats the subject. In other embodiments, the chronic degenerative muscular disease is Duchenne muscular dystrophy. In other embodiments, administering a composition includes about 1 to about 100 mg exosome protein in a single dose. In other embodiments, a single dose is administered multiple times to the subject. In other embodiments, administering a composition includes injection. In other embodiments, the injection includes percutaneous injection. In other embodiments, the injection is directly into heart muscle. In other embodiments, administering a composition includes myocardial infusion. In other embodiments, myocardial infusion is intra-arterial or intravenous. In other embodiments, treatment of the subject results in decreased fibrosis, decreased inflammation, increased mitochondrial function and/or increased cardiomyogenesis. In other embodiments, decreased fibrosis includes a reduction in collagen accumulation. In other embodiments, collagen includes collagen I and/or collagen III. In other embodiments, decreased inflammation includes an increase in cytoplasmic nuclear factor (erythroid-derived 2)-like 2 (Nrf2), reduction in fatty acid peroxidation end products, reduced numbers of inflammatory cells, and/or upregulated expression of antioxidants. In other embodiments, antioxidants include heme oxygenase-1 (HO-1), catalase, superoxide dismutase-2 (SOD-2), and glutamate-cystein ligase catalytic (GCLC) subunit. In other embodiments, inflammatory cells include CD68+ macrophages and CD3+ T-cells. In other embodiments, increased mitochondrial function includes increased mitochondrial ultrastructure and/or increased mitochondrial biogenesis. In other embodiments, increased mitochondrial function includes increased nuclear PPAR-γ co-activator-1 (PGC-1) expression. In other embodiments, the exosomes include one or more microRNAs selected from the group consisting of: microRNAs miR-146a, miR148a, miR22, miR-24, miR-210, miR-150, miR-140-3p, miR-19a, miR-27b, miR-19b, miR-27a, miR-376c, miR-128, miR-320a, miR-143, miR-21, miR-130a, miR-9, miR-185, and miR-23a.

Further described herein is method of treatment, including selecting a subject in need of treatment for heart failure secondary to a chronic muscular disease and administering a composition including cardiosphere-derived cells (CDCs), wherein administration of the composition treats the subject. In other embodiments, the chronic muscular disease is Duchenne muscular dystrophy. In other embodiments, administering a composition includes about 1 x 10⁵ to about 1 x 10⁸ or more CDCs in a single dose. In another example, the number of administered CDCs includes intracoronary 25 million CDCs per coronary artery (i.e., 75 million CDCs total) as another baseline for exosome dosage quantity. In various embodiments, the numbers of CDCs includes 1 x 10⁵, 1 x 10⁶, 1 x 10⁷, 1 x 10⁸, 1 x 10⁹ CDCs in a single dose as another baseline for exosome dosage quantity. In certain instances, this may be prorated to body weight (range 100,000-1M CDCs/kg body weight total CDC dose). In other embodiments, administering a composition includes myocardial infusion. In other embodiments, myocardial infusion is intracoronary. In other embodiments, myocardial infusion is intra-arterial or intravenous. In other embodiments, treatment of the subject results in decreased fibrosis, decreased inflammation, increased mitochondrial function and/or increased cardiomyogenesis. In other embodiments, decreased fibrosis includes a reduction in collagen accumulation. In other embodiments, collagen includes collagen I and/or collagen III. In other embodiments, decreased inflammation includes an increase in cytoplasmic nuclear factor (erythroid-derived 2)-like 2 (Nrf2), reduction in fatty acid peroxidation end products, reduced numbers of inflammatory cells, and/or upregulated expression of antioxidants. In other embodiments, antioxidants include heme oxygenase-1 (HO-1), catalase, superoxide dismutase-2 (SOD-2), and glutamate-cystein ligase catalytic (GCLC) subunit. In other embodiments, inflammatory cells include CD68+ macrophages and CD3+ T-cells. In other embodiments, increased mitochondrial function includes increased mitochondrial ultrastructure and/or increased mitochondrial biogenesis. In other embodiments, increased mitochondrial function includes increased nuclear PPAR-γ co-activator-1 (PGC-1) expression. Further examples are found in U.S. App. No. 11/666,685, 12/622,143, and 12/622,106, which are herein incorporated by reference.

Described herein is a composition including a plurality of exosomes. In certain embodiments, the plurality of exosomes are generated by a method including providing a population of cells, and isolating a plurality of exosomes from the population of cells.

In various embodiments, the cells are stem cells, progenitors and/or precursors. In other embodiments, the stem cells, progenitors and/or precursors are cardiosphere-derived cells (CDCs). In other embodiments, the stem cells, progenitors and/or precursors are pluripotent stem cells (pSCs), such as embryonic stem cells (ESCs) and induced pluripotent stem cells (iPSCs) derived from any one of various somatic sources in the body such as fibroblasts, blood and hematopoietic stem cells (hSCs), immune cells, bone and bone marrow, neural tissue, among others. In other embodiments, the stem cells, progenitors and/or precursors include hSCs, mesenchymal stem cells (MSCs), or endothelial precursor cells (EPCs). In various embodiments, the cells are stem cells, progenitors and/or precursors derived from human biopsy tissue. In various embodiments, the cells are stem cells, progenitors and/or precursors are a primary culture. In various embodiments, the cells are stem cells, progenitors and/or precursors which may constitute a cell line capable of serial passaging.

In various embodiments, the plurality of exosomes is isolated from the supernatants of the population of cells. This includes, for example, exosomes secreted into media as conditioned by a population of cells in culture, further including cell lines capable of serial passaging. In certain embodiments, the cells are cultured in serum-free media. In certain embodiments, the cells in culture are grown to 10, 20, 30, 40, 50, 60, 70, 80, 90, or 90% or more confluency when exosomes are isolated. In certain embodiments, the population of cells has been genetically manipulated. This includes, for example, knockout (KO) or transgenic (TG) cell lines, wherein an endogenous gene has been removed and/or an exogenous introduced in a stable, persistent manner. This further includes transient knockdown of one or more genes and associated coding and non-coding transcripts within the population of cells, via any number of methods known in the art, such as introduction of dsRNA, siRNA, microRNA, etc. This further includes transient expression of one or more genes and associated coding and non-coding transcripts within the population of cells, via any number of methods known in the art, such as introduction of a vector, plasmid, artificial plasmid, replicative and/or non-relicative virus, etc. In other embodiments, the population of cells has been altered by exposure to environmental conditions (e.g., hypoxia), small molecule addition, presence/absence of exogenous factors (e.g., growth factors, cytokines) at the time, or substantially contemporaneous with, isolating the plurality of exosomes in a manner altering the regulatory state of the cell. For example, one may add a differentiation agent to a population of stem cells, progenitors and/or precursors in order to promote partial or full differentiation of the cell, and thereafter derive a plurality of exosomes. In various embodiments, altering the regulatory state of the cell changes composition of one or more exosomes in the plurality of exosomes.

In various embodiments, the plurality of exosomes include one or more exosomes that are about 10 nm to about 250 nm in diameter, including those about 10 nm to about 15 nm, about 15 nm to about 20 nm, about 20 nm to about 25 nm, about 25 nm to about 30 nm, about 30 nm to about 35 nm, about 35 nm to about 40 nm, about 40 nm to about 50 nm, about 50 nm to about 60 nm3 about 60 nm to about 70 nm, about 70 nm to about 80 nm, about 80 nm to about 90 nm, about 90 nm to about 95 nm, about 95 nm to about 100 nm, about 100 nm to about 105 nm, about 105 nm to about 110 nm, about 110 nm to about 115 nm, about 115 nm to about 120 nm, about 120 nm to about 125 nm, about 125 nm to about 130 nm, about 130 nm to about 135 nm, about 135 nm to about 140 nm, about 140 nm to about 145 nm, about 145 nm to about 150 nm, about 150 to about 200 nm, about 200 nm to about 250 nm, about 250 nm or more.

In various embodiments, the plurality of exosomes includes one or more exosomes expressing a biomarker. In certain embodiments, the biomarkers are tetraspanins. In other embodiments, the tetraspanins are one or more selected from the group including CD63, CD81, CD82, CD53, and CD37. In other embodiments, the exosomes express one or more lipid raft associated protiens (e.g., glycosylphosphatidylinositol-anchored proteins and flotillin), cholesterol, sphingomyelin, and/or hexosylceramides.

In several embodiments, the plurality of exosomes includes one or more exosomes containing a biological protein. In various embodiments, the biological protein includes transcription factors, cytokines, growth factors, and similar proteins capable of modulating signaling pathways in a target cell. In various embodiments, the biological protein is capable of facilitating regeneration and/or improved function of a tissue. In various embodiments, the biological protein is capable of modulating pathways related to Irak1, Traf6, toll-like receptor (TLR) signaling pathway, NOX-4, SMAD-4, and/or TGF-β. In other embodiments, the biological protein related to exosome formation and packaging of cytosolic proteins such as Hsp70, Hsp90, 14-3-3 epsilon, PKM2, GW182 and AGO2.

In other embodiments, the plurality of exosomes includes one or more exosomes containing a signaling lipid. This includes ceramide and derivatives. In other embodiments, the plurality of exosomes includes one or more exosomes containing a coding and/or non-coding nucleic acid.

In several embodiments, the plurality of exosomes includes one or more exosomes containing microRNAs. In various embodiments, these microRNAs can include miR-146a, miR22, miR-24, miR-210, miR-150, miR-140-3p, miR-19a, miR-27b, miR-19b, miR-27a, miR-376c, miR-128, miR-320a, miR-143, miR-21, miR-130a, miR-9, miR-185, and/or miR-23a. In several embodiments, the plurality of exosomes include one or more exosomes enriched in at least one of miR-146a, miR22, miR-24, miR-210, miR-150, miR-140-3p, miR-19a, miR-27b, miR-19b, miR-27a, miR-376c, miR-128, miR-320a, miR-143, miR-21, miR-130a, miR-9, miR-185, and/or miR-23a. In several embodiments, the plurality of exosomes includes one or more exosomes enriched in at least one of miR-146a, miR-22, miR-24. Enrichment can be measured by, for example, comparing the amount of one or more of the described microRNAs when derived from cells providing salutary benefit in a therapeutic setting (e.g., cardiosphere-derived cells (CDCs) compared to cells that do not provide such a salutary benefit (e.g., fibroblasts). Enrichment may also be measured in absolute or relative quantities, such as when compared to a standardized dilution series.

In other embodiments, the plurality of exosomes can include one or more exosomes containing microRNAs. This includes various microRNAs known in the art, such as miR-23a, miR-23b, miR-24, miR-26a, miR27-a, miR-30c, let-7e, mir- 19b, miR-125b, mir-27b, let-7a, miR-19a, let-7c, miR-140-3p, miR-125a-5p, miR-132, miR-150, miR-155, mir-210, let-7b, miR-24, miR-423-5p, miR-22, let-7f, and/or miR-146a.

In other embodiments, the plurality of exosomes can include one or more exosomes containing microRNAs. This includes various microRNAs known in the art, such as miR-17, miR-21 , miR-92, miR92a, miR-29, miR-29a, miR-29b, miR-29c, miR-34, mi-R34a, miR-150, miR-451, miR-145, miR-143, miR-144, miR-193a-3p, miR-133a, miR-155, miR-181a, miR-214, miR-199b, miR-199a, miR-210, miR-126, miR-378, miR-363 and miR-30b, and miR-499. Other microRNAs known in the art include miR-92, miR-17, miR-21, miR-92, miR92a, miR-29, miR- 29a, miR-29b, miR-29c, miR-34, mi-R34a, miR-150, miR-451, miR-145, miR-143, miR- 144, miR-193a-3p, miR-133a, miR-155, miR-181a, miR-214, miR-199b, miR-199a, miR- 126, miR-378, miR-363 and miR-30b, and/or miR-499.

In several embodiments, isolating a plurality of exosomes from the population of cells includes centrifugation of the cells and/or media conditioned by the cells. In several embodiments, ultracentrifugation is used. In several embodiments, isolating a plurality of exosomes from the population of cells is via size-exclusion filtration. In other embodiments, isolating a plurality of exosomes from the population of cells includes use of discontinuous density gradients, immunoaffinity, ultrafiltration and/or high performance liquid chromatography (HPLC).

In certain embodiments, differential ultracentrifugation includes using centrifugal force from 1000-2000xg, 2000-3000xg, 3000-4000xg, 4000-5000xg, 5000xg-6000xg, 6000-7000xg, 7000-8000xg, 8000-9000xg, 9000-10,000xg, to 10,000xg or more to separate larger-sized particles from a plurality of exosome derived from the cells. In certain embodiments, differential ultracentrifugation includes using centrifugal force from 10,000-20,000xg, 20,000-30,000xg, 30,000-40,000xg, 40,000-50,000xg, 50,000xg-60,000xg, 60,000-70,000xg, 70,000-80,000xg, 80,000-90,000xg, 90,000-100,000xg, to 10,000xg or more to separate larger-sized particles from a plurality of exosome derived from the cells.

In other embodiments, isolating a plurality of exosomes from the population of cells includes use of filtration or ultrafiltration. In certain embodiments, a size exclusion membrane with different pore sizes is used. For example, a size exclusion membrane can include use of a filter with a pore size of 0.1-0.5 µM, 0.5-1.0 µM, 1-2.5 µM, 2.5-5 µM, 5 or more µM. In certain embodiments, the pore size is about 0.2 µM. In certain embodiments, filtration or ultrafiltration includes size exclusion ranging from 100-500 daltons (Da), 500-1 kDa, 1-2 kDa, 2-5 kDa, 5-10 kDa, 10-25 kDa, 25-50 kDa, 50-100 kDa, 100-250 kDa, 250-500 kDa, 500 or more kDa. In certain embodiments, the size exclusion is for about 2-5 kDa. In certain embodiments, the size exclusion is for about 3 kDa. In other embodiments, filtration or ultrafiltration includes size exclusion includes use of hollow fiber membranes capable of isolating particles ranging from 100-500 daltons (Da), 500-1 kDa, 1-2 kDa, 2-5 kDa, 5-10 kDa, 10-25 kDa, 25-50 kDa, 50-100 kDa, 100-250 kDa, 250-500 kDa, 500 or more kDa. In certain embodiments, the size exclusion is for about 2-5 kDa. In certain embodiments, the size exclusion is for about 3 kDa. In other embodiments, a molecular weight cut-off (MWCO) gel filtration capable of isolating particles ranging from 100-500 daltons (Da), 500-1 kDa, 1-2 kDa, 2-5 kDa, 5-10 kDa, 10-25 kDa, 25-50 kDa, 50-100 kDa, 100-250 kDa, 250-500 kDa, 500 or more kDa. In certain embodiments, the size exclusion is for about 2-5 kDa. In certain embodiments, the size exclusion is for about 3 kDa. In various embodiments, such systems are used in combination with variable fluid flow systems.

In other embodiments, isolating a plurality of exosomes from the population of cells includes use of tangential flow filtration (TFF) systems are used purify and/or concentrate the exosome fractions. In other embodiments, isolating a plurality of exosomes from the population of cells includes use of (HPLC) can also be used to purify exosomes to homogeneously sized particles. In various embodiments, density gradients as used, such as centrifugation in a sucrose density gradient or application of a discrete sugar cushion in preparation.

In other embodiments, isolating a plurality of exosomes from the population of cells includes use of a precipitation reagent. For example, a precipitation reagent, ExoQuick^{®}, can be added to conditioned cell media to quickly and rapidly precipitate a population of exosomes. In other embodiments, isolating a plurality of exosomes from the population of cells includes use of volume-excluding polymers (e.g., polyethylene glycols (PEGs)) are used. In another embodiment, isolating a plurality of exosomes from the population of cells includes use of flow field-flow fractionation (FlFFF), an elution-based technique.

In certain embodiments, isolating a plurality of exosomes from the population of cells includes use of one or more capture agents to isolate one or more exosomes possessing specific biomarkers or containing particular biological molecules. In one embodiment, one or more capture agents include at least one antibody. For example, antibody immunoaffinity recognizing exosome-associated antigens is used to capture specific exosomes. In other embodiments, the at least one antibody are conjugated to a fixed surface, such as magnetic beads, chromatography matrices, plates or microfluidic devices, thereby allowing isolation of the specific exosome populations of interest. In other embodiments, isolating a plurality of exosomes from the population of cells includes use of one or more capture agents that is not an antibody. This includes, for example, use of a "bait" molecule presenting an antigenic feature complementary to a corresponding molecule of interest on the exosome surface, such as a receptor or other coupling molecule. In one embodiment, the non-antibody capture agent is a lectin capable of binding to polysaccharide residues on the exosome surface.

In various embodiments, the CDCs are mammalian. In other embodiments, the CDCs are human. As disclosed above, in some embodiments, synthetic exosomes are generated, which can be isolated by similar mechanisms as those above. In various embodiments, the composition that is a plurality of exosomes is a pharmaceutical composition further including a pharmaceutically acceptable carrier.

In various embodiments, the plurality of exosomes range in size from 30 to 300 nm. In various embodiments, the plurality of exosomes range in size from 40 to 100 nm. In certain embodiments, the plurality of exosomes is cardiosphere-derived cell (CDC) exosomes. In certain embodiments, the plurality of exosomes includes exosomes that are CD63+. In various embodiments, the exosomes include microRNAs miR-146a, miR22, miR-24, miR-210, miR-150, miR-140-3p, miR-19a, miR-27b, miR-19b, miR-27a, miR-376c, miR-128, miR-320a, miR-143, miR-21, miR-130a, miR-9, miR-185, and/or miR-23a. In other embodiments, the exosomes are 2-5 kDa, such as 3 kDa. Other examples or embodiments relating to the composition and techniques involving exosomes are presented, in PCT Pub. No. WO 2014/028,493, which is fully incorporated herein by reference.

Described herein is a method for treatment including, selecting a subject in need of treatment, administering a composition including a plurality of exosomes to the individual, wherein the administration of the composition treats the subject. In certain embodiments, the subject is in need to treatment for a disease and/or condition involving tissue damage or dysfunction. In other embodiments, the disease and/or condition involving tissue damage or dysfunction is heart disease. In other embodiments, the plurality of exosomes includes exosomes including one or more microRNAs.

In certain embodiments, the plurality of exosomes are generated by a method including providing a population of cells, and isolating a plurality of exosomes from the population of cells. In various embodiments, the cells are stem cells, progenitors and/or precursors. In other embodiments, the stem cells, progenitors and/or precursors are cardiosphere-derived cells (CDCs). In other embodiments, the stem cells, progenitors and/or precursors are pluripotent stem cells (pSCs), such as embryonic stem cells (ESCs) and induced pluripotent stem cells (iPSCs) derived from any one of various somatic sources in the body such as fibroblasts, blood and hematopoietic stem cells (hSCs), immune cells, bone and bone marrow, neural tissue, among others. In other embodiments, the stem cells, progenitors and/or precursors includes hSCs, mesenchymal stem cells (MSCs), or endothelial precursor cells (EPCs). In various embodiments, the cells are stem cells, progenitors and/or precursors derived from human biopsy tissue. In various embodiments, the cells are stem cells, progenitors and/or precursors are a primary culture. In various embodiments, the cells are stem cells, progenitors and/or precursors are a cell line capable of serial passaging. In certain embodiments, the exosomes are synthetic.

In various embodiments, the plurality of exosomes is derived from cardiosphere-derived cells (CDCs). In other embodiments, the plurality of exosomes includes exosomes including one or more biological molecules. In other embodiments, the plurality of exosomes includes exosomes enriched for one or more biological molecules when derived from CDCs compared to exosome derived from non-CDC sources. In various embodiments, the one or more biological molecules are proteins, growth factors, cytokines, transcription factors and/or morphogenic factors. In other embodiments, the plurality of exosomes includes exosomes enriched for one or more biological molecules includes microRNAs, further including microRNAs that are enriched when derived from CDCs compared to exosome derived from non-CDC sources. In various embodiments, these microRNAs can include miR-146a, miR22, miR-24, miR-210, miR-150, miR-140-3p, miR-19a, miR-27b, miR-19b, miR-27a, miR-376c, miR-128, miR-320a, miR-143, miR-21, miR-130a, miR-9, miR-185, and/or miR-23a. In several embodiments, the plurality of exosomes includes one or more exosomes enriched in at least one of miR-146a, miR-22, miR-24.

In various embodiments, the CDCs are mammalian. In other embodiments, the CDCs are human. In certain embodiments, the exosomes are synthetic. In certain embodiments, the synthetic exosomes possess substantially similar content (e.g., microRNAs, biological molecules) as exosomes derived from CDCs.

In various embodiments, administration of the plurality of exosomes alters gene expression in the damaged or dysfunctional tissue, improves viability of the damaged tissue, and/or enhances regeneration or production of new tissue in the individual. In various embodiments, the quantities of exosomes that are administered to achieved these effects range from 1 x 10⁶ to 1 x 10⁷, 1 x 10⁷ to 1 x 10⁸, 1 x 10⁸ to 1 x 10⁹, 1 x 10⁹ to 1 x 10¹⁰, 1 x 10¹⁰ to 1 x 10¹¹, 1 x 10¹¹ to 1 x 10¹², 1 x 10¹² or more. In other embodiments, the numbers of exosomes is relative to the number of cells used in a clinically relevant dose for a cell-therapy method. For example, it has been demonstrated that 3mL / 3 x 10⁵ CDCs, is capable of providing therapeutic benefit in intracoronary administration, and therefore, a plurality of exosomes as derived from that number of cells in a clinically relevant dose for a cell-therapy method. In various embodiments, administration can be in repeated doses. In another example, the number of administered CDCs includes intracoronary 25 million CDCs per coronary artery (i.e., 75 million CDCs total) as another baseline for exosome dosage quantity. In various embodiments, the numbers of CDCs includes 1 x 10⁵, 1 x 10⁶, 1 x 10⁷, 1 x 10⁸, 1 x 10⁹ CDCs in a single dose as another baseline for exosome dosage quantity. In certain instances, this may be prorated to body weight (range 100,000-1M CDCs/kg body weight total CDC dose). In various embodiments, exosome quantity may be defined by protein quantity, such as dosages including 1-10, 10-25, 25-50, 50-75, 75-100, or 100 or more mg exosome protein.

Defining an effective dose range, dosing regimen and route of administration, may be guided by studies using fluorescently labeled exosomes, and measuring target tissue retention, which can be >10X, >50X, or >100X background, as measured 5, 10, 15, 30, or 30 or more min as a screening criterion. In certain embodiments, >100X background measured at 30 mins is a baseline measurement for a low and high dose that is then assess for safety and bioactivity (e.g., using MRI endpoints: scar size, global and regional function). In various embodiments, single doses are compared to two, three, four, four or more sequentially-applied doses. In various embodiments, the repeated or sequentially-applied doses are provided for treatment of an acute disease and/or condition. In various embodiments, the repeated or sequentially-applied doses are provided for treatment of a chronic disease and/or condition.

In various embodiments, administration of exosomes to the subject occurs through any of known techniques in the art. In some embodiments, this includes percutaneous delivery. In other embodiments, myocardial infusion is used, for example, the use of intracoronary catheters. In various embodiments, delivery can be intra-arterial or intravenous. Additional delivery sites include any one or more compartments of the heart, such as arterial, venous, and/or ventricular locations. In certain embodiments, administration can include delivery to a tissue or organ site that is different from the site or diseased and/or dysfunctional tissue. In certain embodiments, the delivery is via inhalation or oral administration.

In various embodiments, administration of the plurality of exosomes alters gene expression in the damaged or dysfunctional tissue, improves viability of the damaged tissue, and/or enhances regeneration or production of new tissue in the individual. In various embodiments, administration of the exosomes results in functional improvement in the tissue. In several embodiments, the damaged or dysfunctional tissue includes cardiac tissue.

For example, in certain embodiments in which cardiac tissue is damaged or dysfunctional, functional improvement may include increased cardiac output, contractility, ventricular function and/or reduction in arrhythmia (among other functional improvements). For other tissues, improved function may be realized as well, such as enhanced cognition in response to treatment of neural damage, improved blood-oxygen transfer in response to treatment of lung damage, improved immune function in response to treatment of damaged immunological-related tissues.

In various embodiments, administration of the plurality of exosomes alters gene expression in the damaged or dysfunctional tissue, improves viability of the damaged tissue, and/or enhances regeneration or production of new tissue in the individual. In various embodiments, administration of the exosomes results in functional improvement in the tissue. In several embodiments, the damaged or dysfunctional tissue includes skeletal muscle tissue.

For example, in certain embodiments in which skeletal muscle tissue is damaged or dysfunctional, functional improvement may include increased contractile strength, improved ability to walk (for example, and increase in the six-minute walk test results), improved ability to stand from a seated position, improved ability to sit from a recumbent or supine position, or improved manual dexterity such as pointing and/or clicking a mouse.

In various embodiments, the damaged or dysfunctional tissue is in need of repair, regeneration, or improved function due to an acute event. Acute events include, but are not limited to, trauma such as laceration, crush or impact injury, shock, loss of blood or oxygen flow, infection, chemical or heat exposure, poison or venom exposure, drug overuse or overexposure, and the like. In other embodiments, the damaged tissue is cardiac tissue and the acute event includes a myocardial infarction. In some embodiments, administration of the exosomes results in an increase in cardiac wall thickness in the area subjected to the infarction.

In other embodiments, tissue is also subject to damage due to chronic disease, such as for example congestive heart failure, including as conditions secondary to diseases such as Duchenne muscular dystrophy, ischemic heart disease, hypertension, valvular heart disease, dilated cardiomyopathy, infection, diabetes, and the like. In various embodiments, the administration can be in repeated doses, such as two, three, four, four or more sequentially-applied doses. In various embodiments, the repeated or sequentially-applied doses are provided for treatment of an acute disease and/or condition. In various embodiments, the repeated or sequentially-applied doses are provided for treatment of a chronic disease and/or condition.

Other sources of damage also include, but are not limited to, injury, age-related degeneration, cancer, and infection. In several embodiments, the regenerative cells are from the same tissue type as is in need of repair or regeneration. In several other embodiments, the regenerative cells are from a tissue type other than the tissue in need of repair or regeneration.

In certain embodiments, the method of treatment includes, selecting a subject in need of treatment for a heart related disease and/or condition, administering a composition including a plurality of exosomes to the individual, wherein the administration of the composition treats thesubject. In various embodiments, the heart related disease and/or condition includes heart failure, further including Duchenne muscular dystrophy related heart failure. In various embodiments, the plurality of exosomes range in size from 30 to 300 nm. In various embodiments, the plurality of exosomes range in size from 40 to 100 nm. In certain embodiments, the plurality of exosomes are cardiosphere-derived cell (CDC) exosomes. In certain embodiments, the plurality of exosomes include exosomes that are CD63+. In various embodiments, the exosomes include microRNAs miR-146a, miR22, miR-24, miR-210, miR-150, miR-140-3p, miR-19a, miR-27b, miR-19b, miR-27a, miR-376c, miR-128, miR-320a, miR-143, miR-21, miR-130a, miR-9, miR-185, and/or miR-23a. In other embodiments, the exosomes are 2-5 kDa, such as 3 kDa. In other embodiments, administering a composition includes a dosage of 1 x 10⁸, 1 x 10⁸ to 1 x 10⁹, 1 x 10⁹ to 1 x 10¹⁰, 1 x 10¹⁰ to 1 x 10¹¹, 1 x 10¹¹ to 1 x 10¹², 1 x 10¹² or more exosomes. For example, it has been demonstrated that 3mL / 3 x 10⁵ CDCs, is capable of providing therapeutic benefit in intracoronary administration, and therefore, a plurality of exosomes as derived from that number of cells in a clinically relevant dose for a cell-therapy method. In various embodiments, administration can be in repeated doses. In another example, the number of administered CDCs includes intracoronary 25 million CDCs per coronary artery (i.e., 75 million CDCs total) as another baseline for exosome dosage quantity. In various embodiments, the numbers of CDCs includes 1 x 10⁵, 1 x 10⁶, 1 x 10⁷, 1 x 10⁸, 1 x 10⁹ CDCs in a single dose. In certain instances, this may be prorated to body weight (range 100,000-1M CDCs/kg body weight total CDC dose). In various embodiments, exosome quantity may be defined by protein quantity, such as dosages including 1-10, 10-25, 25-50, 50-75, 75-100, or 100 ore more mg exosome protein. In various embodiments, administering a composition includes multiple dosages of the exosomes. In various embodiments, the repeated or sequentially-applied doses are provided for treatment of an acute disease and/or condition. In various embodiments, the repeated or sequentially-applied doses are provided for treatment of a chronic disease and/or condition. In other embodiments, administering a composition includes percutaneous injection. In other embodiments, administering a composition includes myocardial infusion. In other embodiments, administering a composition includes use of a intracoronary catheter. In other embodiments, administration a composition includes intra-arterial or intravenous delivery.

Further described herein is a method of improving cardiac performance in a subject including, selecting a subject, administering a composition including a plurality of exosomes to the individual, wherein the administration of the composition improves cardiac performance in the subject. In other embodiments, improving cardiac performance can be demonstrated, by for example, improvements in baseline ejection volume. In other embodiments, improving cardiac performance relates to increases in viable tissue, reduction in scar mass, improvements in wall thickness, regenerative remodeling of injury sites, enhanced angiogenesis, improvements in cardiomyogenic effects, reduction in apoptosis, and/or decrease in levels of pro-inflammatory cytokines.

In certain embodiments, the method of improving cardiac performance includes, selecting a subject in need of treatment for a heart related disease and/or condition, administering a composition including a plurality of exosomes to the individual, wherein the administration of the composition treats thesubject. In various embodiments, the heart related disease and/or condition includes heart failure, further including Duchenne muscular dystrophy related heart failure. In various embodiments, the plurality of exosomes range in size from 30 to 300 nm. In various embodiments, the plurality of exosomes range in size from 40 to 100 nm. In certain embodiments, the plurality of exosomes are cardiosphere-derived cell (CDC) exosomes. In certain embodiments, the plurality of exosomes include exosomes that are CD63+. In various embodiments, the exosomes include microRNAs miR-146a, miR22, miR-24, miR-210, miR-150, miR-140-3p, miR-19a, miR-27b, miR-19b, miR-27a, miR-376c, miR-128, miR-320a, miR-143, miR-21, miR-130a, miR-9, miR-185, and/or miR-23a. In other embodiments, the exosomes are 2-5 kDa, such as 3 kDa. In other embodiments, administering a composition includes a dosage of 1 x 10⁸, 1 x 10⁸ to 1 x 10⁹, 1 x 10⁹ to 1 x 10¹⁰, 1 x 10¹⁰ to 1 x 10¹¹, 1 x 10¹¹ to 1 x 10¹², 1 x 10¹² or more exosomes. For example, it has been demonstrated that 3mL / 3 x 10⁵ CDCs, is capable of providing therapeutic benefit in intracoronary administration, and therefore, a plurality of exosomes as derived from that number of cells in a clinically relevant dose for a cell-therapy method. In various embodiments, administration can be in repeated doses. In another example, the number of administered CDCs includes intracoronary 25 million CDCs per coronary artery (i.e., 75 million CDCs total) as another baseline for exosome dosage quantity. In various embodiments, the numbers of CDCs includes 1 x 10⁵, 1 x 10⁶, 1 x 10⁷, 1 x 10⁸, 1 x 10⁹ CDCs in a single dose. In certain instances, this may be prorated to body weight (range 100,000-1M CDCs/kg body weight total CDC dose). In various embodiments, exosome quantity may be defined by protein quantity, such as dosages including 1-10, 10-25, 25-50, 50-75, 75-100, or 100 or more mg exosome protein. In various embodiments, administering a composition includes multiple dosages of the exosomes. In various embodiments, the repeated or sequentially-applied doses are provided for treatment of an acute disease and/or condition. In various embodiments, the repeated or sequentially-applied doses are provided for treatment of a chronic disease and/or condition. In other embodiments, administering a composition includes percutaneous injection. In other embodiments, administering a composition includes myocardial infusion. In other embodiments, administering a composition includes use of a intracoronary catheter. In other embodiments, administration a composition includes intra-arterial or intravenous delivery.

Herein the Inventors demonstrate that cardiosphere-derived cells (CDCs), in advanced clinical testing for therapeutic regeneration after myocardial infarction, reverse the key pathophysiological hallmarks of Duchenne cardiomyopathy (oxidative stress, inflammation, fibrosis and mitochondrial dysfunction) in mdx mice. Exosomes secreted by human CDCs reproduce the benefits of CDCs in mdx mice, and reverse abnormalities of calcium cycling and mitochondrial respiration in human Duchenne cardiomyocytes. Both CDCs and their exosomes improve heart function in mdx mice; a single injection of CDCs suffices to increase maximal exercise capacity and improve survival. Delivery of a microRNA enriched in CDC exosomes, miR-148a, mimics key effects of CDCs and CDC exosomes. Thus, CDCs ameliorate Duchenne cardiomyopathy via exosome-mediated transfer of signaling molecules including miR-148a. The present findings motivate clinical testing of CDCs in patients with Duchenne cardiomyopathy.

### Example 1

### CDC Culture

Endomyocardial biopsies from the right ventricular aspect of the interventricular septum are obtained from healthy hearts of deceased tissue donors. Cardiosphere-derived cells were derived as described previously. *See* Makkar et al., (2012). "Intracoronary cardiosphere-derived cells for heart regeneration after myocardial infarction (CADUCEUS): a prospective, randomized phase 1 trial." Lancet 379, 895-904 (2012), which is fully incorporated by reference herein.

In brief, heart biopsies are minced into small fragments and briefly digested with collagenase. Explants were then cultured on 20 mg/ml fibronectin-coated dishes. Stromal-like flat cells and phase-bright round cells grow out spontaneously from tissue fragments and reach confluence by 2-3 weeks. These cells are harvested using 0.25% trypsin and cultured in suspension on 20 mg/ml poly d-lysine to form self-aggregating cardiospheres. cardiosphere-derived cells (CDCs) are obtained by seeding cardiospheres onto fibronectin-coated dishes and passaged. All cultures are maintained at 5% CO2 at 37°C, using IMDM basic medium supplemented with 20% FBS, 1% penicillin/streptomycin, and 0.1 ml 2-mercaptoethanol.

### Example 2

### Media Conditioning and Exosome Purification

Exosomes are harvested from CDCs at passage 4. One can also isolate exosomes from normal human dermal fibroblasts (NHDF), cells that have been previously utilized as controls providing no salutary benefit, as a control.

CDCs and NHDFs are conditioned in serum-free media for 15 days at 100% confluence. Aspirated media is then centrifuged at 3,000xg for 15 min to remove cellular debris. Exosomes were then isolated using Exoquick Exosome Precipitation Solution.

Exosome pellets are resuspended in the appropriate media and used for assays. Expression of the conserved exosome marker CD63 is verified using ELISA. RNA content of exosome pellets can also be quantified using a Nanodrop spectrophotometer. For generation of miR-146a-deficient exosomes, CDC are transfected in suspension with miRIDIAN miR-146a hairpin inhibitor or a miRIDIAN hairpin control and seeded on to fibronectin-coated flasks. Exosomes are isolated from serum-free conditioned media (48 hr conditioning).

### Example 3

### Exosomal RNA Degradation

Exosomal RNA degradation is performed by suspending exosome pellets in 2 ml of PBS. To one sample, 100 ml of Triton X-100 (Sigma Aldrich) is added to achieve 5% triton concentration. Exosomes are treated with 0.4 mg/ml RNase A treatment for 10 min at 37°C. Samples are further treated with 0.1 mg/ml Proteinase K for 20 min at 37°C. RNA is purified from samples using an microRNA isolation kit. RNA levels are measured using Nanodrop.

### Example 4

### Mass Spectrometry Analysis on Exosome Pellets

Proteins are prepared for digestion using the filter-assisted sample preparation (FASP) method. Concentrations re measured using a Qubitfluorometer. Trypsin is added at a 1:40 enzyme-to-substrate ratio and the sample incubated overnight on a heat block at 37°C. The device was centrifuged and the filtrate collected. Digested peptides are desalted using C18 stop-and-go extraction (STAGE) tips. Peptides are fractionated by strong anion exchange STAGE tip chromatography. Peptides re eluted from the C18 STAGE tip and dried. Each fraction is analyzed with liquid chromatography-tandem mass spectrometry. Samples are loaded to a 2 cm x 100 mm I.D. trap column. The analytical column is 13 cm x 75 mm I.D. fused silica with a pulled tip emitter. The mass spectrometer is programmed to acquire, by data-dependent acquisition, tandem mass spectra from the top 15 ions in the full scan from 400 to 1,400 m/z. Mass spectrometer RAW data files re converted to MGF format using msconvert. MGF files re searched using X!Hunter against the latest spectral library available on the GPM at the time. MGF files are also searched using X!!Tandem using both the native and k-score scoring algorithms and by OMSSA. Proteins re required to have one or more unique peptides with peptide E-value scores of 0.01 or less from X!!Tandem, 0.01 or less from OMSSA, 0.001 or less and theta values of 0.5 or greater from X!Hunter searches, and protein E-value scores of 0.0001 or less from X! !Tandem and X!Hunter.

### Example 5

### Myocyte Isolation, Angiogenesis Assay

For studies establishing the effects of exosome application, a variety of cell types can be used. For example, neonatal rat cardiomyoctes (NRCMs) can be isolated from 1- to 2-day-old Sprague Dawley rat pups and cultured in monolayers. Another useful source includes human vein umbilical vein endothelial cells plated on growth factor-deprived Matrigel (BD Biosciences) to assay angiogenesis.

Cells are then incubated with 7x10⁸ and 4.0x10⁸ CDC exosomes or NHDF exosomes, respectively. Difference in doses reflects the different exosome output from cells during conditioning. Cells were allowed to produce exosomes under similar conditions such that the relative doses might be representative of the relative exosome production *in vivo.* Four hours later, tube formation was measured.

### Example 6

### In Vitro Cardiomyocyte Assay Exosome Treatment

The Inventors plated 1.5x10⁴ NRCMs in fibronectin-coated eight-chamber slides. After 5 days, media is replaced with new fresh media containing 3.5x10⁸ or 2x10⁸ CDC or NHDF exosomes, respectively. Cells are then fixed with 4% paraformaldehyde for 30 min at 4°C. Chambers are washed three times with cold PBS then blocked and permeabilized with Dako/0.1% Saponin (Invitrogen) for 1 hr at 37°C. Cells are incubated (overnight, 4°C) with rabbit anti-Ki-67 (1:100) primary antibody and mouse anti-a-sarcomericactinin (Abcam). Cells are then washed three times with PBS and incubated with goat antimouse (Cy5) and goat antirabbit (FITC) in TUNEL stain solution for 1 hr at 37°C. Slides are then washed three times in PBS, stained with 1:8,000 40,6-diamidino-2-phenylindole stain solution, and mounted using Prolong antifade solution(Invitrogen). Slides were imaged using confocal microscopy.

### Example 7

### Cardiomyocyte Stress Assay

One injury model can include use of NRCMs plated in a monolayer on fibronectin-coated 12-well plates and treated with either 40 nM of miR-146a or mimic for 24 hr. Media is then changed and cells were washed three times with PBS. Cells are then stressed using hydrogen peroxide (100 mM H2O2 in serum free media for 2 hr) or cobalt chloride (5mMCoCl2 in serum-free media for 2 hr). Viability is measured by washing cells with PBS and treating with 20 mM Calcein PBS solution for 20 min at 37°C in dark conditions. Fluorescence is read using a Soft Max Pro 5 Plate Reader (Molecular Devices). Data per well is the average of nine consecutive measurements.

A second model includes plating cardiomyocytes on 25 mm precoated glass coverslips (Fischer Scientific) in six-well plates. Cells are stressed using 50 mM H2O2 for 15min followed incubation with transwell membrane inserts containing CDCs or incubation with CDC exosomes for 4 hr. Cells are then washed, fixed with paraformaldehyde, and stained for analysis as explained above.

### Example 8

### Exosome Inhibition in CDCs

CDCs are grown to confluence in T175 flasks. For *in vitro* studies, CDCs were conditioned for 15 days in 20 mM GW4869 (Sigma Aldrich), serum-free media, or serum-free media containing an equivalent volume of DMSO. For *in vitro* transwell insert assays of cardiomyocyte stress, one can treat CDCs with 20 mM GW4869 (Sigma Aldrich), or 5 mM Spiroepoxide (Santa Cruz Biotechnology) for 12 hr. CDCs are washed three times in PBS and supplanted with serum-free media. Inserts containing treated CDCs are added into six-well plates containing cardiomyocytes. For *in vivo* studies, CDCs are treated with 20 mM GW4869 or an equivalent volume of DMSO for 12 hr. Prior to injection, CDC flasks are washed twice with PBS, trypsinized, and counted; 10⁵ CDCs were injected per animal.

### Example 9

### Acute and Chronic Myocardial Infarction Model

Three-month-old male severe combined immunodeficient (SCID)- beige mice are anesthetized with isoflurane. Following surgical preparation, a 2 cm vertical incision is introduced in the midclavicular line for a lateral thoracotomy. The left anterior descending was ligated using 7-0 silk. Animals are injected with exosomes, microRNAs, CDCs, or media control at two peri-infarct sites with a volume of 40 ml per injection.

For the chronic model of MI, animals are infarcted as described above without any treatment administration. Three weeks later, the animals are given the treatment in the same manner as above. For exosome treatments, pellets are resuspended in Iscove's Modified Dulbecco's (IMDM) basal media. Animals are injected with 2.8x10⁹ and 1.56x10⁹ of CDC and NHDF exosomes, respectively. microRNA-treated animals are injected with 80 ng of miR-146a or microRNA mimic control. In brief, miRIDIAN miR-146a or miRIDIAN negative control is vortexed in Dharmafect (Thermo Scientific) transfection reagent and IMDM basal media and incubated for 10 min at room temperature to allow complexes to form. microRNA complexes are resuspended in IMDM for injection. For CDC treatments, animals are injected with 10⁵ CDCs as described.

Echocardiography SCID beige mice are evaluated via echocardiography 24 hr (baseline), 14 days, and 4 weeks after surgery using Vevo 770 Imaging System (Visual Sonics). After induction of light general anesthesia, hearts are 3D imaged in the long axis view at the level of maximum left ventricular diameter. Left ventricular ejection fraction can be measured with Visual Sonics version 1.3.8 software from 2D views of LV end-diastolic and LV end-systolic area. Each animal/time point is measured multiple times and the average used for statistical analysis.

### Example 10

### Histology

Animals are sacrificed 4 weeks after MI. Hearts are harvested and a transverse cut is made slightly above the MI suture. The apical portion is then imbedded in optimum cutting temperature solution in a base mold/embedding ring block. Blocks are immediately frozen by submersion in cold 2-methylbutane. Hearts are sectioned at a thickness of 5 mM.

### Example 11

### Masson's Trichrome Staining

Two slides containing a total of four sections per heart are stained using Masson's trichrome stain. In brief, sections are treated overnight in Bouin's solution. Slides are then rinsed for 10 min under running water and stained with Weigert's hematoxylin for 5 min. Thereafter, slides are then rinsed and stained with scarlet-acid fuchsin for 5 min and rinsed again. Slides are then further stained with phosphotungstic/phosphomolybdic, aniline blue, and 2% acetic acid for 5 min each. Slides were then rinsed, dried, and mounted using DPX mounting media.

### Example 12

### Morphometry

Morphometric analysis of heart sections was performed using Image J software. Briefly, 2D images of stained sections are split into blue, red, and green channels (only the blue was used). Infarct size can be established by measuring area and intensity of blue in each section to calculate infarct size. Percent viable and infarct mass were calculated by averaging percent infarct across four sections analyzed per heart. Infarct and viable masses were calculated as the product of the infarct or viable tissue, the height of the average mouse heart (3 mm) and the specific gravity of heart tissue (1.05 g/ml). Infarct wall thickness is calculated by measuring the thinnest area of the infarct. In a chronic model of MI where significant hypertrophy and adverse remodeling took place, one can adjust the viable mass of each heart based on the derived mass of cardiomyocytes in the tissue.

Myocyte mass is obtained by measuring the cross-sectional area of perpendicularly sectioned cardiomyocytes (defined as round cells with red cytoplasm and a visible nucleus in the center). The Inventors measured at least 25 myocytes per heart. Myocyte volume is quantified using the simplifying assumption of a cylindrical shape; mass was derived by multiplying volumes by the specific gravity of a cardiomyocyte (1.15 mg/ml). The viable mass of each mouse heart was divided by the mass of the cardiomyocytes in that heart.

### Example 13

### CDC Exosomes Enhance Angiogenesis and Promote Cardiomyocyte Survival and Proliferation

Exosomes are isolated from serum-free media conditioned over 15 days by cultured human CDCs (or normal human dermal fibroblasts (NHDFs) as a therapeutically inert control) (Figure 7 available online). By the end of the conditioning period, most of the CDCs remained alive despite the absence of regular media changes (Figures 7B and 7C). Purified exosome pellets were enriched in RNA (Figure 1A). The Inventors confirmed that the RNA resides within exosomes by exposing the pellet to RNase A in the presence of 5% triton (Figure 1B), with proteinase K added to dissociate protein complexes that may shield RNA. Mass spectrometry confirmed the presence of conserved exosomal biogenesis proteins (Figure 1C) including CD63, which the Inventors used to quantify exosome yield (Figure 1D). Transmission electron microscopy revealed most exosomes to be 30-90 nm in diameter, although smaller and larger particles were also present (Figures 1E and 1F), consistent with reports of exosomes derived from vascular cells. *In vitro* assays revealed major effects of CDC exosomes on angiogenesis, cardiomyocyte proliferation, and apoptosis.

CDC exosomes, but not NHDF exosomes, promoted tube formation in human umbilical cord endothelial cells, indicative of enhanced angiogenesis (Figure 1G). CDC-exosome-treated neonatal cardiomyocytes proliferated more than those exposed to NHDF exosomes or media only, as evidenced by higher proportions of Ki67-positive nuclei (Figure 1H). In addition, CDC-exosome-treated cardiomyocytes exhibited fewer terminal deoxynucleotidyltransferase nick end labeling (TUNEL)-positive nuclei (Figure 1I). Thus, CDC exosomes stimulate angiogenesis, promote cardiomyocyte proliferation, and decrease programmed cell death. These effects reproduce those of the parent CDCs.

### Example 14

### CDC Exosomes Improve Cardiac Function, Impart Structural Benefits, and Increase Viable Mass after MI

It is known that CDCs stimulate functional improvement and regeneration in the infarcted myocardium in both animals and humans, but of central important to the present technology is whether exosomes derived from CDCs can reproduce or are indispensable to these processes. To assess therapeutic efficacy in an established preclinical model, the Inventors induced acute MI in immunodeficient mice then injected CDC exosomes, NHDF exosomes, or serum-free media into the MI border zone.

At 15 and 30 days after injection, global heart function was greater in animals injected with CDC exosomes compared with NHDF exosomes or media controls (Figure 2A). At the histological level, CDC-exosome-treated hearts exhibited decreased scar mass, increased viable mass, and increased infarcted wall thickness compared to NHDF exosome and media controls (Figures 2B-2E). Proinflammatory cytokine levels were also lower in CDC-exosome-treated hearts (Figure 8). In all these respects, CDC exosomes mimic the known benefits of CDCs themselves.

The acute MI model, while used extensively to assess bioactivity, cannot distinguish cardioprotective effects from genuine regeneration. To make this distinction, the Inventors performed another set of experiments in which the Inventors injected exosomes 21 days after MI, when myocardial scar is well established. Three weeks later, hearts injected with CDC exosomes showed multiple structural and functional benefits: improved ejection fraction (Figure 2F; also improved fractional area change, Figure 9A), lower scar mass (representative images in Figure 2G and pooled data in Figure 2H), higher viable mass (Figure 2I), and thicker infarcted walls (Figure 2J). Moreover, hearts treated with CDC exosomes exhibited less chamber dilation (Figures 9B and 9C), smaller infarct circumference (Figure 9D), and diminished compensatory myocyte hypertrophy (Figures 9E and 9F) relative to the grossly distorted control hearts. The density of microvessels was increased (Figures 2K and 9G) and apoptotic cardiomyocyte nuclei were less frequent (Figures 9H and 9I) in CDC-exosome-treated hearts. The net growth of new myocardium in the setting of established scar fulfills the central criterion for therapeutic regeneration; the improvement in function and the attenuation of adverse remodeling attest to the physiological significance of the tissue changes. The Inventors conclude that CDC exosomes indeed mediate genuine cardiac regeneration, while favoring angiogenesis and tissue preservation.

### Example 15

### Inhibition of Exosome Secretion Attenuates CDC Benefit

If exosomes mediate the therapeutic effects of CDC transplantation, then inhibition of exosome secretion would logically be expected to block the benefits. To test this concept, the Inventors treated CDCs with GW4869, a reversible inhibitor of neutral sphingomyelinase that prevents exosome release. Exposure to GW4869 blocked exosome production in a dose-dependent manner (Figure 3A), with complete suppression at 20 mM (a dose without apparent short-term cytotoxicity; e.g., no impairment of proliferation; Figure 3B).

Suppression of exosome release abrogated the indirect benefits of CDCs *in vitro* because media conditioned by GW4869-treated CDCs did not enhance cardiomyocyte proliferation or attenuate apoptosis (Figures 3C and 3D). Spiroepoxide, a specific irreversible inhibitor of neutral sphingomyelinase, mimicked the antiapoptotic effects of GW4869 on stressed cardiomyocytes (Figures 10A and 10B). *In vivo,* CDCs pretreated with GW4869 exerted no functional (Figure 3E) or structural (Figures 3F-3I) benefits in acute MI, in contrast to vehicle-only (DMSO) controls that conferred all the expected therapeutic effects of CDCs. Thus, exosome secretion by CDCs is required for CDCmediated benefits *in vitro* and *in vivo.*

### Example 16

### CDC Exosomes Are Enriched in miR-146a, which Plays an Important Role in MI Pathology

To investigate the basis of the therapeutic benefit of CDC exosomes, the Inventors compared their microRNA repertoire to that of NHDF exosomes using a PCR microarray of the 88 best-defined microRNAs. The microRNA content of the two cell types differed dramatically. Forty-three microRNAs were differentially present in the two groups; among these, miR-146a was the most highly enriched in CDC exosomes (262-fold higher than in NHDF exosomes; Figures 4A, 4B, and 11). Furthermore, miR-146a tissue levels were increased in post-MI hearts from animals injected with CDC exosomes relative to those injected with NHDF exosomes (Figure 4C), rendering plausible the idea that CDC exosomes might act via miR-146a transfer. Exposure of neonatal rat cardiomyocytes to a miR-146a mimic increased cardiomyocyte viability and protected against oxidant stress (Figures 4D and 12A). Whole-transcriptome microarrays revealed downregulation of Irak1 and Traf6, two signaling mediators of the TLR-NFkB axis that are known targets of miR-146a (Figure 4E). Ingenuity pathway analysis pointed to changes in pathways involved in cell survival, cell cycling, cellular organization, and morphology, all of which are relevant to ischemic injury (Figure 11D) and share links to the basal transcription factor Myc (Figure 11E). To probe the biological role of miR-146a in myocardial injury, the Inventors induced acute MI in miR-146a knockout (146a KO) mice and compared them with wild-type mice of the same strain (WT), as well as 146a KO mice "rescued" by injection of a miR-146a mimic at the time of MI (146a KO-R).

After MI, the 146a KO mice showed deeply impaired heart function and adverse remodeling compared to WTor 146a KO-R (Figures 4F and 4G). Histological analysis revealed significant increases in scar mass (Figure 4H) and decreases in infarct wall thickness in the 146a KO, but not in WT or 146a KO-R (Figure 4J). Viable mass was greatest in the 146a KO-R group (Figure 4I), perhaps indicating a supraphysiological effect of the injected miR-146a mimic. These findings point to a critical role of miR-146a in MI and give reason to suspect that miR-146a may mediate some of the therapeutic benefits of CDC exosomes.

Importantly, the Inventors further established that miR-146a leads to thicker infarct wall thickness and increased viable tissue in a mouse model of myocardial infarct. To investigate the contribution of miR-146a to the greater exosome effect, the Inventors developed miR-146a-deficient exosomes by transfecting CDCs with a miR-146a hairpin inhibitor (or a control hairpin) followed by media conditioning and exosome isolation. Successful knockdown of miR-146a was confirmed by qPCR on resultant exosomes and on NRVMs exposed to either control or miR-146adepleted exosomes (Figures 12B and 12C). The antiapoptotic effect of CDC exosomes was evident by comparing TUNEL positivity in untreated NRVMs (left column, Figure 5A) to that in NRVMs treated with control CDC exosomes (right column, Figure 5A). Exosomes deficient in miR-146a conferred less protection from oxidant stress (middle column, Figure 5A) than did control CDC exosomes, but still significantly suppressed apoptosis. These data hint that miR-146a underlies some, but not all, of the beneficial effect of CDC exosomes. To further probe this question *in vivo,* the Inventors implemented the same MI models as in Figure 2 but injecting either a miR-146a mimic or a microRNA mimic control. Mice injected with miR-146a mimic during acute MI exhibited improved pump function (Figure 5B), decreased scar mass, and increased viable heart tissue (Figures 5C-5F). In the chronic MI model, where regeneration can be studied more rigorously, animals treated with miR-146a showed only minor, statistically insignificant functional improvement (Figures 5G and 13A). Furthermore, histological analysis showed no difference in scar mass (Figures 5H and 5I). However, hearts treated with miR-146a mimic did show increased viable tissue, thicker infarcted walls (Figures 5J and 5K), and less adverse remodeling than controls (Figures 13B-13D). Evaluation of angiogenesis showed no significant differences between the two groups (Figures 5L and 13G). However, lower frequencies of cardiomyocyte apoptosis were observed in the miR-146a-injected hearts (Figures 13H and 13I), consistent with the *in vitro* data (Figure 5A). Thus, in the chronic MI model, miR-146a reproduces the cardiomyogenic and antiapoptotic effects, but not the remaining functional and structural benefits, of CDC exosomes (cf. Figures 2F-2J and 9A-9C). Exogenous miR-146a is known to suppress ischemia/reperfusion injury via targeting of Irak-1 and Traf6, both involved in the toll-like receptor (TLR) signaling pathway. TLR signaling underlying innate immunity plays a major role in the pathology of sterile inflammation, including MI.

The CDC-exosome-mediated reductions of proinflammatory cytokines (Figure 8) and suppression of Irak1 and Traf6 by miR-146a (which is augmented in hearts injected with CDC exosomes; Figure 4C) are consistent with blunted TLR signaling. In addition, miR-146a suppresses NOX-4, which has been shown to impart oxidative stress and to potentiate cardiac injury, and SMAD4, a member of the transforming growth factor b (TGF-b) profibrotic pathway. To confirm that these targets are indeed downregulated, the Inventors performed western blots on chronic MI hearts 7 days after treatment with miR-146a. Indeed, all of the aforementioned targets were silenced in miR-146a-treated hearts compared to mimic control (Figures 6A and 6B). The Inventors also found lower levels of myeloperoxidase, a surrogate of neutrophil infiltration.

### Example 17

### Differences in baseline ejection fraction between different mouse strains

The Inventors observed a noticeably high baseline ejection fraction for these animals. It was surmised that this difference is due to the different background strain of mice used in the knockouts (C57BL6). In all other experiments in the manuscript, the strain of mice used is SCID-Beige. SCID-Beige mice lack mature B and T cells as well as Natural Killer (NK) cells. This fundamental difference in immune competence likely accounts for the contrast in the baseline measurement as they respond to injury differently. In most of the experiments in this manuscript the Inventors chose the SCID-Beige mouse since they are permissive to human cells (which are the source of the CDC and the exosomes). However an appropriate control for the 146a KO mouse was a wild type from the same background strain which the BL6 background. This has been previously documented. Strain has previously been shown to be a significant determinant of wound healing after myocardial infarction.

### Example 18

### MiR-146a effect on immune infiltration

Attenuating the inflammatory immune response is not necessarily abrogating it altogether. Innate immune cells including macrophages have been shown to play pro regenerative roles. Furthermore unpublished data from the Inventors' lab show that macrophage trafficking is not affected by CDC treatment, but macrophages treated with CDCs do switch from an M1 (proinflammatory) to an anti-inflammatory and pro-healing phenotype M2.

### Example 19

### Discussion

Cardiosphere-derived cells have been shown to induce therapeutic regeneration of the infarcted human heart. In a form of injury traditionally thought to be irreversible, CDCs led to shrinkage of scar and growth of new, functional myocardium. Similar effects have been corroborated in animal models. Here, the Inventors show that exosomes reproduce CDC-induced therapeutic regeneration, and that inhibition of exosome production undermines the benefits of CDCs. Exosomes contain microRNAs, which have the ability to alter cell behavior through paracrine mechanisms (Figure 6B). Among these, the Inventors have identified miR-146a as being particularly enriched in CDC exosomes. When administered alone, miR-146a reproduces some, but not all, of the salient benefits of CDCs and of CDC exosomes (Figure 3). Likewise, miR-146a-depleted exosomes were still able to suppress cardiomyocyte apoptosis (Figure 5A), albeit more weakly than when miR-146a is present. Treating hearts with miR-146a in a chronic model of MI (after the scar is permanent) does reproduce the increase in viable mass that is the signature of therapeutic regeneration, but fails to mimic two key beneficial effects of CDC exosomes: decreased scar mass and improved global function. The increase in viable myocardium does not suffice to increase function, possibly because of inadequate angiogenesis elicited by miR-146a. The Inventors conclude that miR-146a plays an important part in mediating the effects of CDC exosomes, but alone does not suffice to confer comprehensive therapeutic benefit. Other microRNAs in the repertoire may exert synonymous or perhaps synergistic effects with miR-146a. For instance, miR-22 (another microRNA highly enriched in CDC exosomes) has been shown to be critical for adaptive responses to cardiac stress. Likewise, miR-24 (also identified in CDC exosomes) modulates cardiac fibrosis by targeting furin, a member of the profibrotic TGF-b signaling pathway; overexpression of miR-24 in a model of MI decreased myocardial scar formation. The possible roles of these microRNAs as mediators of CDC exosome benefits, alone or in combination with miR-146a, remain to be studied. Whereas dissection of the active principles within CDC exosomes is worthwhile, deconstruction of the nanovesicles may be counterproductive from a therapeutic perspective. CDC exosomes are naturally cell permeant, and their lipid bilayer coat protects their payloads from degradation as particles shuttle from cell to cell, so that the intact particles themselves may be well suited for disease applications.

Injection of CDC-derived exosomes into the injured heart mimics the structural and functional benefits of CDC transplantation; conversely, inhibition of exosome secretion by CDC s abrogates the therapeutic benefits of transplanted CDCs. Not all exosomes are salutary: Injection of exosomes from dermal fibroblasts, control cells which are therapeutically inert, had no benefit. DC -exosomes decreased acute cardiomyocyte death and inflammatory cytokine release, while attenuating left ventricular (LV) remodeling and fibrosis in the injured heart. MicroRNA arrays reveal several "signature microRNAs" that are highly up-regulated in CDC-exosomes. In contrast, mass spectrometry indicates that the protein composition of CDC-exosomes is conventional and comparable to that of fibroblast exosomes.

This work implicates exosomes, and the microRNAs they contain, as crucial mediators of CDC-induced cardiac regeneration. CDCs exert diverse but coordinated effects: they recruit endogenous progenitor cells and coax surviving heart cells to proliferate; on the other hand, injected CDCs suppress maladaptive LV remodeling, apoptosis, inflammation, and tissue fibrosis after MI. While it is possible that CDCs secrete a medley of individual growth factors and cytokines that collectively produce diverse benefits, the involvement of master-regulator microRNAs within exosomes would help tie together the various effects without postulating complex mixtures of numerous secreted protein factors. Moreover, microRNAs are known to confer long-lasting benefits and fundamental alterations of the injured microenvironment helping to rationalize the sustained benefits of CDCs despite their evanescent survival in the tissue. CDC exosomes contain rich signaling information conferred by a cell type that is the first shown to be capable of producing regeneration in a setting of "permanent" injury, and confer the same benefits as CDCs without transplantation of living cells. For all these reasons, CDC exosomes merit further development as cell-free therapeutic candidates.

Based on the results described herein, CDC-exosomes are demonstrated as capable of treating heart-related conditions, such as treat heart failure (HF) associated with Duchenne muscular dystrophy (DMD). Exosomes secreted by cells are capable of reproducing therapeutic benefits of their parental cells and based on the described knockdown studies, appear to be indispensable in effectuating such therapeutic benefits. Importantly, these results have further identified that within their rich biological cargo of various proteins and RNA, microRNAs play a central role in activating regenerative processes, suggesting compelling applications in clinical therapeutics. Exosomes have significant advantages over traditional cell-based therapies including manufacturing advantages, relative ease of definition and characterization, lack of tumorgenicity and immunogenicity, and possibility of administration in therapeutic scenarios for which cell, tissue, organ or mechanical transplant is not available. Thus, CDC-exosomes represent a significant advance in biologic therapy.

### Example 20

### Statistical analysis

All results are presented as mean±SEM; results for alternans are presented as mean ±SD. Normality and equality of variances of data sets were first tested using Kolmogorov-Smirnov and Levene's tests, respectively. If both were confirmed, t-test or analysis of variance followed by Bonferroni 's post hoc test were used for determination of statistical significance; if either normality or equality of variances was not assured, nonparametric tests (Wilcoxon test or Kruskal-Wallis test followed by Dunn's post-test) were applied (SPSS II, SPSS Inc., Chicago, Illinois). No preliminary data were available for a power analysis. Experiments were planned with a sample size of 4 animals per group as an initial pilot project. Results from the pilot project allowed us to power subsequent studies. The study followed preclinical reporting standards, as described.

### Example 21

### Echocardiography

Echocardiographic studies were performed two days before (Baseline) and 3 weeks, 2 and 3 months after first CDC/CDC exosome (CDC-XO) injection and 3 weeks, 2 and 3 months after second CDC/CDC-XO injection using the Vevo 770 Imaging System (VisualSonics, Toronto, Canada). The same imaging system was used to perform echocardiographic studies at baseline (2 day before) and 3 weeks after miR148a mimic injection. After induction of light general anesthesia, the heart was imaged at the level of the greatest LV diameter. LV ejection fraction (LVEF) was measured with VisualSonics version 1.3.8 software from 2-dimensional long-axis views. Changes in left ventricular (LV) end diastolic and systolic volumes: First and second CDC or CDC-XO transplantation resulted in a sustained improvement of LV end-diastolic (LV EDV) and end-systolic (LV ESV) volumes in *mdx* mice, relative to placebo, for at least 6 months. Delivery of miR-148a partially improved LV EDV and LV ESV (Fig. 27).

### Example 22

### Treadmill Exercise Testing

Exercise capacity was assessed weekly with Exer-3/6 open treadmill (Columbus Instruments, Columbus, OH), beginning 3 weeks after CDC/vehicle injection (exercise capacity measured in a subset of *mdx* mice 1 week pre-operatively was equivalent to that measured 3 weeks post-operatively in the Mdx+Vehicle group; data not shown). After an acclimation period (10 m/min for 20 min) stepwise increases in average speed (1 m/min) were applied every two minutes during treadmill exercise until the mouse became exhausted (spending >10 seconds on shocker; continuous nudging was used during treadmill to help mice stay on the track). Subsequently, the mouse was returned to the cage and the total distance recorded. After 3 months of weekly exercise, CDC/vehicle *mdx* mice along with wild-type age-matched mice were followed for assessment of mortality. The treadmill protocol conformed to guidelines from the American Physiological Society³.

### Example 23

### Expansion of CDCs

Mouse CDCs were expanded from wild-type strain-matched mouse hearts (C57BL/10ScSnJ wild type mouse heart) as described². Briefly, ventricular tissues were minced into ~1 mm explants, partially digested enzymatically and plated on adherent (fibronectin-coated) culture dishes. These explants spontaneously yield outgrowth cells (explant-derived cells) which were harvested once confluent and plated in suspension culture (10⁵ cells/mL on poly-D-lysine-coated dishes) to enable self-assembly of three-dimensional cardiospheres. Subsequent replating of cardiospheres on adherent culture dishes yielded CDCs which were used in all experiments at passage one.

### Example 24

### Assessment of CDC Engraftment by Real-Time Polymerase Chain Reaction

Quantitative polymerase chain reaction (PCR) was performed 1, 2 and 3 weeks after CDC injection to assess cell engraftment. Male CDCs were injected to enable detection of the SRY gene located on the Y chromosome as a marker of engraftment using the TaqMan assay (Applied Biosystems, Foster City, CA). The whole mouse heart was harvested, weighed, and homogenized. A standard curve was generated with multiple dilutions of genomic DNA isolated from the injected CDCs. All samples were spiked with equal amounts of genomic DNA from non-injected mouse hearts as a control. For each reaction, 50 ng of genomic DNA was used. Real-time PCR was performed in triplicate. Engraftment was quantified from the standard curve. Percentage engraftment of CDCs at 1 week was ~8% and <1% at 2 weeks. By 3 weeks, no surviving CDCs could be detected (Fig. 28).

### Example 25

### Cardiomyocyte Proliferation and Cardiac Collagen Content after CDC Injection

Paraffin-embedded sections from apical, middle and basal parts of each heart were used for Masson's trichrome staining and immunostaining with antibodies against Ki67 and aurora B. Myocardial abundance of collagen I A1 and collagen III A1 was measured by Western blot analysis (Fig. 29).

### Example 26

### Exosomes

Exosomes were isolated from serum-free media conditioned overnight (24 hr) by cultured human CDCs (CDC-XO) [or normal human dermal fibroblasts (NHDF) as a control] in hypoxia (2% O₂; default condition) or normoxia (20% O₂, solely for studies comparing RNA content of exosomes). Ultracentrifugation (100,000g for 1 hr) was used to isolate exosomes from conditioned media after sequential centrifugations at 300g (10min) and 10,000g (30min) and filtration with 0.22 micron filters. Isolated exosomes underwent RNA extraction and subsequently RNA sequencing (Fig. 30) or were re-suspended in PBS (for *in vivo* and *in vitro* experiments) and the ratio of exosome to protein was measured using Micro BCA Protein Assay Kit (Life technologies, Grand Island, NY) and Nanosight particle counter (Fig. 31), respectively. Preliminary dose-response studies identified 2x10⁷ and 1x10⁹exosomes/µg protein from hypoxic CDCs as effective doses for *in vitro* and *in vivo* experiments, respectively. Similar concentrations of exosomes were used for the experiments in which NHDF exosomes were applied. Preliminary pilot *in vivo* experiments were performed using exosomes isolated by ultracentrifugation or Exoquick kit (SBI, Mountain View, CA) as described, yielding similar results with the two isolation methods.

### Example 27

### CDC, CDC-exosome and miR-148 Injections

To optimize the process of CDC transplantation, preliminary dose-response experiments were performed, which identified 1x10⁵ cells in first injection and 1x10⁴ cells in second injection (3 months after first injection) as effective doses, consistent with prior dose-ranging experiments in ischemic and nonischemic mouse models. A total of 1x10⁵ cells/40µL phosphate-buffered saline (PBS; first injection) or 1x10⁴ cells/40µL PBS (second injection) or PBS alone were injected into left ventricular (LV) myocardium divided equally among 4 points as described. The LV was visually divided into three zones: basal, middle, and apical, with one injection in the basal, two in the middle and one in the apical zone. 10-month-old CDC/*mdx* and vehicle/mdx mice were injected with CDCs (Mdx+CDC, n=12) or vehicle [placebo: Mdx+Vehicle (PBS), n=12] twice (3 months interval), respectively. Injections were during open-chest thoracotomy via a 28 ½ gauge-needle. All surgical procedures were carried out while the animals were under general anesthesia (Dexmedetomidine (0.5mg/kg)/Ketamine (75mg/kg); IP; once before surgery). Similar protocols were used for injection of CDC-exosomes and miR-148 into myocardium. A miR-148a mimic (hsa-miR-148a-3p, 2µg; Sigma-Aldrich, St. Louis, MO) was mixed with RNAiMAX transfection reagent (life technologies, Grand Island, NY) for 30 min at room temperature at a total volume of 40 µl and injected into 4 points per heart as described above.

### Example 28

### Histology

Mice were sacrificed 3 weeks (CTL: n=4; Mdx+Vehicle: n=6; Mdx+CDC/Mdx+CDC-XO: n=6 each) or 3 months (CTL: n=4; Mdx+Vehicle: n=6; Mdx+CDC/Mdx+CDC-XO: n=6) after first CDC/CDC-XO injections and 3 weeks after miR-148 injection (n=6). Paraffin-embedded sections from apical, middle and basal parts of each heart were used for histology. Masson's trichrome staining (HT15 Trichrome Stain [Masson] Kit; Sigma-Aldrich, St. Louis, MO) was performed for evaluation of fibrosis. T cells, B cells and macrophages were assessed by immunostaining with antibodies against mouse CD3, CD20 and CD68, respectively, and the average number of cells in each heart was calculated from counting cells in 10 fields (20x magnification) from each of 10 sections selected randomly from the apical (3 sections; 50µm interval), middle (4sections; 50µm interval) and basal (3 sections; 50µm interval) regions of each heart. Actively-cycling and proliferating (Ki67⁺ & Aurora B⁺) cardiomyocytes were counted in the same manner, and the cycling and proliferating fractions were expressed as the number of Ki67⁺ and Aurora B⁺ cardiomyocytes divided by the total number of cardiomyocytes per high-power field (HPF), respectively, as described. Measurements were averaged for each heart. Immunofluorescence staining: Heat-induced epitope retrieval in low pH buffer (DAKO, Carpinteria, CA) was followed by 2 hours permeabilization/blocking with Protein Block Solution (DAKO, Carpinteria, CA) contained 1% saponin (Sigma, St. Louis, MO; Protein Block Solution contained 3% saponin was applied for immunofluorescence staining of Ki67). Subsequently, primary antibodies in Protein Block Solution were applied overnight in 4 C° for immunofluorescence staining of 5-µm sections from apical, middle and basal parts of each heart. After 3x wash with PBS, each 10 minutes, Alexa Fluor secondary antibodies (Life Technologies, Grand Island, NY) were used for detection. Images were taken by a Leica TCS SP5 X confocal microscopy system. Immunofluorescence staining was conducted using antibodies against mouse Ki-67 (SP6; 1:50; Thermo Fisher Scientific, Fremont, CA), WGA (Wheat germ agglutinin; 1:200; Life Technologies, Grand Island, NY), Nrf2 (C20; 1:50; Santa Cruz Biotechnology, Santa Cruz, CA), aurora B (1:250; BD Biosciences, San Jose, CA). Immunoperoxidase staining: Immunohistochemical detection of CD3, CD20 and CD68 was performed on 5-µm sections using prediluted rabbit monoclonal antibodies from Ventana Medical System (Tuscon, AZ; CD68) and Cell Marque (Rocklin, CA; CD3, CD20). Staining was conducted on the Leica Bond-Max Ventana automated slide stainer (Chicago, IL) using onboard heat-induced epitope retrieval method in high pH ER2 buffer (Leica Biosystems, Buffalo Grove, IL). The staining was visualized using the Dako Envision⁺ rabbit detection System and Dako DAB (Carpinteria, CA). The slides were subsequently counterstained with mayer's hematoxylin for 1 minute and coverslipped. Electron microscopy: Apical (1 cube), middle (3 cubes from right, middle and left subparts) and basal (3 cubes from right, middle and left subparts) parts of posterior wall from each heart (CTL: n=3; Mdx+Vehicle: n=3; Mdx+CDC: n=3) were fixed by immersion of 1mm² cubes in 2% glutaraldehyde, postfixed in osmium, and embedded in epon. Sections were cut at silver thickness, stained with uranyl acetate and lead citrate, and viewed with JEOL 1010 equipped with AMT digital camera system.

### Example 29

### Western Blots

Western blot analysis was performed to compare myocardial abundance of target proteins contributing to Nrf2 signaling [Nrf2, phospho-Nrf2 (Nrf2-p^{s40}) and Nrf2 downstream gene products: heme oxygenase-1 (HO-1), catalase, superoxide dismutase-2 (SOD-2), and catalytic subunit of glutamate-cysteine ligase (GCLC)], Nrf2 phosphorylation [phospho-Akt(Akt-p³⁰⁸)], oxidative phosphorylation [CI (NDUFB8 subunit), CII (SDHB subunit), CIV (MTCO1 subunit), CIII (UQCRC2 subunit) and CV (ATPSA subunit)], mitochondrial biogenesis (PGC-1), mitophagy (PINK1), inflammation (NF-κB and MCP-1) and fibrosis (Collagen IA1 and collagen IIIA1). Myocardial density of malondialdehyde protein adducts, a marker of oxidative stress, was also measured by Western blotting (WB). Samples from apical, middle and basal parts of each heart (each 1 mm-thick transverse section) were mixed and homogenized, and nuclear and cytoplasmic fractions were extracted per manufacturer's instructions (CelLytic NuCLEAR Extraction Kit, Sigma-Aldrich, St. Louis, MO). Mitochondria were extracted from fresh whole hearts (CTL: n=3; Mdx+Vehicle: n=8; Mdx+CDC: n=8) as described in respirometry section. Cytoplasmic, nuclear and mitochondrial extracts for WB analysis were stored at -80C°. The protein concentrations in extracts were determined by the Micro BCA Protein Assay Kit (Life technologies, Grand Island, NY). Target proteins in the cytoplasmic, nuclear and mitochondrial fractions were measured by Western blot analysis using the following antibodies: antibodies against mouse Nrf2, HO-1, catalase, SOD-2, GCLC, collagen IA1, and collagen IIIA1, and PGC-1 were purchased from Santa Cruz Biotechnology (Santa Cruz, CA), phospho-Nrf2 (Nrf2-p^{s40}; Biorbyt, San Francisco, CA), respiratory chain subunits (Total OXPHOS Rodent WB Antibody Cocktail antibody), malondialdehyde, citrate synthase and TBP (Abcam, Cambridge, MA), Akt and Akt-p^{T308} , IκB-α, p-IκB-α, (Cell Signaling Technology, Denver, CO), PINK1, MCP-1 and NF-κB p65 (Sigma-Aldrich, St. Louis, MO) antibodies were purchased from the cited sources. Antibodies to TBP (TATA binding protein) and citrate synthase were used for measurements of the housekeeping proteins for nuclear (TBP), cytosolic and mitochondrial (citrate synthase) target proteins. Western blot methods: Briefly, aliquots containing 20 µg proteins were fractionated on 8, 10 and 4-12% Bis-Tris gel (Life technologies, Grand Island, NY) at 120 V for 2 h and transferred to a PVDF membrane (Life technologies, Grand Island, NY). The membrane was incubated for 1 h in blocking buffer (1× TBS, 0.05% Tween-20 and 5% nonfat milk) and then overnight in the same buffer containing the given antibodies at optimal dilutions listed in Table 1.

**Table 1. Antibodies and the optimal dilutions applied in Western blot analyses.**

| **Antibody** | **p-Akt** | **Akt** | **Malondialdehyde** | **NF-κB p65** | **Nrf2** | **HO-1** | **p-Nrf2** |
|---|---|---|---|---|---|---|---|
| **Dilution** | 1:500 | 1:1000 | 1:1000 | 1:1000 | 1:500 | 1:500 | 1:250 |

| **Antibody** | **TBP** | **Collagen I** | **Collagen III** | **Citrate syn.** | **p-IκB-α** | **IκB-α** | **PGC-1** |
|---|---|---|---|---|---|---|---|
| **Dilution** | 1:2000 | 1:500 | 1:500 | 1:2000 | 1:500 | 1:1000 | 1:500 |

| **Antibody** | **Catalase** | **SOD-2** | **GCLC** | **MCP** | **OXPHOS** | **PINK1** | |
|---|---|---|---|---|---|---|---|
| **Dilution** | 1:500 | 1:500 | 1:500 | 1:100 | 1:500 | | 2µg/ml |

The membrane was washed 3 times for 5 min in 1× TBS, 0.05% Tween-20 before a 2-h incubation in a buffer (1× TBS, 0.05% Tween-20 and 3% nonfat milk) containing horseradish peroxidase-linked anti-rabbit IgG, anti-mouse IgG (Cell Signaling Technology, Denver, CO) and anti-goat IgG (Sigma-Aldrich, St. Louis, MO) at 1:1000-3000 dilution. The membrane was washed 3 times for 5 min in 1 × TBS, 0.05% Tween-20 and developed by autoluminography using the ECL chemiluminescent agents (Super Signal West Pico Chemiluminescent Substrate; Life Technologies, Grand Island, NY). Citrate synthase and TBP were used as housekeeping proteins against which expressions of the proteins of interest were normalized. Phosphorylated Akt, Nrf2 and IκB-α were normalized to total Akt, Nrf2 and IκB-α. Western blot analyses of collagen I and collagen III were conducted under nonreducing, non-denaturing condition.

### Example 30

### Mitochondrial DNA

Extracted DNAs (QIAamp DNA Mini Kit, QIAGEN, Germantown, MD) from whole heart tissue were used to measure mitochondrial to nuclear DNA ratio using PCR format per manufacturer's instructions (NovaQUANT^{™} Mouse Mitochondrial to Nuclear Ratio kit, EMD Millipore, Billerica, MA).

### Example 31

### Respirometry

Mice were sacrificed via cervical dislocation after isofluorane anesthesia. Hearts were immediately excised, rinsed in PBS and homogenized via polytron in 1mL ice cold HES buffer (250mM sucrose, 1mM EDTA, 10mM HEPES, pH 7.4). Lysates were spun down at 1000g for 5min at 4°C to remove unbroken cells and large debris. Supernatant was then spun down at 7000g for 10min at 4°C to separate mitochondria-enriched fraction from crude cytosol. Pellet was resuspended in 1mL HES buffer (A subportion in lysis buffer for WB). Protein quantification was performed and adjustment with HES buffer to obtain sample containing 10µg protein in 50µL buffer which was loaded into a 24-well Seahorse cell culture plate, which was spun down at 2000g for 20min at 4°C to allow mitochondria adherence to the plate surface. 450µL MAS buffer (70mM sucrose, 220mM mannitol, 5mM KH2PO4, 5mM MgCl2, 1mM EGTA, 0.2% fatty acid-free BSA, pH 7.4) was then added prior to Seahorse XF24 mitochondria stress test. 5mM/5mM pyruvate/malate and 0.25mM ADP was used to stimulate mitochondrial oxidative phosphorylation followed by 1µM oligomycin, 1µM FCCP, and a mixture of 1µM antimycin, 500nM rotenone. Citrate synthase activity was measured in sample lysates to normalize for actual amount of mitochondria loaded for test. Seahorse respirometry on normal and human Duchenne iPs cell derived cardiomyocytes was performed using Seahorse^{™} XF96 Extracellular Flux analyzer as described.

### Example 32

### Intracellular Ca2+ Recordings

iPS- derived cardiomyocytes were loaded for 30 min with 5 µM of the fluorogenic calcium-sensitive dye, Cal-520 (AAT Bioquest, Sunnyvale, CA) and paced via field stimulation at a frequency of 1 Hz using an Ion-Optix Myopacer (IonOptix Corp) delivering 0.2 ms square voltage pulses with an amplitude of 20 V via two platinum wires placed on each side of the chamber base (~1 cm separation). The Inventors used the xyt mode (2D) of a Leica TCS-SP5-II (Leica Microsystems Inc.; Wetzlar, Germany) to image intracellular Ca²⁺. Cal 520 was excited with a 488 nm laser and its emission (>505 nm) was collected with a 10X objective (Leica: N PLAN 10x/0.25) at scan speeds ranging from 36 to 7 ms per frame depending on the field size. The fluorescence intensity (F) proportional to Ca²⁺ concentration was normalized to baseline fluorescence, F0 (F/F0). Time to peak and Ca²⁺ transient amplitude (F/F0) were analyzed with the software Clampfit (ver. 10.2, Molecular Devices, Inc.). Beat-to-beat alternans in each group calculated over the 5-10 sec interval of pacing at 1 Hz. The amplitude of each transient from each cell (n=10 cells in each group) was measured during pacing and mean and standard deviation were calculated and compared among groups.

### Example 33

### Function, Survival and Antioxidant Pathways Improved by CDC Transplantation in mdx Mice

Intramyocardial injection of first and second (lower) doses of CDCs in *mdx* mice produced a sustained improvement of left ventricular function (as manifested by ejection fraction [EF]) and volumes, relative to placebo, for at least 6 months (Fig. 23A and 27). The CDC-induced improvement in EF persisted beyond the point at which no surviving CDCs were detectable in *mdx* hearts (3 weeks after CDC delivery; Fig. 28). In addition to improving EF, CDC injection enhanced ambulatory function (Fig. 23B). Age-matched wild-type mice (CTL) and 10-month-old *mdx* mice (distinct from the *mdx* mice studied for evaluation of cardiac function) were subjected to weekly high-intensity treadmill exercise, starting 3 weeks after CDC or vehicle administration. CDC-treated *mdx* mice showed a substantial increase in maximal exercise capacity, relative to vehicle-treated *mdx* mice, over the 3 mos that it was measured; survival also differed in the two groups (Fig. 23C). By ~23 mos of age all vehicle-treated *mdx* mice had died, whereas >50% of CDC-treated *mdx* mice remained alive (Fig. 23C). Injection of CDCs led to activation of the Nrf2 anti-oxidant pathway and upregulation of downstream gene products (Fig. 23E). Concomitantly, oxidative stress was attenuated (Fig. 23F). Nrf2 is normally sequestered in the cytoplasm via binding to its repressor molecule, Keap1. Oxidative stress (as well as Nrf2 phosphorylation by protein kinases such as Akt) causes dissociation of the Nrf2-Keap1 complex, which culminates in nuclear translocation of Nrf2 and transcriptional activation of antioxidant enzymes. In *mdx* hearts, levels of phosphorylated Akt and cytoplasmic and nuclear Nrf2 were high (as expected in response to oxidative stress); CDC treatment further increased their protein levels (Fig. 23E). As a consequence, downstream effectors heme oxygenase-1 (HO-1), catalase, superoxide dismutase-2 (SOD-2), and the catalytic subunit of glutamate-cysteine ligase (GCLC) were upregulated (Fig. 23E), leading to a profound reduction of malondialdehyde adducts (fatty acid peroxidation end products; Fig. 23F) in CDC-treated *mdx* heart. Histological analysis revealed extensive fibrosis in a typical vehicle-treated *mdx* heart, but much less in a CDC-treated *mdx* heart (comparable to an age-matched wild-type [WT] control). Likewise, Western blot analysis showed that CDC treatment largely reversed the accumulation of collagens I and III in *mdx* heart tissue 3 weeks after treatment (Fig. 29).

### Example 34

### Mitochondrial Dysfunction and Inflammation Attenuated by CDC Transplantation in mdx Mouse Hearts

Mitochondrial structure and function are abnormal in muscular dystrophy-associated heart failure. In *mdx* hearts, mitochondrial integrity improved 3 weeks after CDC injection: CDCs restored mitochondrial ultrastructure (Fig. 24A), increased mitochondrial DNA copy numbers (but not mitochondrial number; Fig. 24B & C), augmented levels of respiratory chain subunits (Fig. 24D) and normalized the deficient respiratory capacity of isolated *mdx* mitochondria (Fig. 24E).

Key regulators of mitochondrial biogenesis and mitophagy, PGC-1 (nuclear PPARγ co-activator1) and PINK1, respectively, were upregulated 3 days and downregulated 3 weeks after CDC treatment (Fig. 24F), consistent with an initial turnover of damaged mitochondria followed by repopulation with stable competent mitochondria. Of note, the improved mitochondrial integrity and decreased mitochondrial turnover observed 3 weeks after CDC treatment in *mdx* mouse hearts were associated with upregulation of antioxidant enzymes and reductions of oxidative stress and inflammation (Fig. 24G & H). NFκB, the master regulator of pro-inflammatory cytokines and chemokines, was activated in vehicle *mdx* hearts: Increases in phosphorylated IκB and nuclear p65 contents were accompanied by marked upregulation of MCP1 (monocyte chemoattractant protein1) and accumulation of CD68⁺ macrophages and CD3⁺ T cells. CDC treatment reversed activation of NFκB and decreased the number of inflammatory cells in *mdx* hearts 3 weeks after CDC injection (Fig. 24G & H). The Inventors also probed the effects of CDCs on cardiomyogenesis. Vehicle-treated *mdx* hearts exhibited a several-fold increase in the numbers of cycling (Ki67⁺) and proliferating (aurora B⁺) cardiomyocytes (Fig. 29), presumably as a compensation for ongoing cardiomyocyte loss. CDCs are known to increase endogenous cardiomyogenesis in ischemic and non-ischemic models. Likewise, CDC treatment promoted cardiomyogenesis in the *mdx* heart, as evidenced by a marked increase in Ki67⁺ and aurora B⁺ cardiomyocytes (Fig. 29).

### Example 35

### CDC-secreted Exosomes Reproduce Benefits of CDCs in mdx Mice

Exosomes secreted by CDCs (CDC-exosomes) mimic the functional and structural benefits of CDCs in a murine model of myocardial infarction. In the *mdx* mouse model of DMD, likewise, functional, anti-fibrotic, and cardiomyogenic benefits of CDCs are reproduced by administration of exosomes isolated from media conditioned by hypoxic CDCs. Intramyocardial injection of two repeat doses of human CDC-exosomes (separated by 3 months) led to sustained improvement in EF in *mdx* mice, relative to vehicle-treated mice (Fig. 25A & Fig. 27). Meanwhile, the amounts of collagen I and III decreased in CDC-exosome-injected *mdx* hearts (Fig. 25B), along with marked increases in the numbers of cycling (Ki67⁺, Fig. 25C1) and proliferating (aurora B⁺, Fig. 25C2) cardiomyocytes.

### Example 36

### CDC-exosomes in Human Duchenne Cardiomyocytes and miR-148a in mdx Mice

Duchenne human iPS-derived cardiomyocytes (DMD CMs) exhibit a number of phenotypic deficits also seen in *mdx* mouse hearts. Decreased oxygen consumption rate (OCR), reminiscent of that observed in *mdx* heart mitochondria (Fig. 24E), and abnormal calcium cycling are among the reported deficits²¹. Priming DMD CMs with CDC-exosomes one week earlier normalized OCR, but priming with exosomes from normal human dermal fibroblasts (NHDFexosomes) had no effect. Beat-to-beat calcium transient alternans during 1Hz burst pacing, a measure of arrhythmogenicity, was likewise suppressed by priming DMD CMs with CDCexosomes (Fig. 26A & B). Comparison of microRNA (miR) content of CDC-exosomes isolated from hypoxic versus normoxic CDCs revealed differences in miR expression (Fig. 26C), with notable augmentation of miR-148a in hypoxia. Given that the Inventors' CDC-exosomes were grown under hypoxia, the Inventors tested the effects of miR-148a administration. Three weeks after intramyocardial injection of miR-148a mimic, EF fraction was partially restored, and NFκB was suppressed, by miR-148a, but phospho-Akt level decreased (Fig. 26D & E). The phospho-Akt changes are directionally opposite to those seen with CDC injection (Fig. 23E), indicating that miR-148a mimics some, but not all, of the effects of CDCs and CDC-exosomes.

**Table 2. 95% Confidence interval of difference**

| ***pAkt(CDC)** | ***pNrf2** | ***Nuclear Nrf2** | ***HO-1** |
|---|---|---|---|
| 1 (0.20 , 0.43) | 1 (0.46 , 0.62) | 1 (0.14 , 0.20) | 1 (0.04 , 0.23) |
| 2 (0.11 , 0.31) | 2 (0.42 , 0.56) | 2 (0.06 , 0.13) | 2 (0.02 , 0.23) |

| **MDA** | **p-IκB** | **p65** | **MCP-1** |
|---|---|---|---|
| 1(1.37, 1.50) | 1 (0.43 , 0.52) | 1 (0.32 , 0.36) | 1 (0.28 , 0.41) |
| 3 (1.08 , 1.21) | 3 (0.28 , 0.37) | 3 (0.28 , 0.32) | 3 (0.27 , 0.36) |

| **CIII** | **CV** | **Collagen I(CDC)** | **Collagen III(CDC)** |
|---|---|---|---|
| 1 (-1.27 , -1.07) | 1 (-0.82 , -0.63) | 1 (0.10, 0.19) | 1 (0.05 , 0.23) |
| 3 (-0.42 , -0.29) | 3 (-0.57 , -0.39) | 3 (0.07 , 0.14) | 3 (0.05 ,0.11) |

| **PINK1(Wk3)** | ***PGC-1(D3)** | **PGC-1(Wk3)** | **Collagen I(XO)** |
|---|---|---|---|
| 1 (0.22 , 0.26) | 1 (0.36 , 0.46) | 1 (0.13 , 0.26) | 1 (0.38 , 0.71) |
| 3 (0.05 , 0.16) | 2 (0.13 , 0.22) | 3 (0.11 , 0.17) | 3 (0.36, 0.69) |

| **CD3** | *** AuroraB(CDC)** | ***Ki67(CDC)** | ***AuroraB(XO)** |
|---|---|---|---|
| 1 (0.79 , 1.73) | 1 (0.001 ,0.01) | 1 (0.001 , 0.01) | 1 (0.001, 0.004) |
| 3 (0.04 , 1.08) | 2 (0.0001, 0.004) | 2 (0.0001 , 0.01) | 2 (0.0002, 0.004) |

| *** Catalase** | ***SOD-2** | ***GCLC** | |
|---|---|---|---|
| 1 (0.04 , 0.21) | 1 (0.16 , 0.25) | 1 (0.09 , 0.13) | |
| 2 (0.07 , 0.11) | 2 (0.10 , 0.14) | 2 (0.08 , 0.15) | |

| **CI** | **CII** | **CIV** | |
|---|---|---|---|
| 1 (-1.69 , -1.56) | 1 (-1.85 , -1.72) | 1 (-1.14 ,-0.97) | |
| 3 (-0.79 , -0.70) | 3 (-0.70, - 0.54) | 3 (-0.51 , -0.41) | |

| **P65(miR-148)** | **pAkt(miR-148)** | ***PINK1(D3)** | |
|---|---|---|---|
| 1 (0.37 , 0.46) | 1 (0.096 , 0.15) | 1 (0.25 , 0.34) | |
| 3 (0.08 , 0.53) | 3 (0.054 , 0.15) | 2 (0.05 , 0.19) | |

| **Collagen III(XO)** | **Alternans** | **CD68** | |
|---|---|---|---|
| 1 (0.36 , 0.59) | 1 (-0.024 , 0.83) | 1 (0.18 , 0.67) | |
| 3 (0.25, 0.35) | 3 (-0.59 , 0.37) | 3 (0.01 , 0.56) | |

| ***Ki67(XO)** | | | |
|---|---|---|---|
| 1 (0.001, 0.01) | | | |
| 2 (0.0001, 0.01) | | | |

| | | | |
|---|---|---|---|
| 1: Wild type 2: Mdx+Vehicle 3: Mdx+CDC or Mdx+CDC-exosome or Mdx+miR148 * 95% confidence interval (CI) for the difference between 3 vs 1; 3 vs 2. The rest denotes 95% CI for the difference between 2 vs 1, 2 vs 3. | | | |

### Example 37

### Discussion

Although heart disease may not be apparent in DMD patients for a decade or more after the diagnosis of skeletal myopathy, cardiomyopathy progresses rapidly once it becomes evident. Serial cardiac magnetic resonance imaging studies have revealed that fibrosis, while often initially restricted to just one segment of the heart, spreads quickly and inexorably thereafter²³. The result is impairment of global heart function and early death. There is no effective treatment to reverse, prevent, or slow the progression of DMD cardiomyopathy. Recognizing that CDCs exert regenerative effects that may be salutary in DMD, the Inventors tested the effects of CDC injection early in the course of DMD cardiomyopathy. The Inventors discovered that CDCs attenuate fibrosis and inflammation in the *mdx* heart, while improving pump function, increasing exercise capacity and enhancing survival. The salient benefits of CDCs were reproduced by CDC-exosomes. The Inventors' findings support the hypothesis that CDCs act by secreting exosomes laden with genetic signals, including (but not limited to) miR-148a. These exosomes are taken up by the surrounding myocardium, where they antagonize multiple pathophysiological pathways that underlie DMD cardiomyopathy. The constellation of effects is synergistic: oxidative stress, inflammation and fibrosis are blunted, while cardiomyogenesis and mitochondrial function are augmented. The results are notable in that CDCs and their exosomes not only forestall progression, but actually reverse the central functional deficits of DMD cardiomyopathy. Major improvements in mortality and exercise capacity occur without targeting dystrophin, providing proof of concept that the root genetic cause need not be corrected in order for DMD therapies to be highly effective. Given that CDCs are already in advanced clinical testing, the Inventors' results support the initiation of clinical trials of CDCs in patients with DMD cardiomyopathy. Indeed, based upon the present findings, the HOPE-Duchenne trial will soon investigate the safety and tolerability of allogeneic CDCs administered by multi-vessel intracoronary infusion in subjects with heart failure secondary to DMD.

The various methods and techniques described above provide a number of ways to carry out the invention. Of course, it is to be understood that not necessarily all objectives or advantages described may be achieved in accordance with any particular embodiment described herein. Thus, for example, those skilled in the art will recognize that the methods can be performed in a manner that achieves or optimizes one advantage or group of advantages as taught herein without necessarily achieving other objectives or advantages as may be taught or suggested herein. A variety of advantageous and disadvantageous alternatives are mentioned herein. It is to be understood that some preferred embodiments specifically include one, another, or several advantageous features, while others specifically exclude one, another, or several disadvantageous features, while still others specifically mitigate a present disadvantageous feature by inclusion of one, another, or several advantageous features.

Furthermore, the skilled artisan will recognize the applicability of various features from different embodiments. Similarly, the various elements, features and steps discussed above, as well as other known equivalents for each such element, feature or step, can be mixed and matched by one of ordinary skill in this art to perform methods in accordance with principles described herein. Among the various elements, features, and steps some will be specifically included and others specifically excluded in diverse embodiments.

Although the invention has been disclosed in the context of certain embodiments and examples, it will be understood by those skilled in the art that the embodiments of the invention extend beyond the specifically disclosed embodiments to other alternative embodiments and/or uses and modifications and equivalents thereof.

Many variations and alternative elements have been disclosed in embodiments of the present invention. Still further variations and alternate elements will be apparent to one of skill in the art. Among these variations, without limitation, are sources of cardiosphere derived cells, the use of alternative sources such as cells derived directly from heart biopsies (explant-derived cells), or from self-assembling clusters of heart-derived cells (cardiospheres), exosomes produced by such cells, method of isolating, characterizing or altering exosomes produced by such cells, and the particular use of the products created through the teachings of the invention. Various embodiments of the invention can specifically include or exclude any of these variations or elements.

In some embodiments, the numbers expressing quantities of ingredients, properties such as concentration, reaction conditions, and so forth, used to describe and claim certain embodiments of the invention are to be understood as being modified in some instances by the term "about." Accordingly, in some embodiments, the numerical parameters set forth in the written description and attached claims are approximations that can vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the invention are approximations, the numerical values set forth in the specific examples are reported as precisely as practicable. The numerical values presented in some embodiments of the invention may contain certain errors necessarily resulting from the standard deviation found in their respective testing measurements.

In some embodiments, the terms "a" and "an" and "the" and similar references used in the context of describing a particular embodiment of the invention (especially in the context of certain of the following claims) can be construed to cover both the singular and the plural. The recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g. "such as") provided with respect to certain embodiments herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member can be referred to and claimed individually or in any combination with other members of the group or other elements found herein. One or more members of a group can be included in, or deleted from, a group for reasons of convenience and/or patentability. When any such inclusion or deletion occurs, the specification is herein deemed to contain the group as modified thus fulfilling the written description of all Markush groups used in the appended claims.

Preferred embodiments of this invention are described herein, including the best mode known to the inventors for carrying out the invention. Variations on those preferred embodiments will become apparent to those of ordinary skill in the art upon reading the foregoing description. It is contemplated that skilled artisans can employ such variations as appropriate, and the invention can be practiced otherwise than specifically described herein. Accordingly, many embodiments of this invention include all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

Furthermore, numerous references have been made to patents and printed publications throughout this specification. Each of the above cited references and printed publications are herein individually incorporated by reference in their entirety.

In closing, it is to be understood that the embodiments of the invention disclosed herein are illustrative of the principles of the present invention. Other modifications that can be employed can be within the scope of the invention. Thus, by way of example, but not of limitation, alternative configurations of the present invention can be utilized in accordance with the teachings herein. Accordingly, embodiments of the present invention are not limited to that precisely as shown and described.

Yet further aspect and embodiment of the invention are enumerated with reference to the following numbered clauses.
1. A method of treatment, comprising:
   selecting a subject in need of treatment for heart failure secondary to a chronic degenerative muscular disease; and
   administering a composition comprising a plurality of exosomes to the subject, wherein the plurality of the exosomes are isolated from cardiosphere-derived cells (CDCs) grown in serum-free media, comprise exosomes with a diameter of about 90 nm to about 200 nm and are CD81+, CD63+, or both, and further wherein administration of the composition treats the subject.
2. The method of clause 1, wherein the chronic degenerative muscular disease is Duchenne muscular dystrophy.
3. The method of clause 1, wherein administering a composition comprises about 1 to about 100 mg exosome protein in a single dose.
4. The method of clause 3, wherein a single dose is administered multiple times to the subject.
5. The method of clause 1, wherein administering a composition comprises injection.
6. The method of clause 5, wherein the injection comprises percutaneous injection.
7. The method of clause 5, wherein the injection is directly into heart muscle.
8. The method of clause 1, wherein administering a composition comprises myocardial infusion.
9. The method of clause 8, wherein myocardial infusion is intra-arterial or intravenous.
10. The method of clause 1, wherein treatment of the subject results in decreased fibrosis, decreased inflammation, increased mitochondrial function and/or increased cardiomyogenesis.
11. The method of clause 10, wherein decreased fibrosis comprises a reduction in collagen accumulation.
12. The method of clause 11, wherein collagen comprises collagen I and/or collagen III.
13. The method of clause 10, wherein decreased inflammation comprises an increase in cytoplasmic nuclear factor (erythroid-derived 2)-like 2 (Nrf2), reduction in fatty acid peroxidation end products, reduced numbers of inflammatory cells, and/or upregulated expression of antioxidants.
14. The method of clause 13, wherein antioxidants comprise heme oxygenase-1 (HO-1), catalase, superoxide dismutase-2 (SOD-2), and glutamate-cystein ligase catalytic (GCLC) subunit.
15. The method of clause 13, wherein inflammatory cells comprise CD68+ macrophages and CD3+ T-cells.
16. The method of clause 10, wherein increased mitochondrial function comprises increased mitochondrial ultrastructure and/or increased mitochondrial biogenesis.
17. The method of clause 10, wherein increased mitochondrial function comprises increased nuclear PPAR-y co-activator-1 (PGC-1) expression.
18. The method of clause 1, wherein the exosomes comprise one or more microRNAs selected from the group consisting of: microRNAs miR-146a, miR148a, miR22, miR-24, miR-210, miR-150, miR-140-3p, miR-19a, miR-27b, miR-19b, miR-27a, miR-376c, miR-128, miR-320a, miR-143, miR-21, miR-130a, miR-9, miR-185, and miR-23a
19. A method of treatment, comprising:
   selecting a subject in need of treatment for heart failure secondary to a chronic muscular disease; and
   administering a composition comprising cardiosphere-derived cells (CDCs), wherein administration of the composition treats the subject.
20. The method of clause 19, wherein the chronic muscular disease is Duchenne muscular dystrophy.
21. The method of clause 19, wherein administering a composition comprises about 1 x 10⁵ to about 1 x 10⁸ or more CDCs in a single dose.
22. The method of clause 21, wherein administering a composition comprises myocardial infusion.
23. The method of clause 22, wherein myocardial infusion is intracoronary.
24. The method of clause 22, wherein myocardial infusion is intra-arterial or intravenous.
25. The method of clause 19, wherein treatment of the subject results in decreased fibrosis, decreased inflammation, increased mitochondrial function and/or increased cardiomyogenesis.
26. The method of clause 25, wherein decreased fibrosis comprises a reduction in collagen accumulation.
27. The method of clause 26, wherein collagen comprises collagen I and/or collagen III.
28. The method of clause 25, wherein decreased inflammation comprises an increase in cytoplasmic nuclear factor (erythroid-derived 2)-like 2 (Nrf2), reduction in fatty acid peroxidation end products, reduced numbers of inflammatory cells, and/or upregulated expression of antioxidants.
29. The method of clause 28, wherein antioxidants comprise heme oxygenase-1 (HO-1), catalase, superoxide dismutase-2 (SOD-2), and glutamate-cystein ligase catalytic (GCLC) subunit.
30. The method of clause 28, wherein inflammatory cells comprise CD68+ macrophages and CD3+ T-cells.
31. The method of clause 25, wherein increased mitochondrial function comprises increased mitochondrial ultrastructure and/or increased mitochondrial biogenesis.
32. The method of clause 25, wherein increased mitochondrial function comprises increased nuclear PPAR-y co-activator-1 (PGC-1) expression.

## Claims

1. A composition comprising a plurality of cardiosphere-derived cells (CDCs), wherein the plurality of CDCs are grown in serum-free media, for use in a method of decreasing fibrosis and inflammation secondary to a chronic muscular disease in a human.

2. The composition for use according to claim 1, wherein the chronic muscular disease is Duchenne muscular dystrophy.

3. The composition for use according to claim 1 or 2, wherein the composition is administered by a method comprising 1×10⁵ to about 1×10⁸ or more CDC's in a single dose.

4. The composition for use according to any of the preceding claims, wherein the composition is administered by a method comprising injection or myocardial infusion, wherein the injection is directly into heart muscle, and wherein myocardial infusion is intra-arterial or intravenous.

5. The composition for use according to any of the preceding claims, wherein treatment of the subject results in increased mitochondrial function and/or increased cardiomyogenesis, further wherein treatment of the subject results in a reduction in collagen accumulation, and wherein collagen comprises collagen I and/or collagen II.

6. The composition for use according to claim 5, wherein treatment of the subject results in an increase in cytoplasmic nuclear factor (erythroid-derived 2)-like 2 (Nrf2), reduction in fatty acid peroxidation end products, reduced numbers of inflammatory cells, and/or upregulated expression of antioxidants, wherein antioxidants comprise heme oxygenase-1 (HO-1), catalase, superoxide dismutase-2 (SOD-2), and glutamate-cystein ligase catalytic (GCLC) subunit, and further wherein the inflammatory cells comprise CD68+ macrophages and CD3+ T-cells.

7. The composition for use according to claim 5 or 6, wherein increased mitochondrial function comprises increased mitochondrial ultrastructure, increased mitochondrial biogenesis, and/or increased nuclear PPAR-γ co-activator- 1 (PGC-1) expression.

8. The composition for use according to any of the preceding claims, wherein the CDCs secrete exosomes that comprise one or more microRNAs selected from the group consisting of: microRNAs miR-146a, miR148a, miR22, miR-24, miR-210, miR-150, miR-140- 3p, miR-19a, miR-27b, miR-19b, miR-27a, miR-376c, miR-128, miR- 320a, miR-143, miR-21, miR-130a, miR-9, miR-185, and miR-23a.

9. A composition comprising a plurality of exosomes derived from cardiosphere-derived cells (CDC) from a human, for use in a human in a method of decreasing fibrosis and inflammation secondary to a chronic muscular disease.

10. The composition for use according to claim 9, wherein the chronic muscular disease is Duchenne muscular dystrophy.

11. The composition for use according to any of claims 9 and 10, wherein the composition is administered by a method comprising intraarterial or intravenous myocardial infusion.

12. The composition for use according to any of claims 9-11, wherein treatment of the subject results in increased mitochondrial function and/or increased cardiomyogenesis, further wherein treatment of the subject results in a reduction in collagen accumulation, and wherein collagen comprises collagen I and/or collagen III.

13. The composition for use according to claim 12, wherein treatment of the subject results in an increase in cytoplasmic nuclear factor (erythroid-derived 2)-like 2(Nrf2), reduction in fatty acid peroxidation end products, reduced numbers of inflammatory cells, and/or upregulated expression of antioxidants, wherein antioxidants comprise heme oxygenase-1 (HO-1), catalase, superoxide dismutase-2 (SOD-2) and glutamate-cystein ligase catalytic (GCLC) subunit, and further wherein the inflammatory cells comprise CD68+ macrophages and CD3+ T-cells,

14. The composition for use according to claim 12 or 13, wherein increased mitochondrial function comprises increased mitochondrial ultrastructure, increased mitochondrial biogenesis, and/or increased nuclear PPAR-γ co-activator- 1 (PGC-1) expression.
